# EUROPEAN PATENT APPLICATION

(11) **EP 2 206 721 A1**
(43) Date of publication of application: **14.07.2010**
(21) Application number: 08836074.8
(22) Date of filing: 06.10.2008
(51) Int. Cl.: C07K 7/06, A61K 38/00, A61K 47/48, A61P 3/04, C07K 14/47

(54) **NEUROMEDIN U DERIVATIVE**

(30) Priority: 05.10.2007 JP 2007261847
(71) Applicant: Takeda Pharmaceutical Company Limited, Osaka-shi, Osaka 540-8645 (JP)
(72) Inventor: OHTAKI, Tetsuya, Tsukuba-shi Ibaraki 300-4293 (JP); MASUDA, Yasushi, Tsukuba-shi Ibaraki 300-4293 (JP); KUMANO, Satoshi, Tsukuba-shi Ibaraki 300-4293 (JP); INOOKA, Hiroshi, Tsukuba-shi Ibaraki 300-4293 (JP)
(74) Representative: Brearley, Helen Rebecca
(86) International application number: PCT/JP2008/068185
(87) International publication number: WO 2009/044918

(57) **Abstract**

The objective of the present invention is to provide a new antifeedant.

The other objective of the present invention is to provide a NMU derivative showing a high antifeedant activity even in common administration forms such as peripheral administration.

A neuromedin U derivative wherein a methoxypolyethylene glycol is bound via a linker having a specific structure to a polypeptide which contains at least 8 amino acids of the C-terminus of an amino acid sequence of neuromedin U and which consists of the same or substantially the same amino acid sequence as the amino acid sequence of neuromedin U.

### [Sequence List]

SEQUENCE LISTING

<110>  Takeda Pharmaceutical Company Limited

<120>  Neuromedin U derivative

<130>  TKDWO071060P

<150>  JP2007-261847
<151>  2007-10-05

<160>  11

<170>  PatentIn version 3.4

<210>  1
<211>  25
<212>  PRT
<213>  Sus scrofa


<220>
<221>  MOD_RES
<222>  (25)..(25)
<223>  AMIDATION

<400>  1

Phe Lys Val Asp Glu Glu Phe Gln Gly Pro Ile Val Ser Gln Asn Arg
1               5                   10                  15


Arg Tyr Phe Leu Phe Arg Pro Arg Asn
            20                  25


<210>  2
<211>  8
<212>  PRT
<213>  Sus scrofa


<220>
<221>  MOD_RES
<222>  (8)..(8)
<223>  AMIDATION

<400>  2

Tyr Phe Leu Phe Arg Pro Arg Asn
1               5


<210>  3
<211>  25
<212>  PRT
<213>  Homo sapiens


<220>
<221>  MOD_RES

<222>  (25)..(25)
<223>  AMIDATION

<400>  3

Phe Arg Val Asp Glu Glu Phe Gln Ser Pro Phe Ala Ser Gln Ser Arg
1               5                   10                  15

Gly Tyr Phe Leu Phe Arg Pro Arg Asn
            20                  25

<210>  4
<211>  8
<212>  PRT
<213>  Homo sapiens

<220>
<221>  MOD_RES
<222>  (8)..(8)
<223>  AMIDATION

<400>  4

Tyr Phe Leu Phe Arg Pro Arg Asn
1               5

<210>  5
<211>  23
<212>  PRT
<213>  Rattus norvegicus

<220>
<221>  MOD_RES
<222>  (23)..(23)
<223>  AMIDATION

<400>  5

Tyr Lys Val Asn Glu Tyr Gln Gly Pro Val Ala Pro Ser Gly Gly Phe
1               5                   10                  15

Phe Leu Phe Arg Pro Arg Asn
            20

<210>  6
<211>  8
<212>  PRT
<213>  Rattus norvegicus

<220>
<221>  MOD_RES
<222>  (8)..(8)
<223>  AMIDATION

<400>  6

Phe Phe Leu Phe Arg Pro Arg Asn
1               5


<210>  7
<211>  426
<212>  PRT
<213>  Homo sapiens

<400>  7

Met Thr Pro Leu Cys Leu Asn Cys Ser Val Leu Pro Gly Asp Leu Tyr
1               5                   10                  15

Pro Gly Gly Ala Arg Asn Pro Met Ala Cys Asn Gly Ser Ala Ala Arg
            20                  25                  30

Gly His Phe Asp Pro Glu Asp Leu Asn Leu Thr Asp Glu Ala Leu Arg
        35                  40                  45

Leu Lys Tyr Leu Gly Pro Gln Gln Thr Glu Leu Phe Met Pro Ile Cys
    50                  55                  60

Ala Thr Tyr Leu Leu Ile Phe Val Val Gly Ala Val Gly Asn Gly Leu
65                  70                  75                  80

Thr Cys Leu Val Ile Leu Arg His Lys Ala Met Arg Thr Pro Thr Asn
                85                  90                  95

Tyr Tyr Leu Phe Ser Leu Ala Val Ser Asp Leu Leu Val Leu Leu Val
            100                 105                 110

Gly Leu Pro Leu Glu Leu Tyr Glu Met Trp His Asn Tyr Pro Phe Leu
        115                 120                 125

Leu Gly Val Gly Gly Cys Tyr Phe Arg Thr Leu Leu Phe Glu Met Val
    130                 135                 140

Cys Leu Ala Ser Val Leu Asn Val Thr Ala Leu Ser Val Glu Arg Tyr
145                 150                 155                 160

Val Ala Val Val His Pro Leu Gln Ala Arg Ser Met Val Thr Arg Ala
                165                 170                 175

His Val Arg Arg Val Leu Gly Ala Val Trp Gly Leu Ala Met Leu Cys
            180                 185                 190

Ser Leu Pro Asn Thr Ser Leu His Gly Ile Gln Gln Leu His Val Pro
        195                 200                 205

Cys Arg Gly Pro Val Pro Asp Ser Ala Val Cys Met Leu Val Arg Pro
    210                 215                 220

Arg Ala Leu Tyr Asn Met Val Val Gln Thr Thr Ala Leu Leu Phe Phe
225                 230                 235                 240

Cys Leu Pro Met Ala Ile Met Ser Val Leu Tyr Leu Leu Ile Gly Leu
                245                 250                 255

Arg Leu Arg Arg Glu Arg Leu Leu Leu Met Gln Glu Ala Lys Gly Arg
            260                 265                 270

Gly Ser Ala Ala Ala Arg Ser Arg Tyr Thr Cys Arg Leu Gln Gln His
        275                 280                 285

Asp Arg Gly Arg Arg Gln Val Thr Lys Met Leu Phe Val Leu Val Val
    290                 295                 300

Val Phe Gly Ile Cys Trp Ala Pro Phe His Ala Asp Arg Val Met Trp
305                 310                 315                 320

Ser Val Val Ser Gln Trp Thr Asp Gly Leu His Leu Ala Phe Gln His
                325                 330                 335

Val His Val Ile Ser Gly Ile Phe Phe Tyr Leu Gly Ser Ala Ala Asn
            340                 345                 350

Pro Val Leu Tyr Ser Leu Met Ser Ser Arg Phe Arg Glu Thr Phe Gln
        355                 360                 365

Glu Ala Leu Cys Leu Gly Ala Cys Cys His Arg Leu Arg Pro Arg His
    370                 375                 380

Ser Ser His Ser Leu Ser Arg Met Thr Thr Gly Ser Thr Leu Cys Asp
385                 390                 395                 400

Val Gly Ser Leu Gly Ser Trp Val His Pro Leu Ala Gly Asn Asp Gly
                405                 410                 415

Pro Glu Ala Gln Gln Glu Thr Asp Pro Ser
            420                 425

<210> 8
<211> 415
<212> PRT
<213> Homo sapiens

<400> 8

Met Ser Gly Met Glu Lys Leu Gln Asn Ala Ser Trp Ile Tyr Gln Gln
1               5                   10                  15

Lys Leu Glu Asp Pro Phe Gln Lys His Leu Asn Ser Thr Glu Glu Tyr
            20                  25                  30

Leu Ala Phe Leu Cys Gly Pro Arg Arg Ser His Phe Phe Leu Pro Val
        35                  40                  45

Ser Val Val Tyr Val Pro Ile Phe Val Val Gly Val Ile Gly Asn Val
    50                  55                  60

Leu Val Cys Leu Val Ile Leu Gln His Gln Ala Met Lys Thr Pro Thr
65                  70                  75                  80

Asn Tyr Tyr Leu Phe Ser Leu Ala Val Ser Asp Leu Leu Val Leu Leu
                85                  90                  95

Leu Gly Met Pro Leu Glu Val Tyr Glu Met Trp Arg Asn Tyr Pro Phe
            100                 105                 110

Leu Phe Gly Pro Val Gly Cys Tyr Phe Lys Thr Ala Leu Phe Glu Thr
        115                 120                 125

Val Cys Phe Ala Ser Ile Leu Ser Ile Thr Thr Val Ser Val Glu Arg
    130                 135                 140

Tyr Val Ala Ile Leu His Pro Phe Arg Ala Lys Leu Gln Ser Thr Arg
145                 150                 155                 160

Arg Arg Ala Leu Arg Ile Leu Gly Ile Val Trp Gly Phe Ser Val Leu
                165                 170                 175

Phe Ser Leu Pro Asn Thr Ser Ile His Gly Ile Lys Phe His Tyr Phe
            180                 185                 190

Pro Asn Gly Ser Leu Val Pro Gly Ser Ala Thr Phe Thr Val Ile Lys
        195                 200                 205

Pro Met Trp Ile Tyr Asn Phe Ile Ile Gln Val Thr Ser Phe Leu Phe
    210                 215                 220

Tyr Leu Leu Pro Met Thr Val Ile Ser Val Leu Tyr Tyr Leu Met Ala
225                 230                 235                 240

Leu Arg Leu Lys Lys Asp Lys Ser Leu Glu Ala Asp Glu Gly Asn Ala
                245                 250                 255

Asn Ile Gln Arg Pro Cys Arg Lys Ser Val Asn Lys Met Leu Phe Val
            260                 265                 270

Leu Val Leu Val Phe Ala Ile Cys Trp Ala Pro Phe His Ile Asp Arg
        275                 280                 285

Leu Phe Phe Ser Phe Val Glu Glu Trp Ser Glu Ser Leu Ala Ala Val
    290                 295                 300

Phe Asn Leu Val His Val Val Ser Gly Val Phe Phe Tyr Leu Ser Ser
305                 310                 315                 320

Ala Val Asn Pro Ile Ile Tyr Asn Leu Leu Ser Arg Arg Phe Gln Ala
                325                 330                 335

Ala Phe Gln Asn Val Ile Ser Ser Phe His Lys Gln Trp His Ser Gln
            340                 345                 350

His Asp Pro Gln Leu Pro Pro Ala Gln Arg Asn Ile Phe Leu Thr Glu
        355                 360                 365

Cys His Phe Val Glu Leu Thr Glu Asp Ile Gly Pro Gln Phe Pro Cys
    370                 375                 380

Gln Ser Ser Met His Asn Ser His Leu Pro Thr Ala Leu Ser Ser Glu
385                 390                 395                 400

Gln Met Ser Arg Thr Asn Tyr Gln Ser Phe His Phe Asn Lys Thr
                405                 410                 415

<210>  9
<211>  36
<212>  PRT
<213>  Rattus norvegicus

<220>
<221>  MOD_RES
<222>  (36)..(36)
<223>  AMIDATION

<400>  9

Leu Pro Arg Leu Leu His Thr Asp Ser Arg Met Ala Thr Ile Asp Phe
1               5                   10                  15

Pro Lys Lys Asp Pro Thr Thr Ser Leu Gly Arg Pro Phe Phe Leu Phe
            20                  25                  30

Arg Pro Arg Asn
        35

<210>  10
<211>  33
<212>  PRT
<213>  Homo sapiens

<220>
<221>  MOD_RES
<222>  (33)..(33)
<223>  AMIDATION

<400>  10

Ile Leu Gln Arg Gly Ser Gly Thr Ala Ala Val Asp Phe Thr Lys Lys
1               5                   10                  15

Asp His Thr Ala Thr Trp Gly Arg Pro Phe Phe Leu Phe Arg Pro Arg
            20                  25                  30

Asn

<210>  11
<211>  8
<212>  PRT
<213>  Homo sapiens

<220>
<221>  MOD_RES
<222>  (8)..(8)
<223>  AMIDATION

<400>  11

Phe Phe Leu Phe Arg Pro Arg Asn
1               5

## Description

### [Technical Field]

The present invention relates to a neuromedin U derivative.

### [Background Technology]

Neuromedin U (NMU) was first isolated from the pig small intestine using the contraction activity of the uterine smooth muscle as a peptide consisting of 25 amino acid residues [Sequence No. 1], or as a peptide consisting of 8 amino acid residues [Sequence No. 2]. These peptides are named as porcine NMU-25 [Sequence No. 1] or porcine NMU-8 [Sequence No. 2] based on the number of amino acid residues. The porcine NMU-8 [Sequence No. 2] contains 8 residues of the C-terminus of the porcine NMU-25 [Sequence No. 1] and is produced when the porcine NMU-25 is broken down.
In humans, similarly a NMU-25 [Sequence No. 3] is known and the amino acid sequence of the C-terminal 8 residues [Sequence No. 4] contains the same sequence as that of porcine NMU-8.
Further, the number of amino acid residues of rat NMU is 23 and it is named as NMU-23 [Sequence No. 5]. The amino acid sequence of C-terminal 8 residues [Sequence No. 6] contains one different residue from that of the porcine NMU-8.

As a NMU receptor, FM3 which is an orphan GPCR was initially identified and subsequently TGR1 was identified. Today, these receptors are called NMUR1 [Sequence No. 7] and NMUR2 [Sequence No. 8] respectively. The FM3 is primarily distributed in the intestinal tract, whereas the TGR1 is localized in the hypothalamus.
Furthermore, as a TGR1 receptor, a new peptide was isolated from rat brains. Since this peptide is localized in the suprachiasmatic nucleus within the hypothalamus, it was named as neuromedin S (NMS) [Sequence No. 9] after the capital letters of the suprachiasmatic nucleus.
Although human NMS [Sequence No. 10] consists of 33 amino acid residues, its amino acid sequence of the C-terminal 8 amino acid residues was the same as the amino acid sequence of C-terminal 8 residues [Sequence No. 11] of the rat NMU-23 [Sequence No. 5].
NMUR1 and NMUR2 exhibit almost the same affinity to NMU, NMS, and NMU-8, and it was suggested that these receptors strongly recognized amino acid sequences consisting of C-terminal 8 residues that are common sequences of NMU and NMS.
An intraventricular administration of the rat NMU-23 in rats induces eating suppression. A local injection of NMU to the paraventricular nucleus (PVN) and arcuate nucleus (ARC) was also reported to express an antifeedant activity as in the case of its intraventricular administration so that the active sites of NMU are assumed to be PVN and ARC. Further, an intraventricular administration of anti-NMU antibody was shown to increase the amount of food intake, suggested that the central NMU exhibits physiologically an antifeedant effect. Furthermore, it was also reported that NMU KO mice exhibited an expression type of the obesity and that NMU over-expressing mice exhibited lower bodyweight and less amount of food intake. Thus, physiological implication of endogenous NMU was clarified.
Further, it was reported that an intraventricular administration of NMU causes an elevation of body temperature, generation of heat and elevation of oxygen consumption. This activity is assumed to be due to the activation of fat tissue and the muscle system by sympathetic nerves.
It was also reported that suppression of gastric acid secretion and suppression of gastric emptying are caused by the intraventricular administration of NMU. This activity is assumed to be due to the central action via CRH secretion. These activities act in such a direction suppressing the amount of food intake.
Although details have not yet been investigated on the activity of peripheral administration to the intestinal tract, NMUR1 is represented in the intestinal tract so that it is possible that the peripheral administration of NMU has a certain action on the intestinal tract. Based on these hypotheses, the effects of NMU peripheral administration on the stomach and intestinal tract were investigated and colon-specific prokinetic activity was discovered.

According to the pamphlet of International Publication No. 2007-075439, it was disclosed that an antifeedant effect was achieved by peripheral administration of NMU.
The inventors also independently discovered that the NMU-23 expresses an antifeedant activity via peripheral administration. In contrast, despite the fact that the NMU-8 has a sufficiently strong agonist activity to the receptor NMUR1 and NMUR2, it did not express an antifeedant activity via peripheral administration.
In order for the neuromedin U to be useful as an antifeedant, it is very important that it expresses a high antifeedant activity even in a common administration form such as via peripheral administration.

Further, as PEG derivatives which are used for usages such as chemical modifications in the field of medicine, various compounds are known.

### [Disclosure of the Invention]

### [Problems to be Solved by the Invention]

The objective of the present invention is to provide a novel antifeedant. The present invention also intended to provide a neuromedin U derivative expressing a high antifeedant activity even in a common administration form such as peripheral administration.

### [Means for Solving the Problems]

The inventors hypothesized that a cause for the absence of an antifeedant activity via peripheral administration is instability of the NMU-8 in the blood. Further, the inventors assumed that a NMU-8 derivative (or modified compound) having high stability in the blood exhibits a sufficient antifeedant activity. Thus, polyethylene glycol was added to the NMU-8 to produce a NMU-8 derivative having high stability in the blood, specifically to prepare a neuromedin U derivative which is a polypeptide which contains at least 8 amino acids of the C terminus of an amino sequence of neuromedin U, which consists of the same or substantially the same amino acid sequence as that of neuromedin U, and to which methoxypolyethylene glycol is bound via a linker. These NMU-8 modified compounds were found to express a sufficiently strong antifeedant activity and bodyweight reducing activity even via peripheral administration.

Based on this finding, the inventors further continued the research and completed the present invention.

That is, the present invention provides the following items [1] through [18].
[1]
A neuromedin U derivative
which is a polypeptide consisting of an amino acid sequence which is bound with a methoxypolyethylene glycol(s) via a linker,
said amino acid sequence contains at least 8 amino acids of the C-terminus of an amino acid sequence of neuromedin U, and is the same or substantially the same as the amino acid sequence of neuromedin U, and
which is represented by a formula:

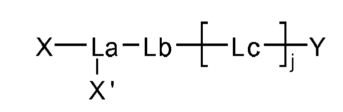

wherein
Y represents a polypeptide consisting of a amino acid sequence which contains at least 8 amino acids of the C-terminus of neuromedin U and is the same or substantially the same as the amino acid sequence of neuromedin U;
X represents a methoxyethylene glycol;
X' is absent or represents a methoxypolyethylene glycol;
the part represented by a formula (II): 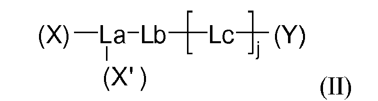 represents a linker,
La represents a divalent or trivalent group selected from 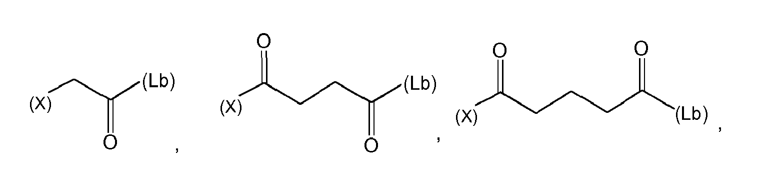 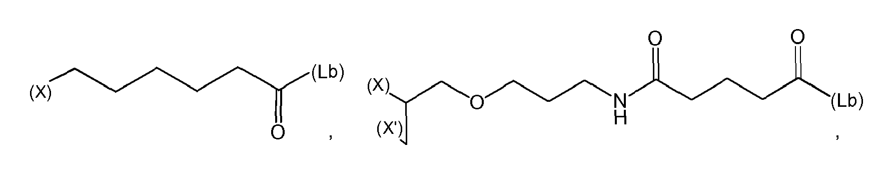 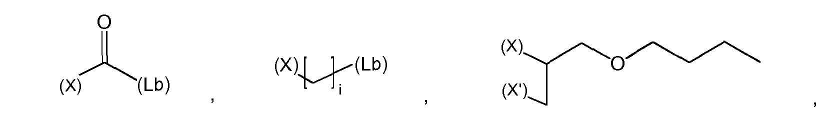 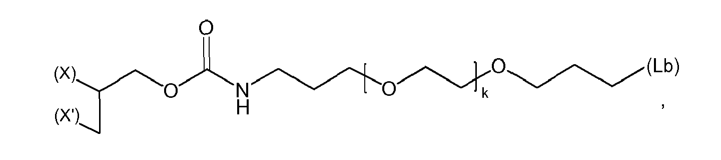 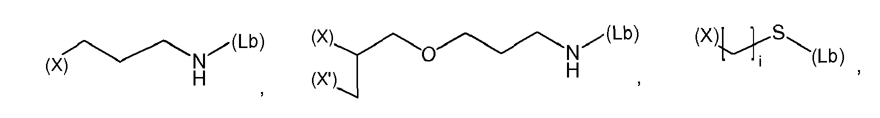 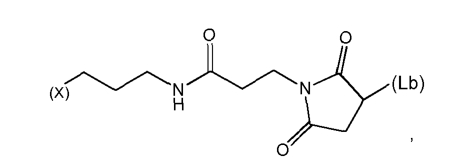 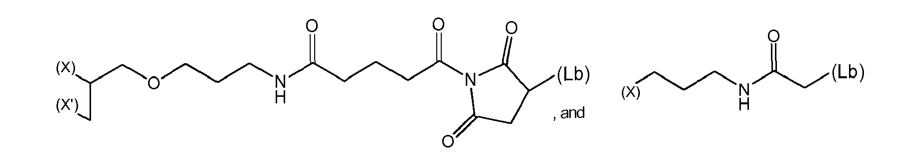 wherein
i represents an integer ranging from 1 to 5 and
k represents an integer ranging from 1 to 100;
Lb represents
(i) a bond,
(ii) a divalent group represented by a formula: -B^{1a}-Q^{b1}-B^{1b}-
   wherein
   B^{1a} and B^{1b} represent -CO-,
   Q^{b1} represents a divalent group selected from 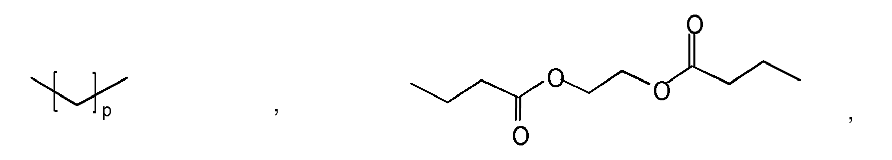 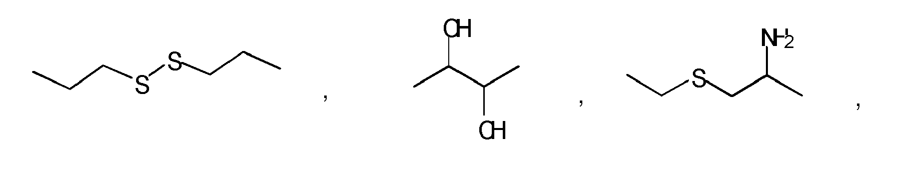 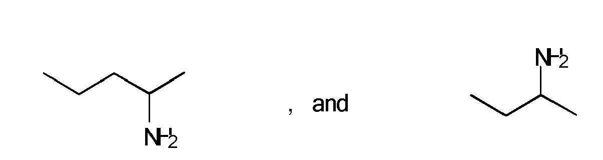 wherein p represents an integer ranging from 2 to 8,
(iii) a divalent group represented by a formula: -B^{2a}-Q^{b2}-B^{2b}-
   wherein
   B^{2a} represents -CO-,
   B^{2b} represents 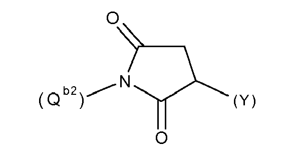 Q^{b2} represents a divalent group selected from 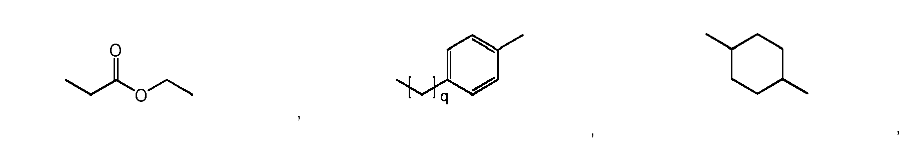 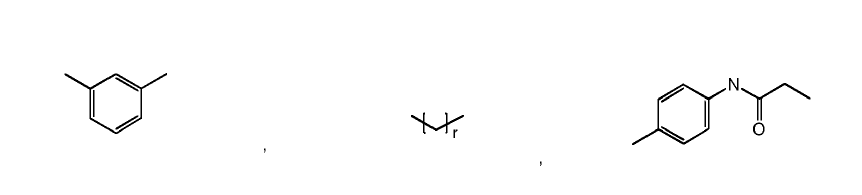 , and 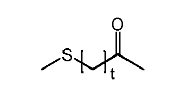 wherein
   q represents an integer ranging from 3 to 10,
   r represents an integer ranging from 1 to 10, and
   t represents an integer ranging from 1 to 10, or
(iv) a divalent group represented by a formula: -B^{3a}-Q^{b3}-B^{3b}-
   wherein
   B^{3a} represents 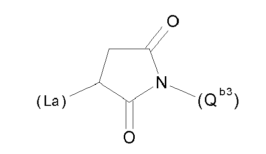 or a bond,
   B^{3b} represents -CO-,
   Q^{b3} represents a divalent group represented by a formula: -(CH₂)ₙ₁-Z-(CH₂)ₙ₂-
   wherein n1 represents an integer ranging from 0 to 5,
   n2 represents an integer ranging from 0 to 5,
   Z represents a bond, -O-CO-, -CO-NH-, -CO-O-, -NH-CO-, 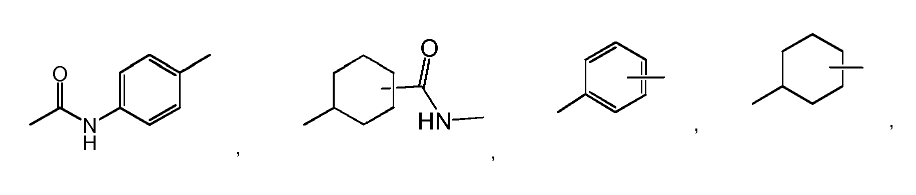 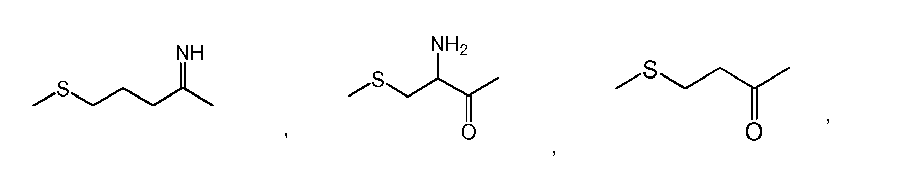 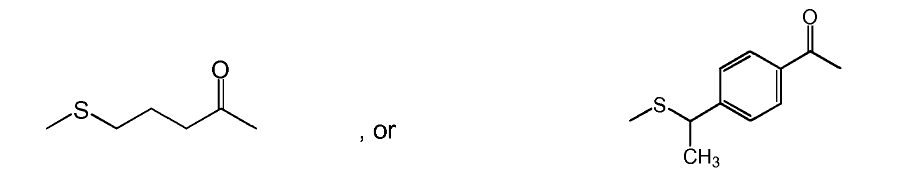 Lc represents
   (i) a divalent group represented by a formula: -C^{a}-Q^{c}-C^{b}-
      wherein
      C^{a} represents -NH-,
      Q^{c} represents a divalent group of a formula: -(CH2)ₘ₁-Z^{c}-(CH₂)ₘ₂-
      wherein
      m1 represents an integer ranging from 0 to 15,
      Z^{c} represents
      (a) a bond or
      (b) a divalent group selected from -CO-, -O-CO-, -CO-O-, -CO-NH-, -NH-CO-, -CO-NH-CO-, -NH-CO-NH-, -CH(NH₂)-, -CH(-NHR^{Zc1})-, -CH(R^{Zc2})-, -CH(OH)-, -CH(COOH)-, -C(=NH)-, -CH(-NHX)-, 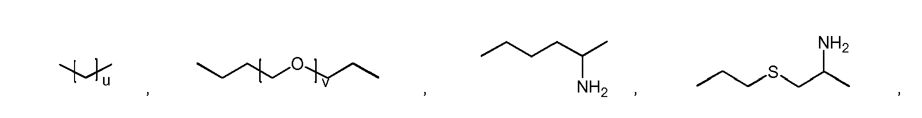 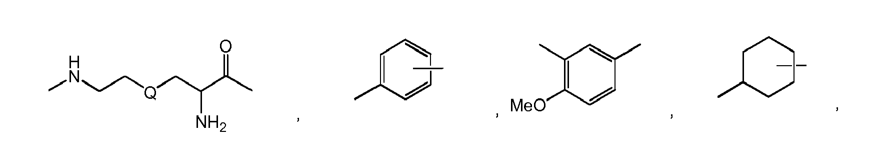 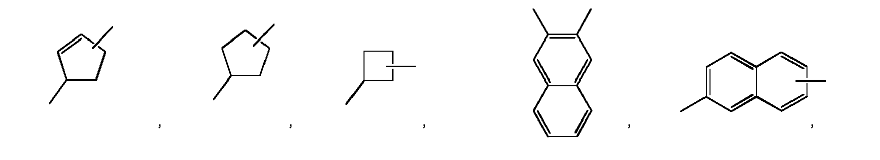 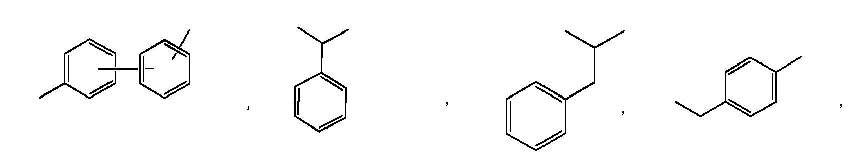 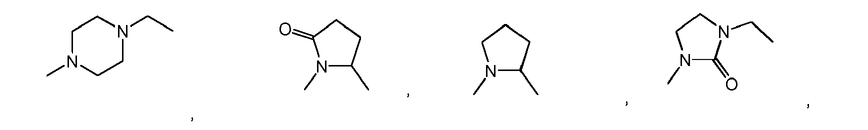 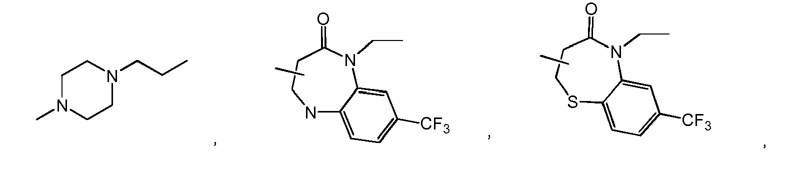 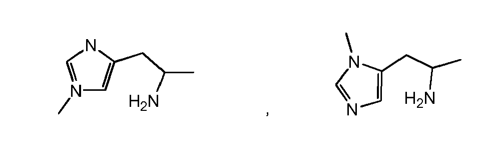 , and 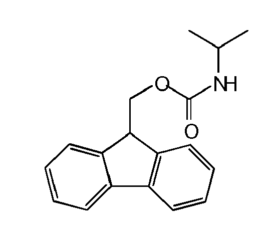 wherein
         u represents an integer ranging from 1 to 18,
         v represents an integer ranging from 1 to 12,
         R^{Zc1} represents an amino - straight chain C₁-₅ alkyl-carbonyl group or X -straight chain C₁-₅ alkyl group,
         R^{Zc2} represents an amino-straight chain C₁-₅ alkyl-carbonyl amino - straight chain C₁-₅ alkyl group, and
         X represents the same as mentioned above, and
         m2 represents an integer ranging from 0 to 15, and
         C^{b} represents a bond, -CO-, or -SO₂-, or
   (ii) a divalent group represented by a formula: -Q^{c'}-C^{b'}-
      wherein
      Q^{c'} represents a divalent group represented by a formula: -(CH2)_{m1'}-Z^{c'}-(CH₂)_{m2'}-
      wherein
      m1' represents an integer ranging from 0 to 15,
      Z^{c'} represents 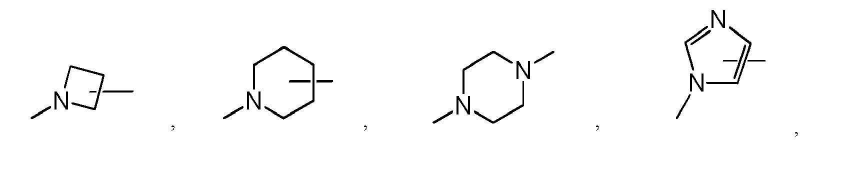 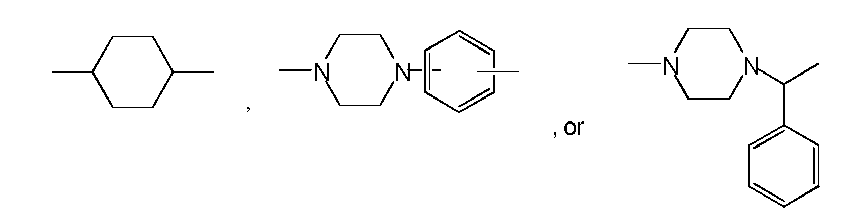 and
      m2' represents an integer ranging from 0 to 15,
      C^{b'} represents -CO- or -SO₂-;
      j represents an integer ranging from 0 to 3,
      provided that, if La is 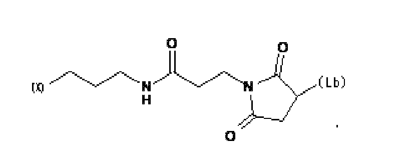 and Lb is a bond,
      then Lc is not a bond; and
      further provided that
      if La is 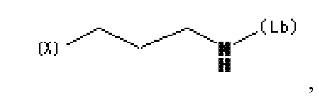 and
      Lb is a divalent group represented by a formula: -CO-Q^{b2}-B^{2b}-
      wherein
      Q^{b2} is 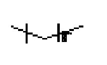 wherein r is 2,
      B^{2b} is 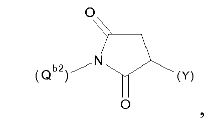 then Lc is not a bond.

[2] The neuromedin U derivative as in the aforementioned [1] wherein the neuromedin U consists of an amino acid sequence represented by one of the sequence numbers: 1 to 6.

[3] The neuromedin U derivative as in the aforementioned [1] wherein the polypeptide consists of 8 amino acids of the C-terminus of an amino acid sequence of neuromedin U.

[4] The neuromedin U derivative as in the aforementioned [3] wherein the polypeptide consists of an amino acid sequence represented by one of the sequence numbers: 2, 4 and 6.

[5] The neuromedin U derivative as in the aforementioned [1] which is represented by a formula: 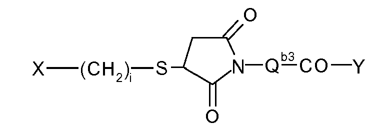 wherein
X represents a methoxypolyethylene glycol;
i represents an integer ranging from 0 to 5;
Q^{b3} represents a divalent group represented by a formula: -(CH₂)ₙ₁-Z-(CH₂)ₙ₂-
wherein
n1 represents an integer ranging from 0 to 5,
n2 represents an integer ranging from 0 to 5,
Z represents a bond, -O-CO-, -CO-NH-, -CO-O-, -NH-CO-, 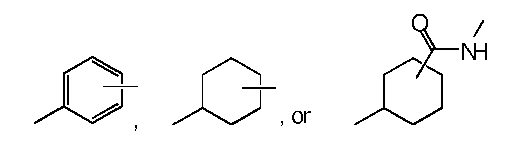 Y represents a polypeptide consisting of a amino acid sequence which contains at least 8 amino acids of the C-terminus of neuromedin U and is the same or substantially the same as the amino acid sequence of neuromedin U.

[6] The neuromedin U derivative as in the aforementioned [1] which is represented by a formula: 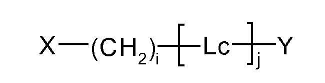 wherein
X represents a methoxypolyethylene glycol;
i represents an integer ranging from 1 to 5;
Lc represents
(i) a divalent group represented by a formula: -C^{a}-Q^{c}-C^{b}-
   wherein
   C^{a} represents -NH-,
   Q^{c} represents a divalent group represented by a formula: -(CH₂)ₘ₁-Z^{c}-(CH₂)ₘ₂-
   wherein
   m1 represents an integer ranging from 0 to 15,
   Z^{c} represents
   (a) a bond or
   (b) a divalent group selected from -CO-, -O-CO-, -CO-NH-, -NH-CO-, - CO-NH-CO-, -NH-CO-NH-, -CH(NH₂)-, -CH(NHR^{Zc1})-, -CH(R^{Zc2})-, -CH(OH)-, - CH(COOH)-, -C(=NH)-, -CH(-NHX)-, 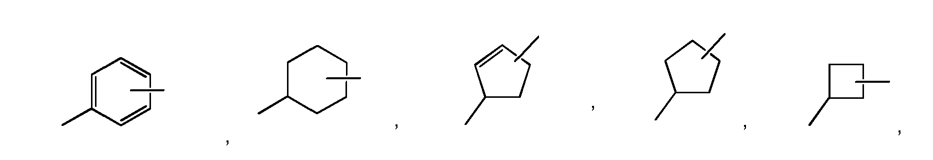 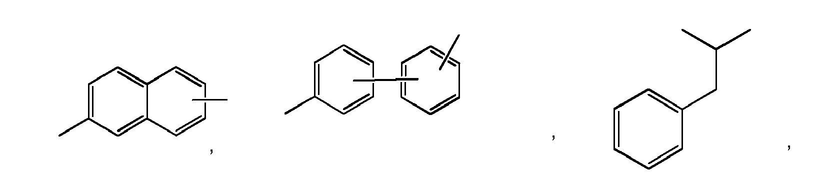 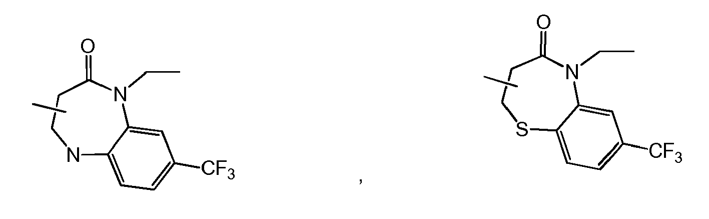 , or 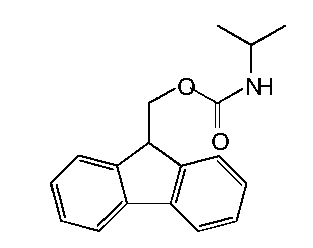 wherein
      R^{Zc1} represents an amino-straight chain C₁-₅ alkyl - carbonyl group or X -straight chain C₁-₅ alkyl group,
      R^{Zc2} represents an amino-straight chain C₁-₅ alkyl - carbonyl amino-straight chain C₁-₅ alkyl group, and
      X represents the same as mentioned above, and
      m2 represents an integer ranging from 0 to 15, and
      C^{b} represents -CO-, or -SO₂-, or
(ii) a divalent group represented by a formula: -Q^{c'}-C^{b'}-
   wherein
   Q^{c'} represents a divalent group represented by a formula: -(CH₂)ₘ₁-Z^{c'}-(CH₂)_{m2'}-
   wherein
   m1' represents an integer ranging from 0 to 15,
   Z^{c'} represents 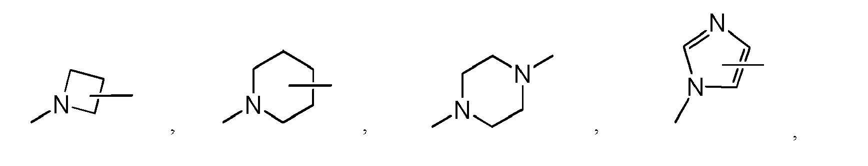 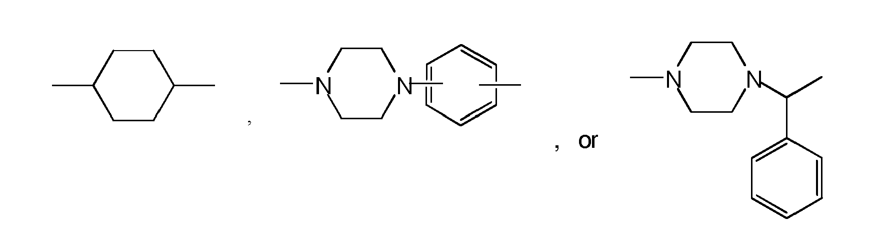 and
   m2' represents an integer ranging from 0 to 15)
   C^{b'} represents -CO- or -SO₂-;
   j' represents an integer ranging from 0 to 3; and
   Y represents a polypeptide consisting of a amino acid sequence which contains at least 8 amino acids of the C-terminus of neuromedin U and is the same or substantially the same as the amino acid sequence of neuromedin U,
   Lc can be identical or different when repeated.

[7] The neuromedin U derivative as in the aforementioned [1] which is represented by a formula: 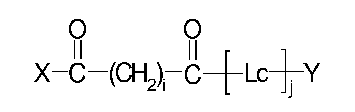 wherein
X represents a methoxypolyethylene glycol;
i represents an integer ranging from 1 to 5;
Lc represents
(i) a divalent group represented by a formula: -C^{a}-Q^{c}-C^{b'}-
   wherein
   C^{a} represents -NH-,
   Q^{c} represents a divalent group represented by a formula: -(CH₂)ₘ₁-Z^{c}-(CH₂)ₘ₂-
   wherein
   m1 represents an integer ranging from 0 to 15,
   Z^{c} represents a bond, -CO-, -O-CO-, -CO-NH-, -NH-CO-, -CO-NH-CO-, - NH-CO-NH-, -CH(NH₂)-, -CH(OH)-, -CH(COOH)-, -C(=NH)-, 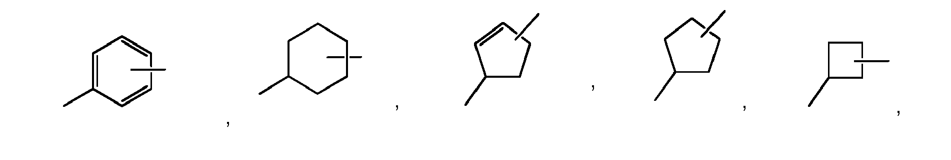 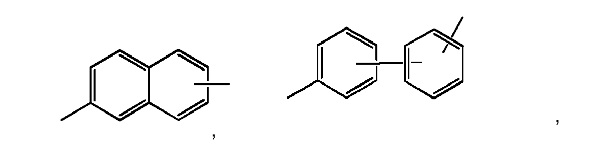 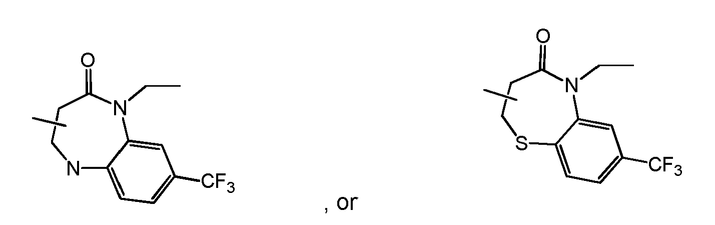 m2 represents an integer ranging from 0 to 15,
   C^{b'} represents -CO-, or -SO₂-, or
(ii) a divalent group represented by a formula: -Q^{c'}-C^{b'}-
   wherein
   Q^{c'} represents a divalent group represented by a formula: -(CH_{2)m1'}-Z^{c'}-(CH₂)_{m2'}-
   wherein
   m1' represents an integer ranging from 0 to 15,
   Z^{c'} represents 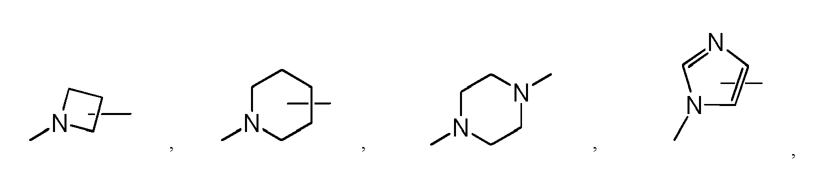 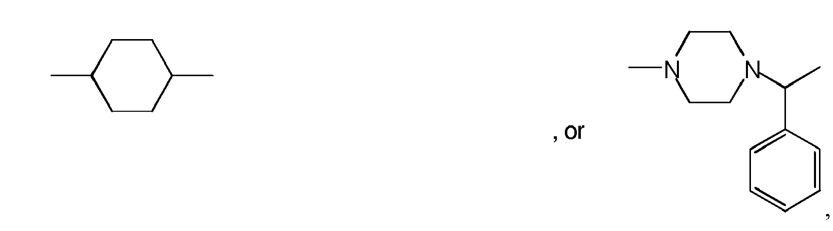 and
   m2' represents an integer ranging from 0 to 15, and
   C^{b'} represents -CO- or -SO₂-,
   j' represents an integer ranging from 0 to 3, and
   Y represents a polypeptide consisting of a amino acid sequence which contains at least 8 amino acids of the C-terminus of neuromedin U and is the same or substantially the same as the amino acid sequence of neuromedin U, and
   Lc can be identical or different when repeated.

[8] An antifeedant containing the neuromedin U derivative as in the aforementioned [1].
[9] An agent for preventing or treating obesity which contains the neuromedin U derivative as in the aforementioned [1].
[10] A method for preventing and treating obesity which is **characterized in that** an effective amount of the neuromedin U derivative as in the aforementioned [1] is administered in mammals.
[11] Use of the neuromedin U derivative as in the aforementioned [1] for manufacturing an agent for preventing or treating.

[12] A neuromedin U derivative
which is a polypeptide consisting of an amino acid sequence which is bound with a methoxypolyethylene glycol(s) via a linker,
said amino acid sequence contains at least 8 amino acids of the C-terminus of an amino acid sequence of neuromedin U, and is the same or substantially the same as the amino acid sequence of neuromedin U, and
which is represented by a formula: 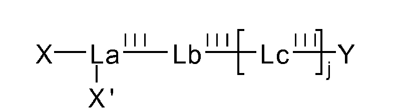 wherein
Y represents a polypeptide consisting of a amino acid sequence which contains at least 8 amino acids of the C-terminus of neuromedin U and is the same or substantially the same as the amino acid sequence of neuromedin U;
X represents a methoxyethylene glycol;
X' is absent or represents a methoxypolyethylene glycol;
the part represented by a formula (III): 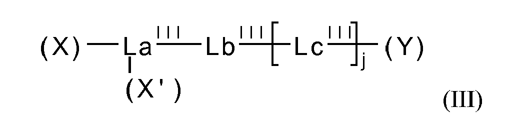 represents a linker,
La^{III} represents a divalent or trivalent group represented by a formula: 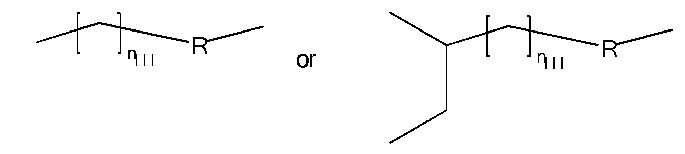 wherein
R represents a bond, -O-, -CO-O-, -O-CO-, -NH-, -CO-, -S-, -S-S-, -SO-, -SO₂-, -NH-SO₂, -SO₂-NH-, -C(=O)-NH-N=CH-, -C(=NH)-NH-, -CO-CH₂-S-, or 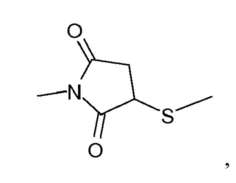 and
n _{III} represents an integer ranging from 0 to 5;
Lb^{III} represents -(CH₂)ᵢ- (wherein i represents an integer ranging from 1 to 5); Lc^{III} represents
(i) a divalent group represented by a formula: -NH-Q^{cIII}-C^{bIII}₋
   wherein
   Q^{cIII} represents a divalent group represented by a formula: -(CH₂)ₘ₁-Z^{cIII}-(CH₂)ₘ₂-
   wherein
   m1 represents an integer ranging from 0 to 15,
   Z^{cIII} represents
   (a) a bond or
   (b) a divalent group selected from -CO-, -O-CO-, -CO-O-, -CO-NH-, -NH-CO-, -CO-NH-CO-, -NH-CO-NH-, -CH(NH₂)-, -CH(-NHR^{Zc1})-, -CH(R^{Zc2})-, - CH(OH)-, -CH(COOH)-, -C(=NH)-, -S-, -S-S-, -SO-, -SO₂-, -NH-SO₂, -SO₂-NH-, 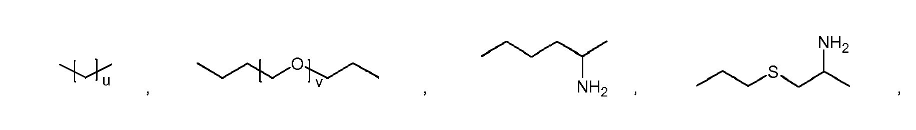 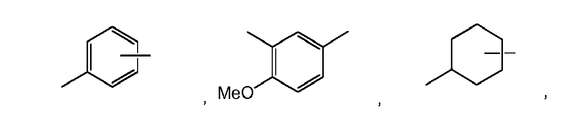 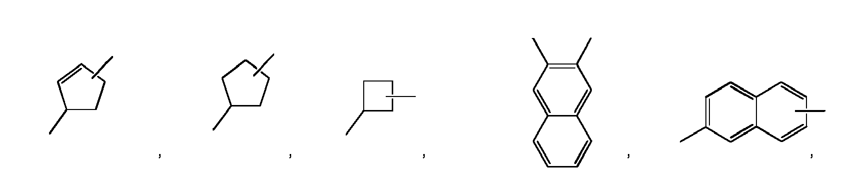 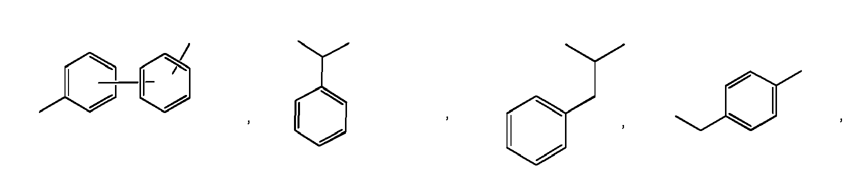 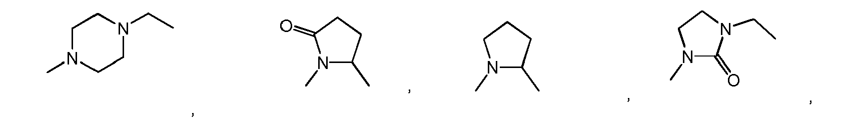 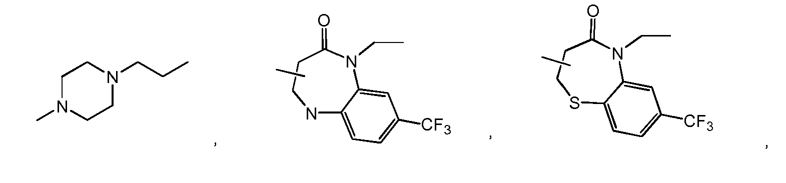 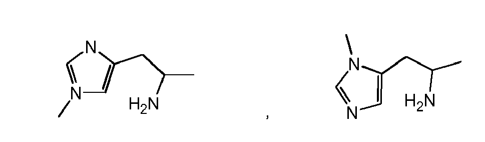 , and 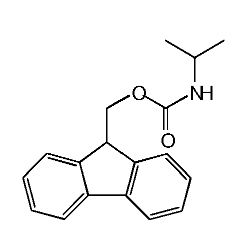 wherein
      u represents a integer ranging from 1 to 18,
      v represents an integer ranging from 1 to 12,
      R^{Zc1} represents an amino-straight chain C₁-₅ alkyl-carbonyl group or X -straight chain C₁-₅ alkyl group,
      R^{Zc2} represents an amino-straight chain C₁-₅ alkyl-carbonyl amino-straight chain C₁-₅ alkyl group, and
      X represents the same as mentioned above), and
      m2 represents an integer ranging from 0 to 15),
      C^{bIII} represents a bond, -CO-, or -SO₂-, or
(ii) a divalent group represented by a formula: ₋Q^{cIII'}-C^{bIII'}-
   wherein
   Q^{cIII}' represents a formula: -(CH₂)_{m1'}-Z^{cIII'}-(CH₂)_{m2'}-
   wherein
   m1' represents an integer ranging from 0 to 15,
   Z^{cIII'} represents a divalent group selected from 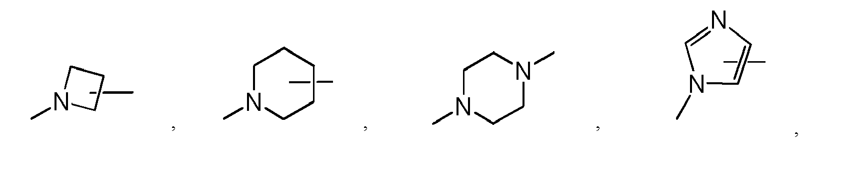 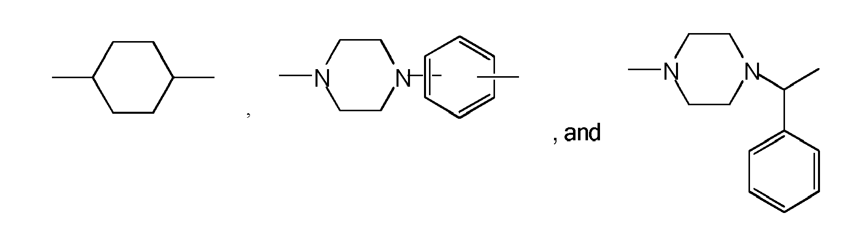 and
   m2' represents an integer ranging from 0 to 15, and
   C^{bIII'} represents -CO- or -SO₂-; and
   j^{III} represents an integer ranging from 1 to 3.

[13] The neuromedin U derivative as in the aforementioned [12] wherein the distance from the nitrogen atom closest to the Lb in the Lc to the nitrogen atom at the N-terminus of neuromedin U ranges from 3.5 to 30 Å.

[14] The neuromedin U derivative as in the aforementioned [12] wherein
if L^{cIII} is
(i) a divalent group represented by a formula: -NH-Q^{cIII}-C^{bIII}-
   wherein
   Q^{cIII} represents a divalent group represented by a formula: -(CH₂)ₘ₁-
   wherein m1 is an integer ranging from 0 to 15, and
   C^{bIII} represents a bond, -CO-, or -SO₂-,
   then the distance from the nitrogen atom ofNH in the formula: -NH-Q^{cIII}-C^{bIII}- to the nitrogen atom of the N-terminus of neuromedin U ranges from 3.5 to 7.0 Å.

[15] The neuromedin U derivative as in the aforementioned [12] wherein
if L^{cIII} is
(i) a divalent group represented by a formula: -NH-Q^{cIII}-C^{bIII}-
   wherein
   Q^{cIII} represents a divalent group represented by a formula: -(CH₂)ₘ₁-Z^{cIII}-(CH₂)ₘ₂-
   wherein
   m1 is an integer ranging from 0 to 10,
   Z^{cIII} represents a divalent group selected from -CO-, -O-CO-, -CO-O-, - CO-NH-, -NH-CO-, -CO-NH-CO-, -NH-CO-NH-, -CH(NH₂)-, -CH(-NHR^{Zc1})-, - CH(R^{Zc2})-, -CH(OH)-, -CH(COOH)-, -C(=NH)-, -S-, -S-S-, -SO-, -SO₂-, -NH-SO₂, -SO₂-NH-, 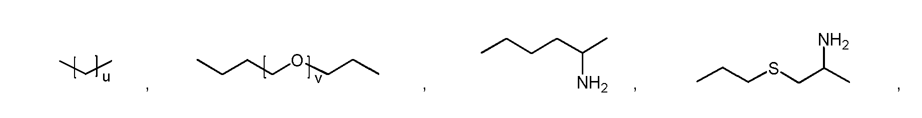 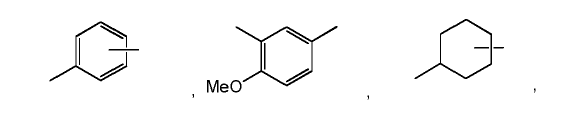 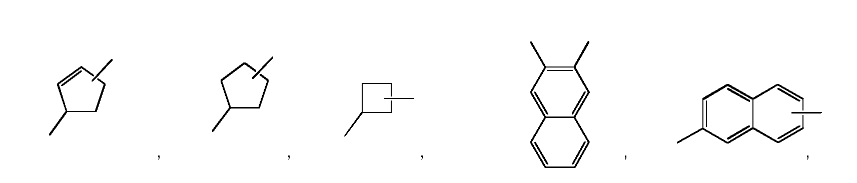 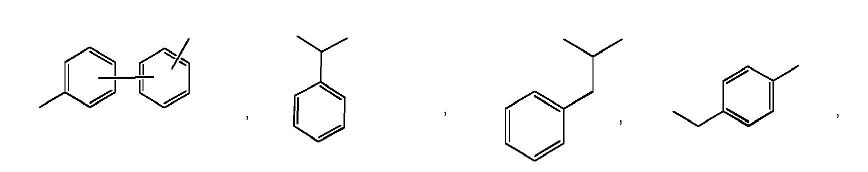 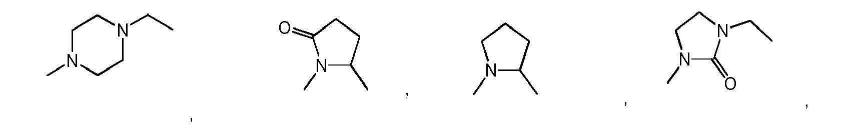 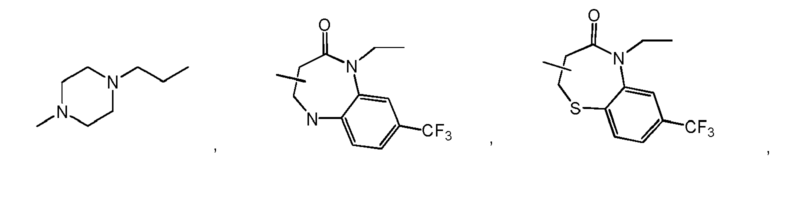 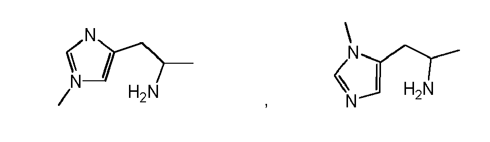 , and 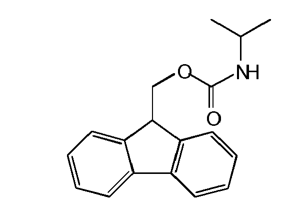 wherein
   u represents an integer ranging from 1 to 10,
   v represents an integer ranging from 1 to 10,
   R^{Zc1} represents an amino-straight chain C₁-₅ alkyl-carbonyl group or X -straight chain C₁-₅ alkyl group,
   R^{Zc2} represents an amino-straight chain C₁-₅ alkyl-carbonyl amino-straight chain C₁-₅ alkyl group, and
   m2 represents an integer ranging from 0 to 5, and
   C^{bIII} represents a bond, -CO-, or -SO₂-,
   then the distance from the nitrogen atom ofNH in the formula: -NH-Q^{c}-C^{b}- to the atom nearest to the -(CH₂)ₘ₁- part in the Z^{c} ranges from 3.5 to 10 Å, and the distance from the atom nearest to the -(CH₂)ₘ₁- part in the Z^{c} to the nitrogen atom of the N terminus of neuromedin U ranges from 3.5 to 7.0 Å.

[16] The neuromedin U derivative as in the aforementioned [12] wherein
if L^{cIII} is
(ii) a divalent group represented by a formula: -Q^{cIII'}-C^{bIII'}-
   wherein
   Q^{cIII'} is a formula: -(CH₂)ₘ₁,-Z^{c'}-(CH₂)_{m2'}-
   wherein
   m1' represents an integer ranging from 0 to 15,
   Z^{c'} represents 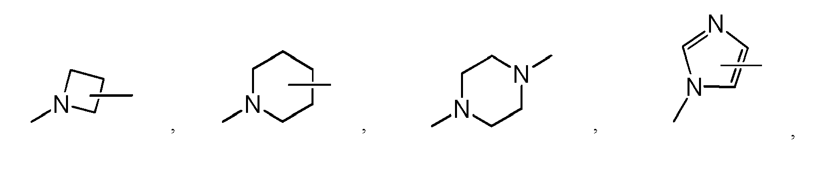 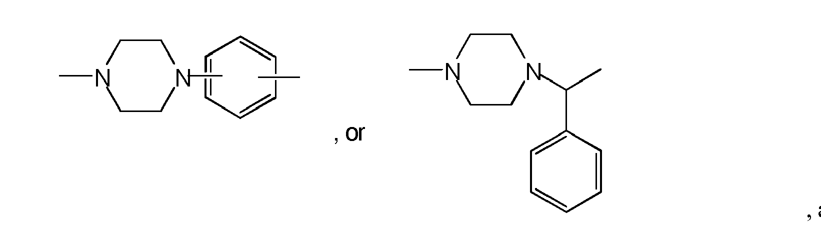 and
   m2' represent an integer ranging from 0 to 15), C^{bIII'} represents a bond, -CO- or -SO₂-,
   then the distance from the nitrogen atom nearest to Lb in 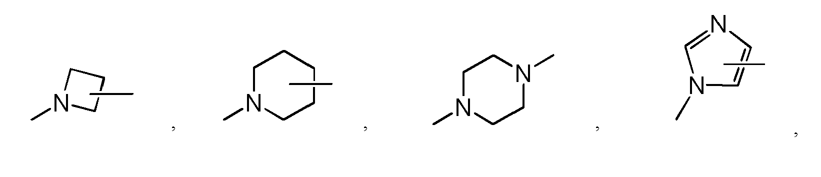 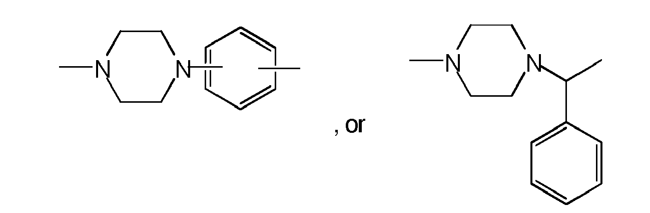 to the nitrogen atom of the N terminus of neuromedin U ranges from 5 to 10 Å.

[17] A neuromedin U derivative
which is a polypeptide consisting of an amino acid sequence which is bound with a methoxypolyethylene glycol(s) via a linker,
said amino acid sequence contains at least 8 amino acids of the C-terminus of an amino acid sequence of neuromedin U, and is the same or substantially the same as the amino acid sequence of neuromedin U, and
which is represented by a formula: 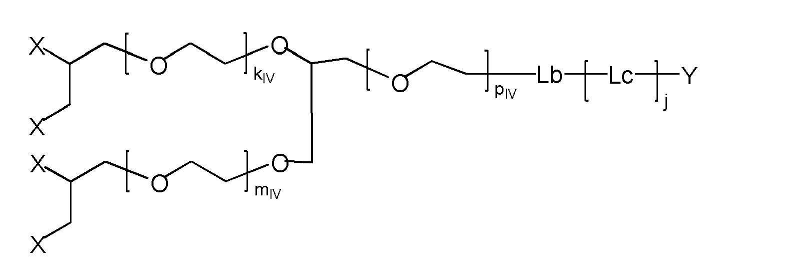 , or 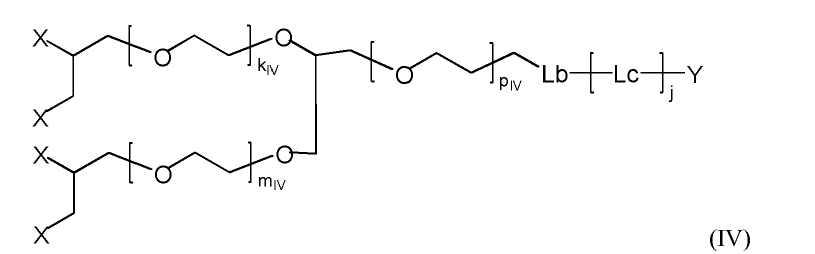 wherein
Y represents a polypeptide consisting of a amino acid sequence which contains at least 8 amino acids of the C-terminus of neuromedin U and is the same or substantially the same as the amino acid sequence of neuromedin U;
X represents a methoxypolyethylene glycol (here, the methoxypolyethylene glycol represented by plural Xs can be identical or different;
Lb represents
(i) a bond,
(ii) a divalent group represented by a formula: -B^{1a}-Q^{b1}-B^{1b}-
   wherein
   B^{1a} and B^{1b} represent -CO-,
   Q^{b1} represents a divalent group selected from 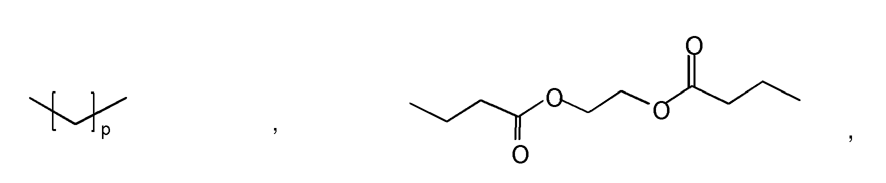 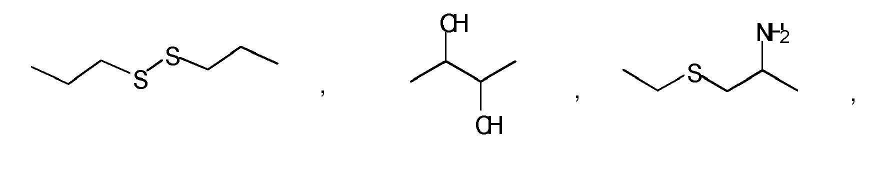 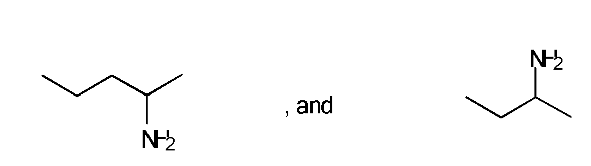 wherein p represents an integer ranging from 2 to 8, (iii) a divalent group represented by a formula: -B^{2a}-Q^{b2}-B^{2b}-
   wherein
   B^{2a} represents -CO-,
   B^{2b} represents 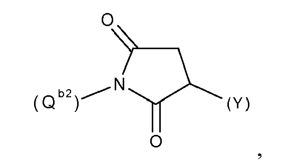 Q^{b2} represents a divalent group selected from 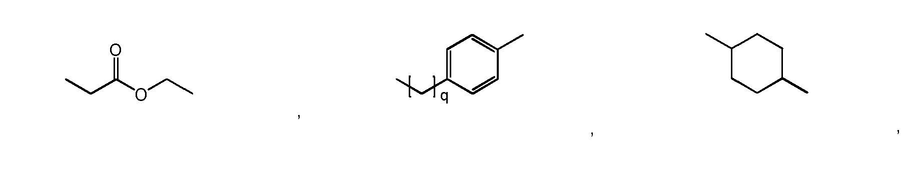 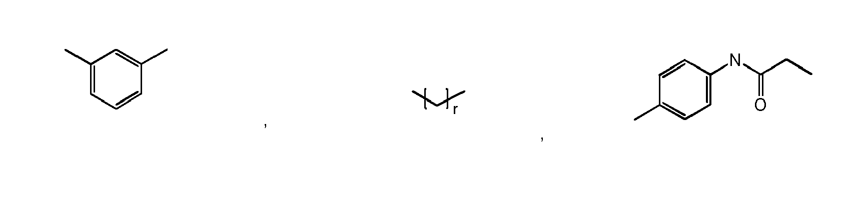 , and 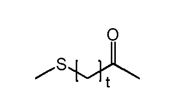 wherein
   q represents an integer ranging from 3 to 10,
   r represents an integer ranging from 1 to 10, and
   t represents an integer ranging from 1 to 10, or (iv) a divalent group represented by a formula: -B^{3a}-Q^{b3}-B^{3b}-
   wherein
   B^{3a} represents 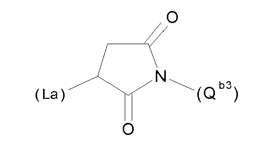 or a bond,
   B^{3b} represents -CO-,
   Q^{b3} represents a divalent group represented by -(CH₂)ₙ₁-Z-(CH₂)ₙ₂-
   wherein
   n1 represents an integer ranging from 0 to 5,
   n2 represents an integer ranging from 0 to 5,
   Z represents a bond, -O-CO-, -CO-NH-, -CO-O-, -NH-CO-, 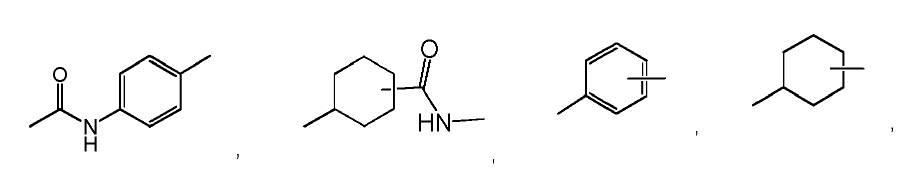 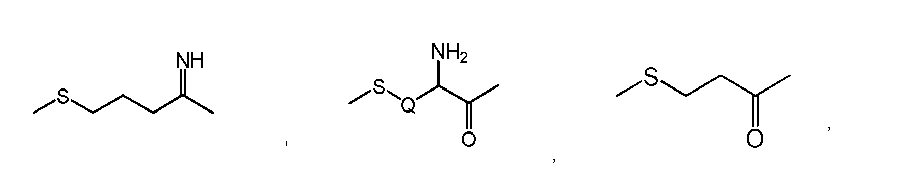 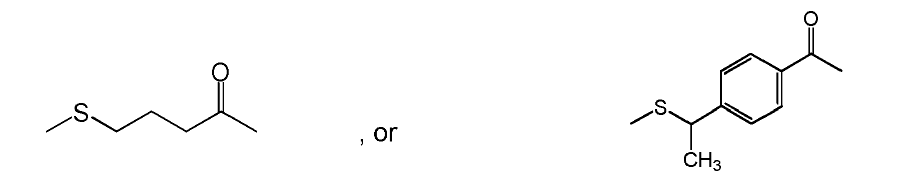 Lc represents
   (i) a divalent group represented by a formula: -C^{a}-Q^{c}-C^{b}-
      wherein
      C^{a} represents -NH-,
      Q^{c} represents a divalent group represented by a formula: -(CH₂)ₘ₁-Z^{c}-(CH₂)ₘ₂-
      wherein
      m1 represents an integer ranging from 0 to 15,
      Z^{c} represents
      (a) a bond or
      (b) a divalent group selected from -CO-, -O-CO-, -CO-O-, -CO-NH-, -NH-CO-, -CO-NH-CO-, -NH-CO-NH-, -CH(NH₂)-, -CH(-NHR^{Zc1})-, -CH(R^{Zc2})-, - CH(OH)-, -CH(COOH)-, -C(=NH)-, -CH(NHX)-, 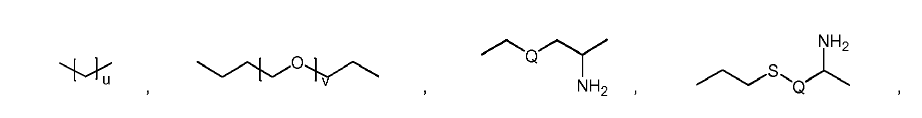 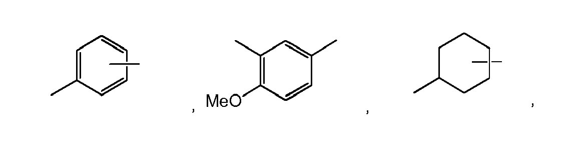 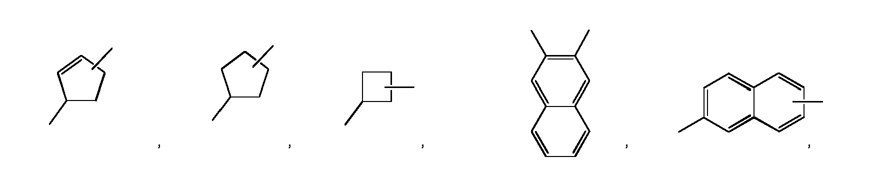 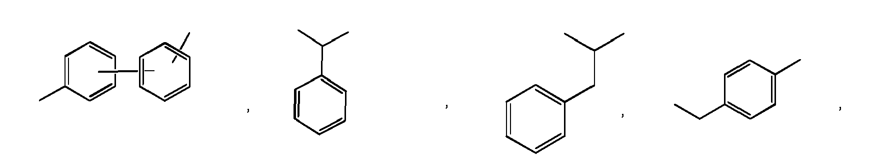 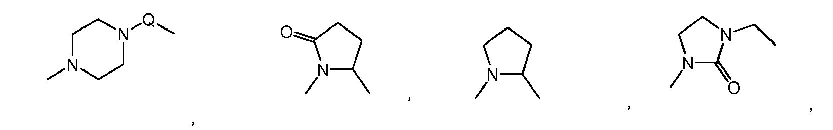 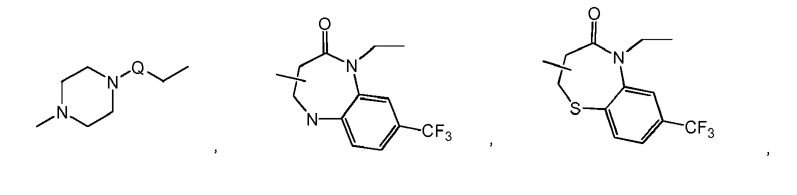 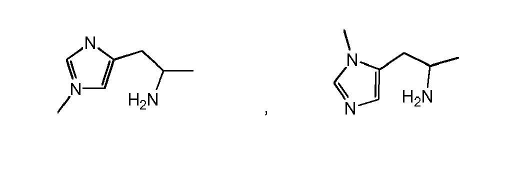 , and 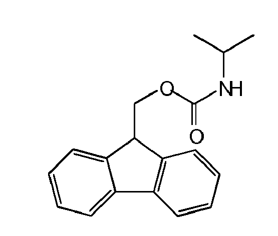 wherein
         u represents an integer ranging from 1 to 18,
         v represents an integer ranging from 1 to 12,
         R^{Zc1} represents an amino-straight chain C₁-₅ alkyl-carbonyl group or X -straight chain C₁-₅ alkyl group,
         R^{Zc2} represents an amino-straight chain C₁-₅ alkyl-carbonyl amino-straight chain C₁-₅ alkyl group,
         X represents the same as above,
         m2 represents an integer ranging from 0 to 15,
         C^{b} represents a bond, -CO- or -SO₂-, or
   (ii) a divalent group represented by -Q^{c'}-C^{b'}-
      wherein
      Q^{c'} represents a formula: -(CH₂)_{m1'}-Z^{c'}-(CH₂)_{m2'}-
      wherein
      m1' represents an integer ranging from 0 to 15,
      Z^{c'} represents 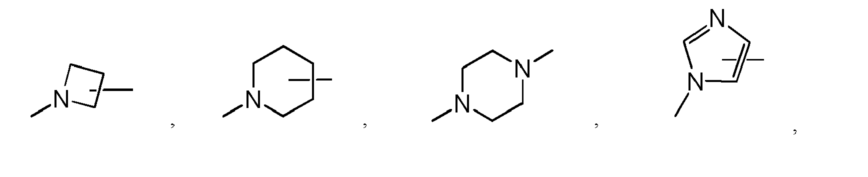 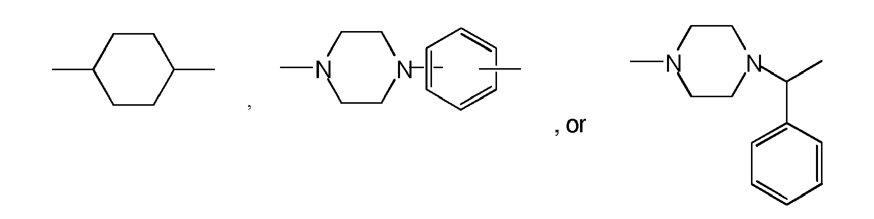 and
      m2' represents an integer ranging from 0 to 15,
      C^{b'} represents -CO- or -SO₂-;
      k_{IV} represents an integer ranging from 1 to 100;
      m_{IV} represents an integer ranging from 1 to 100;
      p_{IV} represents an integer ranging from 1 to 100; and
      j represents an integer ranging from 0 to 3.

[18] A neuromedin U derivative
which is a polypeptide consisting of an amino acid sequence which is bound with a methoxypolyethylene glycol(s) via a linker,
said amino acid sequence contains at least 8 amino acids of the C-terminus of an amino acid sequence of neuromedin U, and is the same or substantially the same as the amino acid sequence of neuromedin U, and
which is represented by a formula: 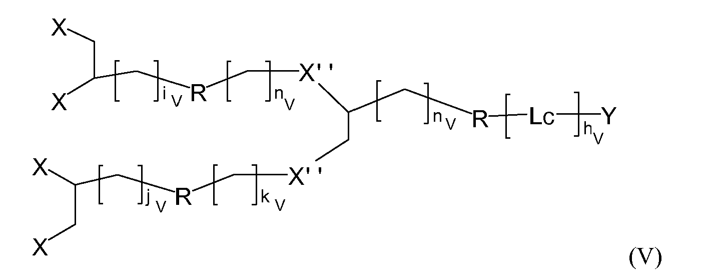 wherein
Y represents a polypeptide consisting of a amino acid sequence which contains at least 8 amino acids of the C-terminus of neuromedin U and is the same or substantially the same as the amino acid sequence of neuromedin U;
X represents a methoxypolyethylene glycol (here, the methoxypolyethylene glycols represented by plural Xs can be identical or different),
X" represents a polyethylene glycol (here, the polyethylene glycols represented by plural X" can be identical or different),
Lc represents
(i) a divalent group represented by a formula: -C^{a}-Q^{c}-C^{b}-
   wherein
   C^{a} represents -NH-,
   Q^{c} represents a divalent group: -(CH₂)ₘ₁-Z^{c}-(CH₂)ₘ₂-
   wherein
   m1 represents an integer ranging from 0 to 15,
   Z^{c} represents
   (a) a bond or
   (b) a divalent group selected from -CO-, -O-CO-, -CO-O-, -CO-NH-, -NH-CO-, -CO-NH-CO-, -NH-CO-NH-, -CH(NH₂)-, -CH(-NHR^{Zc1})-, -CH(R^{Zc2})-, -CH(OH)-, -CH(COOH)-, -C(=NH)-, -CH(NHX)-, -S-, -S-S-, -SO-, -SO₂-, -NH-SO₂-, - SO₂ -NH-, 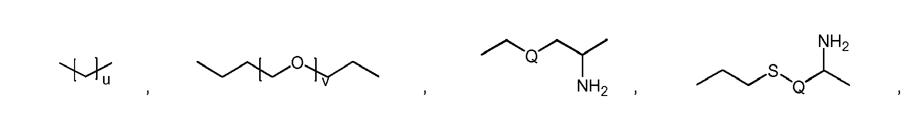 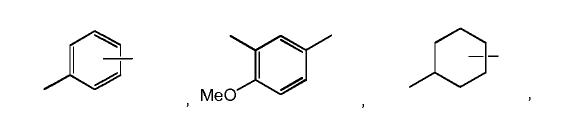 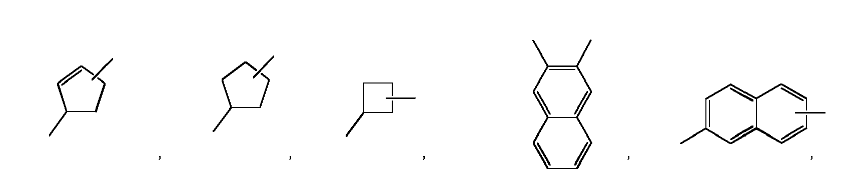 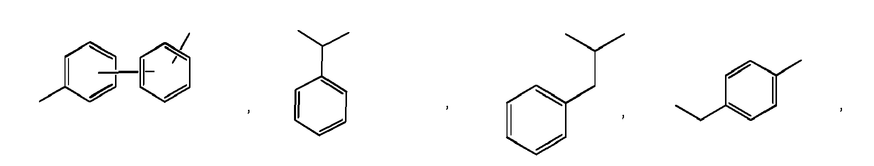 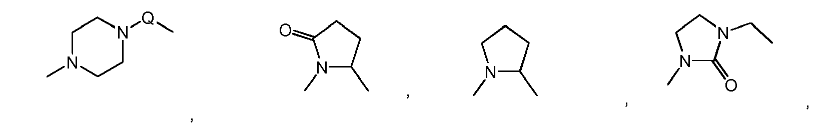 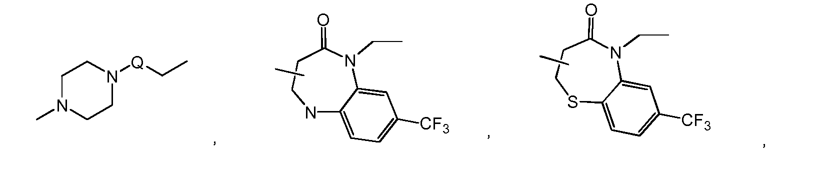 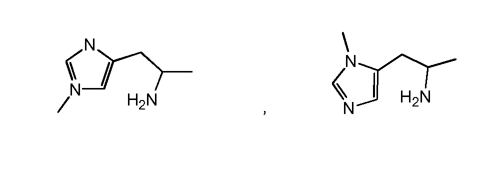 , and 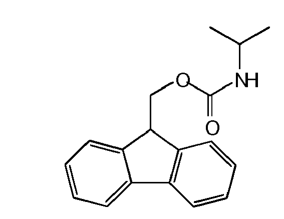 wherein
      u represents an integer ranging from 1 to 18,
      v represents an integer ranging from 1 to 12,
      R^{Zc1} represents an amino-straight chain C₁-₅ alkyl-carbonyl group or X -straight chain C₁-₅ alkyl group,
      R^{Zc2} represents an amino-straight chain C₁-₅ alkyl-carbonyl amino-straight chain C₁-₅ alkyl group,
      X represents the same as above, and
      m2 represents an integer ranging from 0 to 15, and
      C^{b} represents a bond, -CO- or -SO₂-, or
(ii) a divalent group represented by -Q^{c'}-C^{b'}-
   wherein
   Q^{c'} represents a formula: -(CH₂)_{m1'}-Z^{c'}-(CH₂)_{m2'}-
   wherein
   m1' represents an integer ranging from 0 to 15,
   Z^{c'} represents 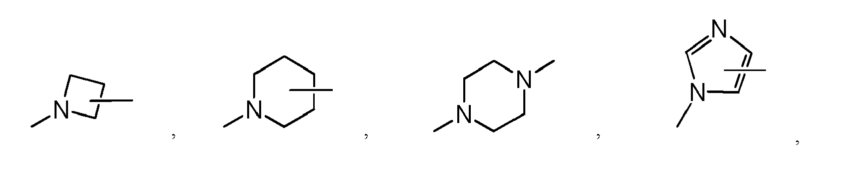 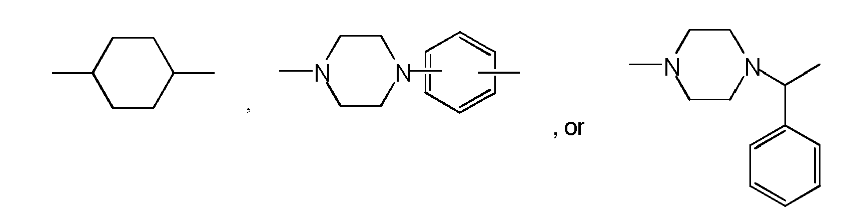 m2' represents an integer ranging from 0 to 15, and
   C^{b'} represents -CO- or -SO₂-;
   R is , at each occurrence, identical or different, and represents a divalent group selected from a bond, -O-, -CO-O-, -O-CO-, -NH-, -CO-, -S-, -S-S-, -SO-, -SO₂-, -NH-SO₂-, -SO₂-NH-, -C(=O)-NH-N=CH-, -C(=NH)-NH-, -CO-CH₂-S-, and 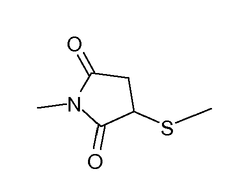 hᵥ represents an integer ranging from 0 to 3; and
   iᵥ, jᵥ, kᵥ, mᵥ and nᵥ can be respectively identical or different, which represent an integer ranging from 0 to 5.

### [Effects of the Invention]

The neuromedin U derivative of the present invention exhibits high stability and expresses a high antifeedant activity even in the common administration form such as peripheral administration so that it is useful as an antifeedant.

### [Preferred Embodiment of Carrying out the Invention]

In the present specification, examples of "straight chain C₁-₅ alkyl" include methyl, ethyl, *n*-propyl, *n*-butyl, and *n*-pentyl.
The neuromedin U derivative of the present invention is a polypeptide consisting of an amino acid sequence which is bound with a methoxypolyethylene glycol(s) via a linker, said amino acid sequence contains at least 8 amino acids of the C-terminus of an amino acid sequence of neuromedin U, and is the same or substantially the same as the amino acid sequence of neuromedin U. That is, the neuromedin U derivative of the present invention is a conjugate.
The peptide used in the present invention is bound to a linker preferably at an α amino group of the N-terminus.
That is, a neuromedin U derivative of the present invention is a compound represented by the formula: 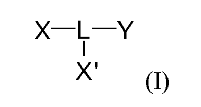 wherein
Y represents a polypeptide consisting of a amino acid sequence which contains at least 8 amino acids of the C-terminus of neuromedin U and is the same or substantially the same as the amino acid sequence of neuromedin U,
X represents a methoxypolyethylene glycol,
X' is absent or represents a methoxypolyethylene glycol, and
L represents a linker,
or a salt thereof.

In the present specification, the phrase "a polypeptide consisting of a amino acid sequence which contains at least 8 amino acids of the C-terminus of neuromedin U and is the same or substantially the same as the amino acid sequence of neuromedin U" may be simply called "the peptide to be used in the present invention".
In the present specification, in accordance with the customary expression of peptides, the N-terminal (amino terminal) is written at the left end, while the C-terminal (carboxyl terminal) is written at the right end.
"The peptide to be used in the present invention" preferably contains at least 8 amino acids of the C-terminus of an amino acid sequence of neuromedin U.
"Neuromedin U" preferably consists of an amino acid sequence represented by any one of the below described sequence numbers 1 through 6.
"The peptide to be used in the present invention" is preferably consisting of 8 amino acids of the C-terminus of an amino sequence of neuromedin U.
"The peptide to be used in the present invention" is preferably consisting of any one of the sequence numbers 2, 4 and 6.
"Substantially the same amino acid sequence as the amino acid sequence of neuromedin U" includes the amino acid sequences having a similarity to the amino acid sequence of neuromedin U by approximately 60% or greater, preferably by approximately 70% or greater, further preferably by approximately 80% or greater, particularly preferably by approximately 90% or greater, and most preferably by approximately 95% or greater.

Similarity of the amino acid sequence in the present specification can be calculated using a similarity computation algorithm NCBI BLAST (National Center for Biotechnology Information Basic Local Alignment Search Tool) under the following conditions: (expectation value = 10; gap allowed; matrix = BLOSUM 62; filtering = OFF). Other algorithms for identification of the similarity of amino acid sequences are available, for example, the algorithm described in Proc. Natl. Acad. Sci. USA, 990: 5873 -5877 (1993) by Karlin *et al.* [This algorithm was incorporated in the NBLAST and XBLAST program (Version 2.0) (Nucleic Acid Res., 25: 3389 -3402 (1997) by Altschul *et al*.]; the algorithm described in J. Mol. Biol., 48:444 -453 (1970) by Needleman *et al.,* [This algorithm was incorporated in the GAP program in the GCG Software Package]; the algorithm described in CABIOS, 4: 11-17 (1988) by Myers and Miller [This algorithm was incorporated in the ALIGN program (Version 2.0) which was a part of the CGC Sequence Alignment Software Package; the algorithm described in Proc. Natl. Acad. Sci. USA, 85: 2444 -2448 (1988) by Pearson *et al*. [This algorithm was incorporated in the FASTA program in the GCG Software Package]. Similarly, these algorithms can be also used favorably.

The peptide to be used in the present invention has substantially the same activity as that of neuromedin U.
"Examples of activities which are substantially the same as those of neuromedin U" include a FM3 binding activity, TGR1 binding activity, and antifeedant activity. "Substantially the same" implies that properties are the same characteristically (e.g., physiologically or pharmacologically). Thus, it is desirable that these activities are similar (e.g., approximately 0.01 to 100 times, preferably approximately 0.1 to 10 times, and further preferably approximately 0.5 to 2 times). However, potency of these activities can be different. These activities can be measured according to the methods described in embodiments of the specification.
Further, examples of the peptide to be used in the present invention include a polypeptide which consists of the following amino acid sequences:
(1) an amino acid sequence represented by the sequence No. 1 wherein one or two or more amino acids (e.g., 1 to 10 amino acids, 1 to 5 amino acids, 1 to 3 amino acids, 1 to 2 amino acids) are deleted, added, inserted and/or substituted (in this case, including an amino acid sequence represented by the sequence number 2 at the C-terminus);
(2) an amino acid sequence represented by the sequence No. 3 wherein one or two or more amino acids (e.g., 1 to 10 amino acids, preferably 1 to 5 amino acids, more preferably 1 to 3 amino acids, and further preferably 1 to 2 amino acids) are deleted, added, inserted and/or substituted (in this case, including an amino acid sequence represented by the sequence number 4 at the C-terminus);
(3) an amino acid sequence represented by the sequence No. 5 wherein one or two or more amino acids (e.g., 1 to 10 amino acids, preferably 1 to 5 amino acids, more preferably 1 to 3 amino acids, and further preferably 1 to 2 amino acids) are deleted, added, inserted and/or substituted (in this case, including an amino acid sequence represented by the sequence number 6 at the C-terminus), and
   has substantially the same activities as that of neuromedin U are included. Here the phrase "substantially the same activity" means the same as that mentioned earlier. Thus, it is essential that the aforementioned deletion, addition, insertion, substitution and their combinations thereof do not have any qualitative effect on the activity of neuromedin U.

The peptides to be used in the present invention are as follows:
Porcine NMU -25 composed of the amino acid sequence: Phe-Lys-Val-Asp-Glu-Glu-Phe-Gln-Gly-Pro-Ile-Val-Ser-Gln-Asn-Arg-Tyr-Phe-Leu-Phe-Arg-Pro-Arg-Asn-NH₂ (Sequence No. 1),
Porcine NMU-8 composed of the amino acid sequence: Try-Phe-Leu-Phe-Arg-Pro-Arg-Asn-NH₂ (Sequence No. 2),
Human NMU-25 composed of the amino acid sequence: Phe-Arg-Val-Asp-Glu-Glu-Phe-Gln-Ser-Pro-Phe-Ala-Ser-Gln-Ser-Arg-Gly-Tyr-Phe-Leu-Phe-Arg-Pro-Arg-Asn-NH₂ (Sequence No. 3),
Human NMU-8 composed of the amino acid sequence: Tyr-Phe-Leu-Phe-Arg-Pro-Arg-Asn-NH₂ (Sequence No. 4),
Rat NMU-23 composed of the amino acid sequence: Tyr-Lys-Val-Asn-Glu-Tyr-Gln-Gly-Pro-Val-Ala-Pro-Ser-Gly-Gly-Phe-Phe-Leu-Phe-Arg-Pro-Arg-Asn-NH₂ (Sequence No. 5), and
Rat NMU-8 composed of the amino acid sequence: Phe-Phe-Leu-Phe-Arg-Pro-Arg-Asn-NH₂ (Sequence No. 6), and
   their homologues in other mammals, and their natural allele variants.

The peptides to be used in the present invention can be peptides originated from the cells of warm-blooded animals (e.g., humans, mice, rats, guinea pigs, hamsters, rabbits, sheep, goats, pigs, horses, roosters, cats, dogs, monkeys, chimpanzees) [e.g., pancreatic cells, neural cells, glial cells, pancreatic β-cells, bone marrow cells, mesangial cells, Langerhans cells, epidermal cells, epithelial cells, goblet cells, endothelial cells, smooth muscle cells, fibroblasts, fibrocytes, muscle cells, lipocytes, immune cells (macrophages, T-cells, B-cells, natural killer cells, mast cells, neutrophils, basophils, eosinophils, monocytes, dendritic cells), megakaryocytes, synovial cells, chondrocytes, osteocytes, osteoblasts, osteoclasts, mammary cells, hepatic cells or interstitial cells, or precursor cells, stem cells, or cancer cells of these cells], or the peptides originated from all tissues where such cells are present [e.g., brain, cerebral sites (e.g., olfactory, tonsil nucleus, cerebral basal cell, hippocampus, thalamus, hypothalamus, cerebral cortex, medulla oblongata, cerebellum), spinal cord, pituitary gland, stomach, pancreas, kidney, liver, gonads, thyroid gland, gall bladder, bone marrow, adrenal gland, skin, muscle, lung, gastrointestinal tract (e.g., colon, small intestine), blood vessel, heart, thymus gland, spleen, submandibular gland, peripheral blood, prostate gland, testis, ovary, placenta, uterus, bone, joints, adipose tissue, skeletal muscle, peritoneum]. The peptides to be used in the present invention can be the peptides that are synthesized chemically or in a cell-free translation system. Alternatively, the peptides to be used in the present invention can be genetically modified peptides produced from transformants by introducing nucleic acids containing a base sequence for coding of the amino acid sequence.

"Methoxypolyethylene glycol" represented by X and X', and "polyethylene glycol" represented by X" can be straight chains or branched chains. The molecular weight of the "methoxypolyethylene glycol" and "polyethylene glycol" (or average molecular weight), not particularly limited to, preferably ranges from approximately 20,000 daltons to approximately 40,000 daltons, more preferably ranges from approximately 25,000 daltons to approximately 35,000 daltons (or average molecular weight), and further preferably approximately 30,000 daltons.

The "methoxypolyethylene glycol" is represented by a formula: MeO-(CH₂-CH₂-O)ₙ wherein n represents the degree of polymerization (or average degree of polymerization). A desirable value ranges from approximately 350 to approximately 1350 and more preferably it ranges from approximately 550 to approximately 1350.

A linker in the neuromedin U derivative of the present invention (namely a linker represented by L) is not particularly limited as long as it can connect methoxypolyethylene glycol to the peptide used in the present invention. Linkers that are commonly used for pegylation of polypeptides can be used.

### [Embodiment 1]

In one embodiment of the present invention, a linker represented by L is represented by a formula: 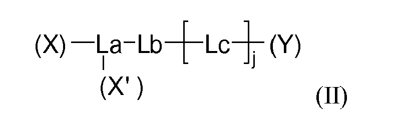 In the formulas in the present specification, symbols enclosed by round parentheses connected to the bonds such as (X), (X'), and (Lb) are used for the purpose of indicating the direction of the partial structure in the compounds.
In this embodiment 1, La is a divalent or trivalent group selected from 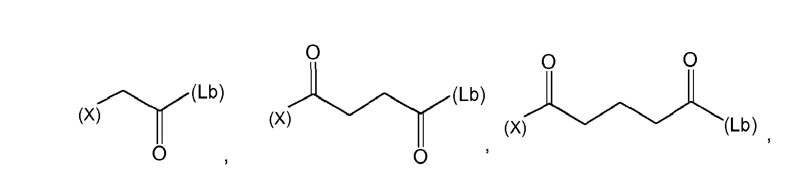 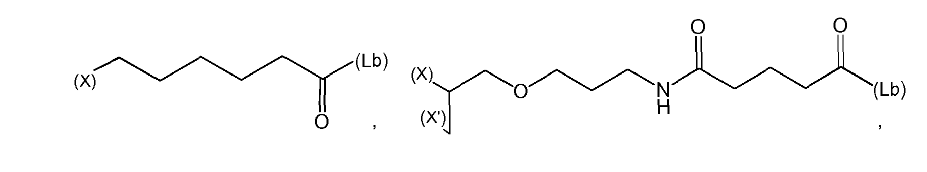 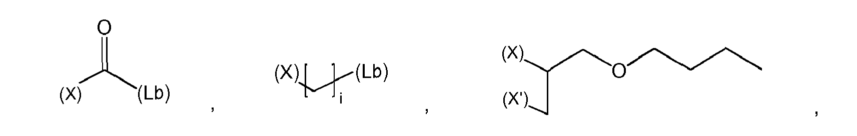 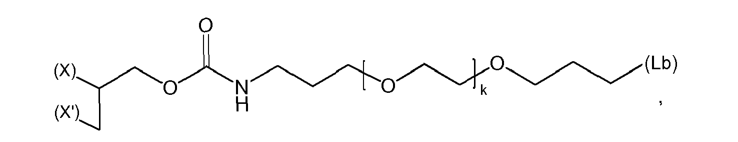 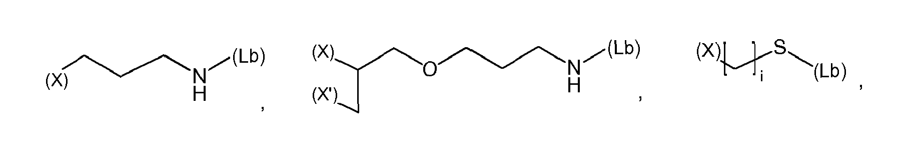 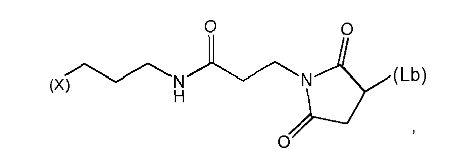 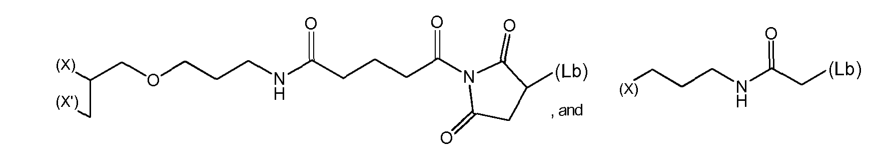 wherein i represents an integer ranging from 1 to 5 and k represents an integer ranging from 1 to 100.
In the Embodiment 1, Lb represents
(i) a bond
(ii) a divalent group represented by a formula: -B^{1a}-Q^{b1}-B^{1b}-
   wherein
   B^{1a} and B^{1b} represents -CO-,
   Q^{b1} represents a divalent group selected from 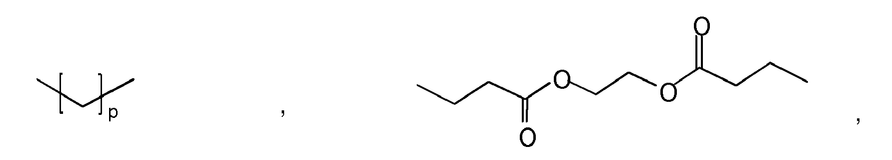 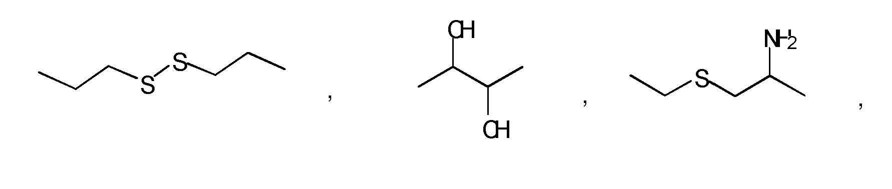 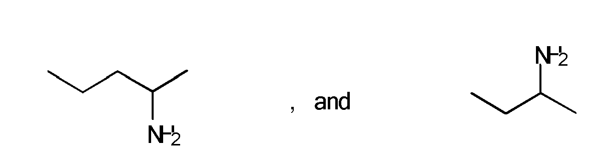 wherein p represents an integer ranging from 2 to 8,
(iii) a divalent group represented by a formula: -B^{2a}-Q^{b2}-B^{2b}-
   wherein
   B^{2a} represents -CO-,
   B^{2b} represents 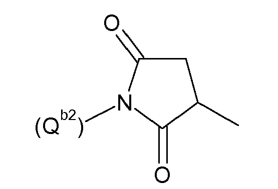 Q^{b2} represents a divalent group selected from 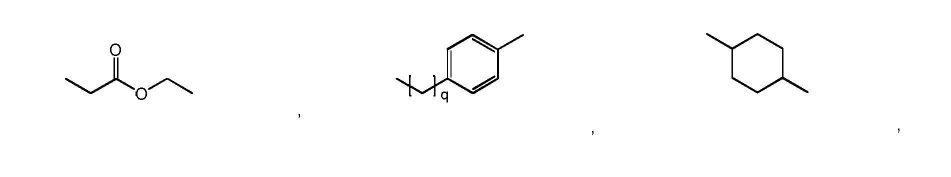 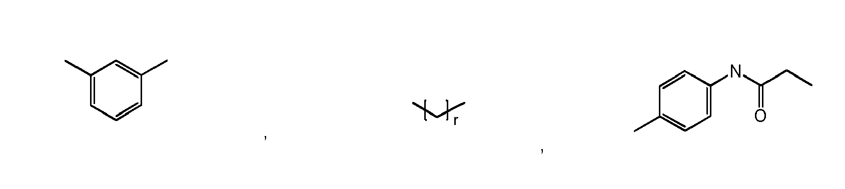 , and 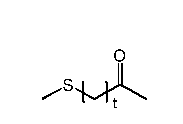 wherein q represents an integer ranging from 3 to 10, r represents an integer ranging from 1 to 10, and t represents an integer ranging from 1 to 10,
(iv) a divalent group represented by a formula: -B^{3a}-Q^{b3}-B^{3b}-
   wherein
   B^{3a} represents 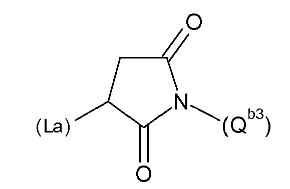 or a bond,
   B^{3b} represents -CO-,
   Q^{b3} represents -(CH₂)ₙ₁-Z-(CH₂₎ₙ₂-
   wherein
   n1 represents an integer ranging from 0 to 5,
   n2 represents an integer ranging from 0 to 5,
   Z represents a bond, -O-CO-, -CO-NH-, -CO-O-, -NH-CO-,

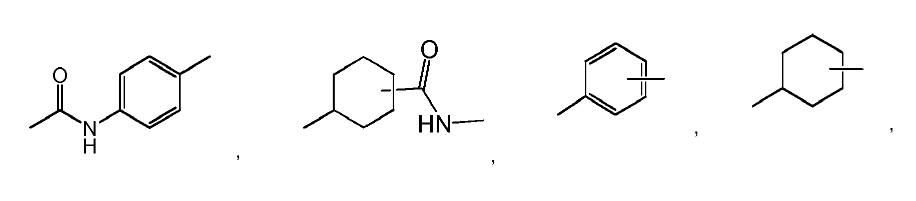

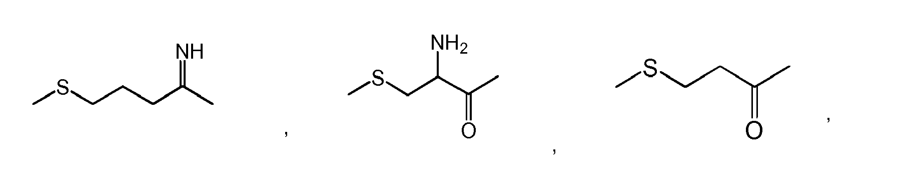

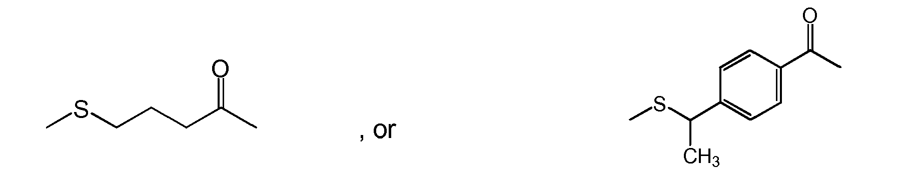

In the embodiment 1, Lc represents
(i) a divalent group represented by a formula: -C^{a}-Q^{c}-C^{b}-
   wherein
   C^{a} represents -NH-,
   Q^{c} represents a divalent group represented by a formula: -(CH₂)ₘ₁-Z^{c}-(CH₂)ₘ₂-
   wherein
   m1 represents an integer ranging from 0 to 15,
   Z^{c} represents
   (a) a bond or
   (b) a divalent group selected from -CO-, -O-CO-, -CO-NH-, -NH-CO-, -CO-NH-CO-, -NH-CO-NH-, -CH(NH₂)-, -CH(-NHR^{Zc1})-, -CH(R^{Zc2})-, -CH(OH)-, -CH(COOH)-, -C(=NH)-, -CH(-NHX)- 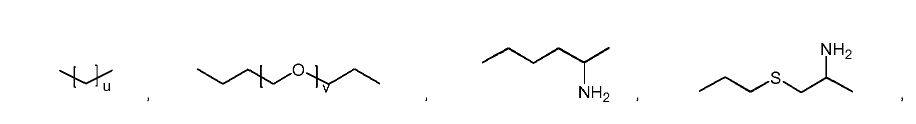 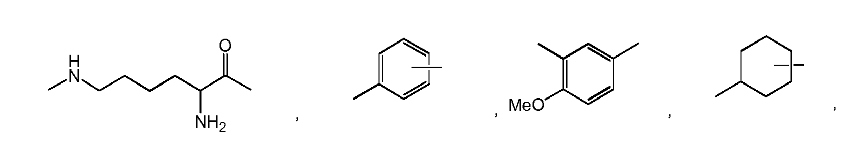 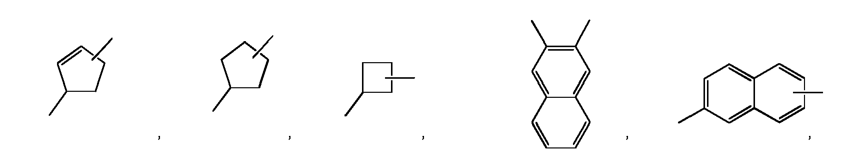 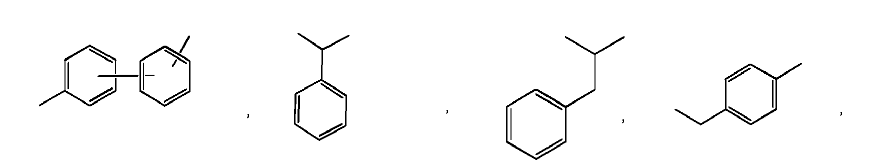 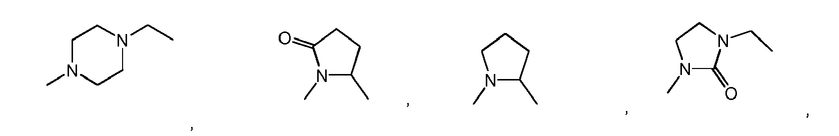 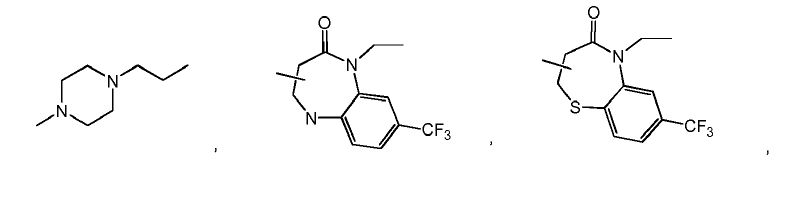 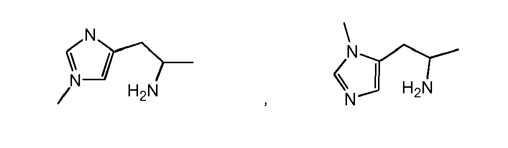 , and 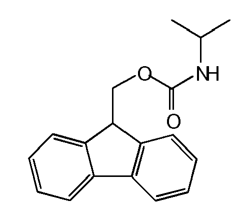 wherein
      u represents an integer ranging from 1 to 18,
      v represents an integer ranging from 1 to 12, R^{Zc1} represents an amino-straight chain C₁-₅ alkyl-carbonyl group or X -straight chain C₁-₅ alkyl group,
      R^{Zc2} represents an amino-straight chain C₁-₅ alkyl-carbonyl amino-straight chain C₁-₅ alkyl group, and
      X represents the same as mentioned above,
      m2 represents an integer ranging from 0 to 15,
      C^{b} represents a bond, -CO- or -SO₂-, or
(ii) a divalent group represented by a formula: -Q^{c'}-C^{b'}-
   wherein
   Q^{c'} represents a formula: -(CH₂)_{m1'}-Z^{c'}-(CH₂)_{m2'}-
   wherein
   m1' represents an integer ranging from 0 to 15,
   Z^{c'} represents 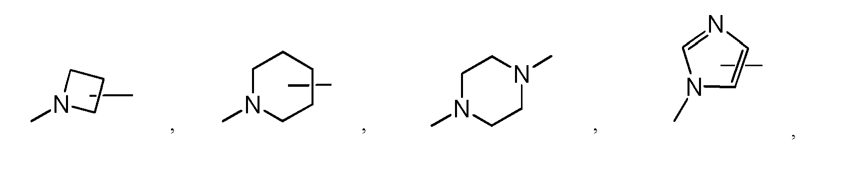 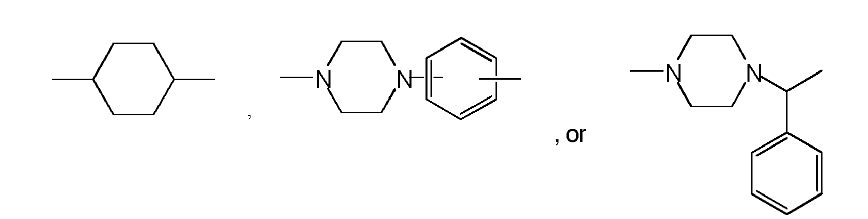 m2' represents an integer ranging from 0 to 15,
   C^{b'} represents -CO-.
   In the embodiment 1, j represents an integer ranging from 0 to 3. Lc can be identical or different when they are repeated. It will be easily understood that if j is 0, (Lc)ⱼ represents a bond.
   In the Embodiment 1, La is a divalent or trivalent group preferably selected from 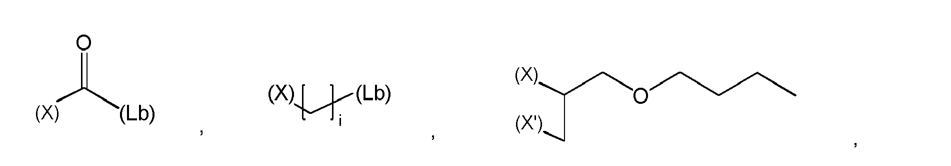 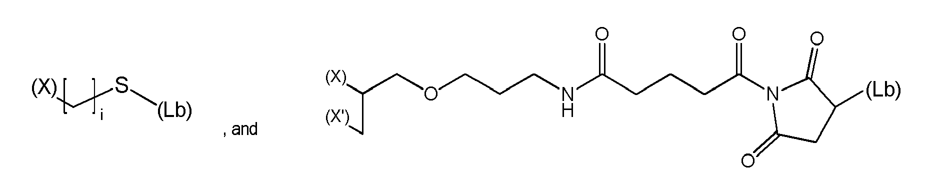 wherein i represents an integer ranging from 1 to 5 and k represents an integer ranging from 1 to 100.

In the embodiment 1, Lb preferably represents
(i) a bond
(ii) a divalent group represented by a formula: -B^{1a}-Q^{b1}-B^{1b}-
   wherein
   B^{1a} and B^{1b} represents -CO-,
   Q^{b1} represents 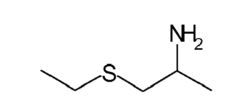 wherein p represents an integer ranging from 2 to 8 or
(iv) a formula: -B^{3a}-Q^{b3}-B^{3b}-
   wherein
   B^{3a} represents 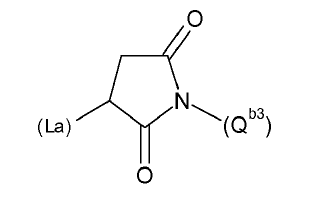 or a bond,
   B^{3b} represents -CO-,
   Q^{b3} represents a divalent group represented by -(CH₂)ₙ₁-Z-(CH₂)ₙ₂-
   wherein
   n1 represents an integer ranging from 0 to 5,
   n2 represents an integer ranging from 0 to 5,
   Z represents a bond,

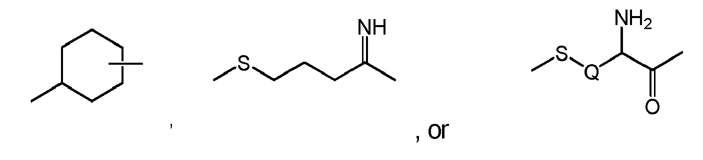

In the embodiment 1, Lc represents
(i) a divalent group represented by a formula: -C^{a}-Q^{c}-C^{b}-
   wherein
   C^{a} represents -NH-,
   Q^{c} represents a divalent group represented by -(CH₂)ₘ₁-Z^{c}-(CH₂)ₘ₂-
   wherein
   m1 represents an integer ranging from 0 to 11 (more preferably 2 to 6)
   Z^{c} represents
   (a) a bond or
   (b) a divalent group selected from -CO-, -O-CO-, -CO-NH-, -NH-CO-, -CO-NH-CO-, -NH-CO-NH-, -CH(NH₂)-, -CH(-NHR^{Zc1})-, -CH(R^{Zc2})-, -CH(OH)-, -CH(COOH)-, -C(=NH)-, -CH(-NHX)- 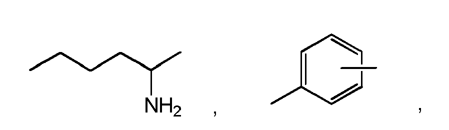 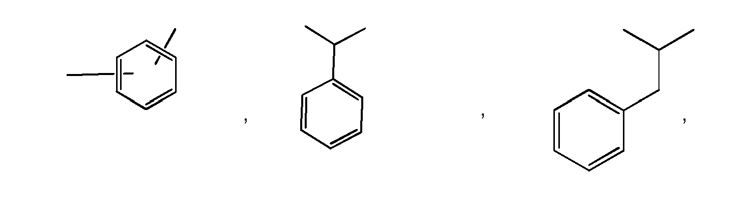 , and 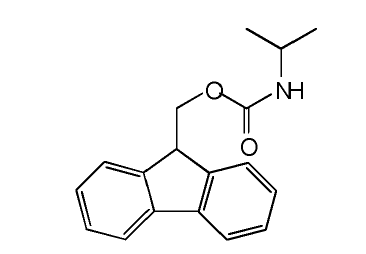 wherein
      u represents an integer ranging from 1 to 18,
      v represents an integer ranging from 1 to 12,
      R^{Zc1} represents an amino-straight chain C₁-₅ alkyl-carbonyl group or X -straight chain C₁-5 alkyl group,
      R^{Zc2} represents an amino-straight chain C₁-₅ alkyl-carbonyl amino-straight chain C₁-₅ alkyl group, and
      X represents the same as mentioned above,
      m2 represents an integer ranging from 0 to 15 (more preferably 1 to 4, further preferably 1 to 2),
      C^{b} represents a bond, -CO-, or -SO₂-,
      or
(ii) a divalent group represented by a formula: -Q^{c'}-C^{b'}-
   wherein
   Q^{c'} represents a divalent group represented by a formula: -(CH₂)_{m1'}-Z^{c'}-(CH₂)_{m2'}-
   wherein
   m1' represents an integer ranging from 0 to 15,
   Z^{c'} represents 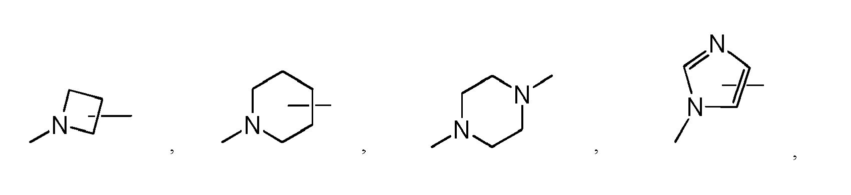 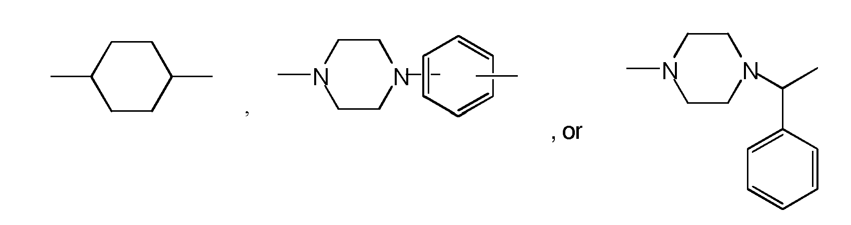 and
   m2' represents 0
   C^{b'} represents -CO-.

In the Embodiment 1,
(Lc)ⱼ preferably represents
(a) a bond or
(b) a divalent group selected from -NH-(CH₂)_{mc1}-CO-, -NH-(CH₂)_{mc2}-CO-NH-(CH₂)_{mc3}-CO-, 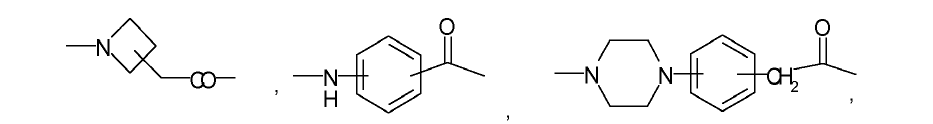 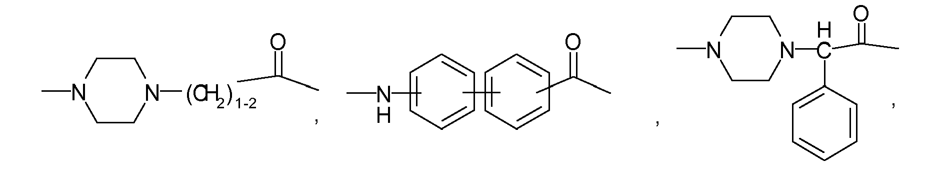 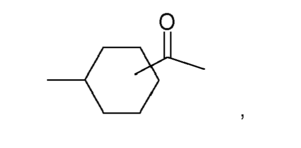 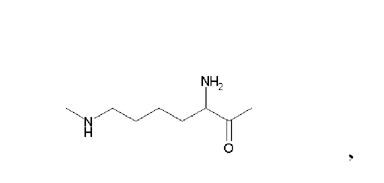 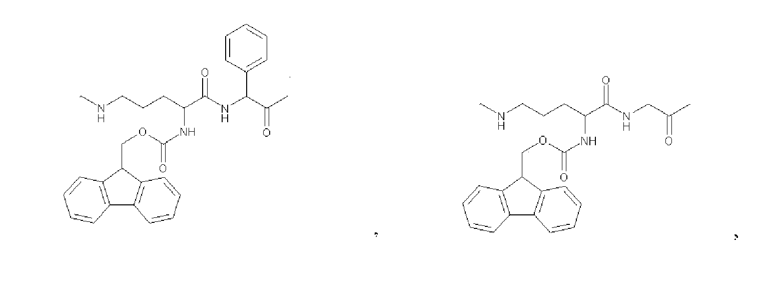 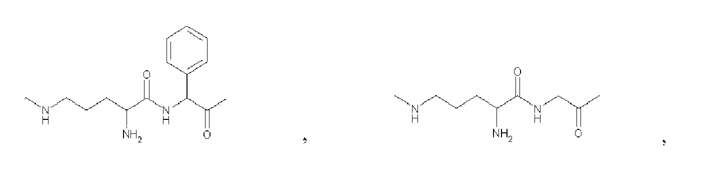 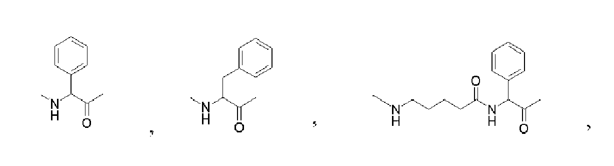 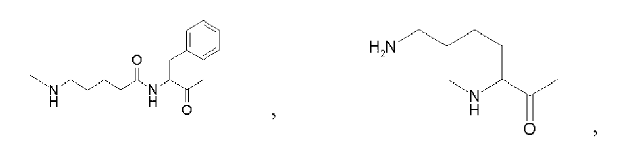 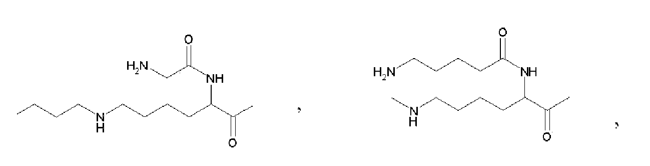 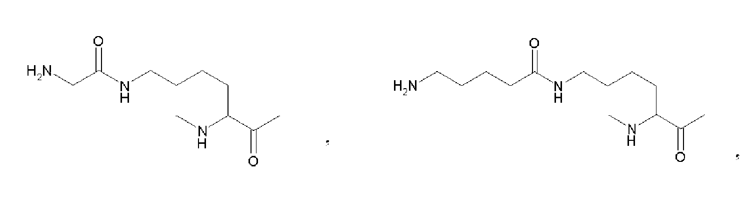 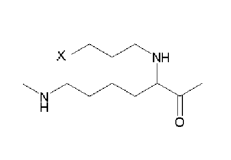 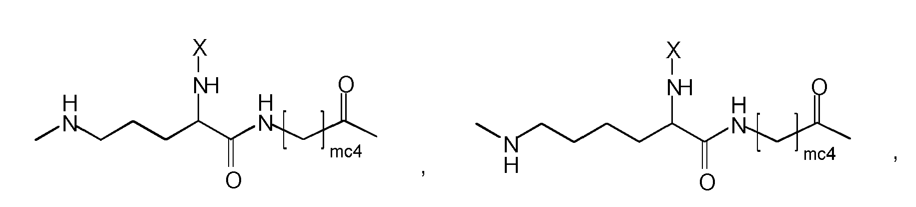 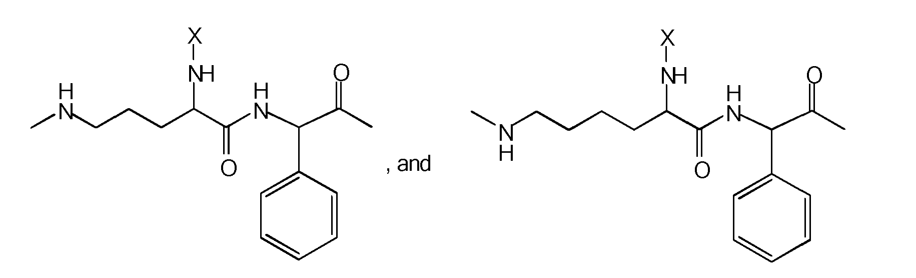 wherein
   mc1 represents an integer ranging from 1 to 11, mc2 and mc3 respectively represent an integer ranging from 1 to 5 (preferably the sum of mc2 and mc3 ranges from 4 to 7), mc4 represents an integer ranging from 1 to 5, and X represents the same meaning as mentioned above).

In the Embodiment 1, preferably
La is a divalent or trivalent group selected from 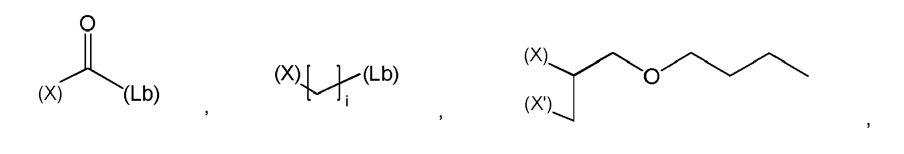 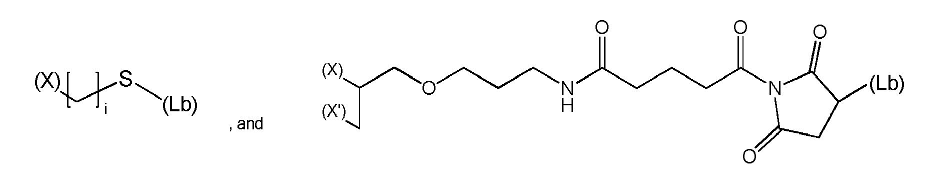 wherein i represents an integer ranging from 1 to 5, k represents an integer ranging from 1 to 100;
Lb is
(i) a bond,
(ii) a divalent group represented by a formula: -B^{1a}-Q^{b1}-B^{1b}-
   wherein
   B^{1a} and B^{1b} represent-CO-,
   Q^{b1} represents a divalent group selected from 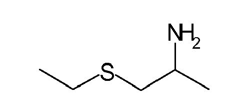 wherein p represents an integer ranging from 2 to 8, or
(iv) a divalent group represented by a formula: -B^{3a}-Q^{b3}-B^{3b}-
   wherein
   B^{3a} represents 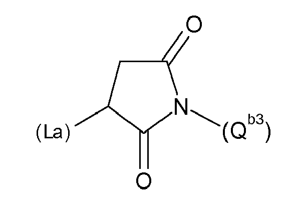 or a bond,
   B^{3b} represents -CO-,
   Q^{b3} represents a divalent group represented by -(CH₂)ₙ₁-Z-(CH₂)ₙ₂-
   wherein
   n1 represents an integer ranging from 0 to 5,
   n2 represents an integer ranging from 0 to 5,
   Z represents a bond, 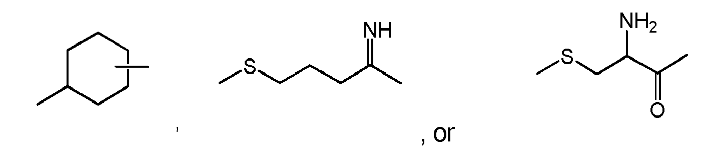 ; and
   Lc represents
(i) a divalent group represented by a formula: -C^{a}-Q^{c}-C^{b}-
   wherein
   C^{a} represents -NH-,
   Q^{c} represents a divalent group represented by a formula: -(CH₂)ₘ₁-Z^{c}-(CH₂)ₘ₂-
   wherein m1 represents an integer ranging from 0 to 11 (more preferably 2 to 6)
   Z^{c} represents
   (a) a bond or
   (b) a divalent group selected from -CO-, -O-CO-, -CO-NH-, -NH-CO-, -CO-NH-CO-, -NH-CO-NH-, -CH(NH₂)-, -CH(-NHR^{Zc1})-, -CH(R^{Zc2})-, -CH(OH)-, -CH(COOH)-, -C(=NH)-, -CH(-NHX)- 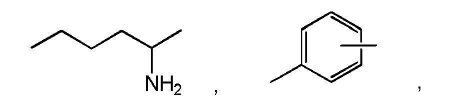 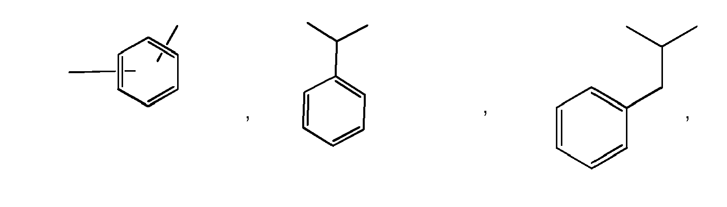 , and 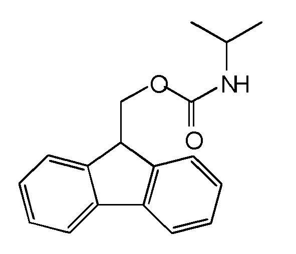 wherein
      u represents an integer ranging from 1 to 18,
      v represents an integer ranging from 1 to 12,
      R^{Zc1} represents an amino-straight chain C₁-₅ alkyl-carbonyl group or X -straight chain C₁-₅ alkyl group,
      R^{Zc2} represents an amino-straight chain C₁-₅ alkyl-carbonyl amino-straight chain C₁-₅ alkyl group, and
      X represents the same as mentioned above,
      m2 represents an integer ranging from 0 to 15 (more preferably from 1 to 4, further preferably 1 or 2)
      C^{b} represents a bond, -CO- or -SO₂-, or
(ii) a divalent group represented by a formula: -Q^{c'}-C^{b'}-
   wherein
   Q^{c'} represents a formula: -(CH₂)_{m1'}-Z^{c'}-(CH₂)_{m2'}-
   wherein
   m1' represents an integer ranging from 0 to 15,
   Z^{c'} represents 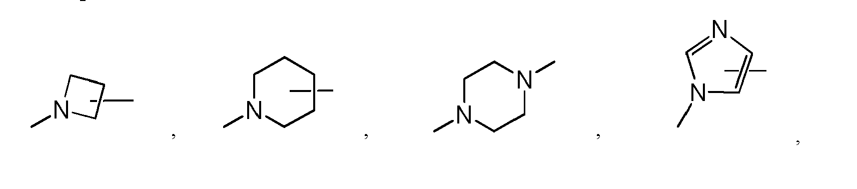 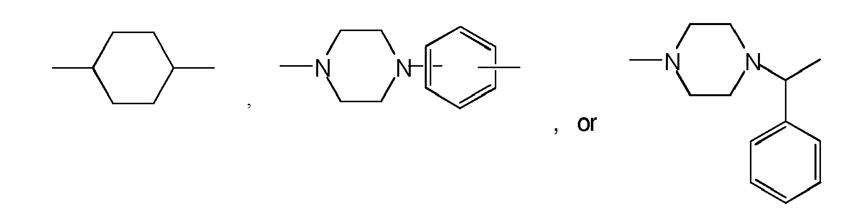 m2' represents an integer ranging from 0 to 15,
   C^{b'} represents -CO-.

In the embodiment 1,
(Lc)ⱼ preferably represents
(a) a bond,
(b) a divalent group selected from -NH-(CH₂)_{mc1}-CO-, -NH-(CH₂)_{mc2}-CO-NH-(CH₂)_{mc3}-CO-, 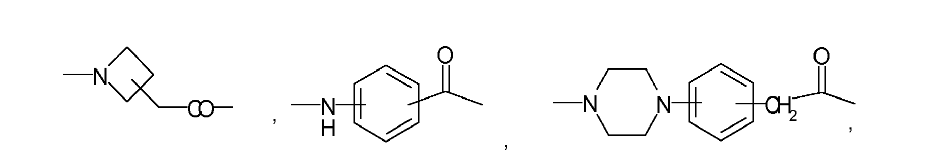 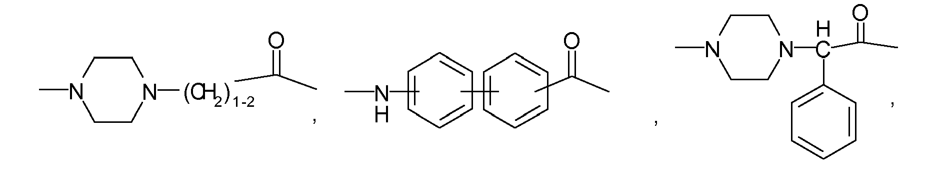 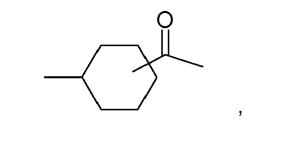 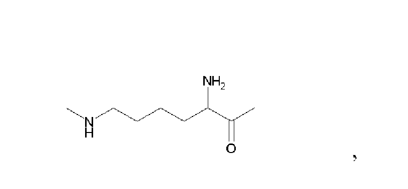 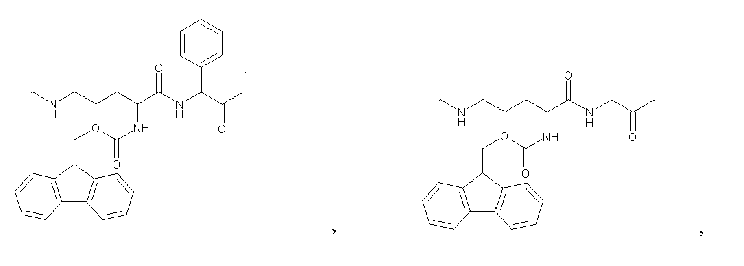 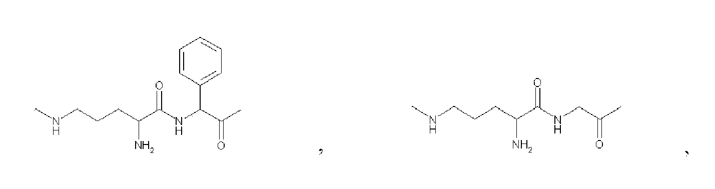 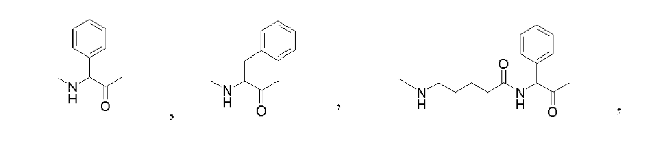 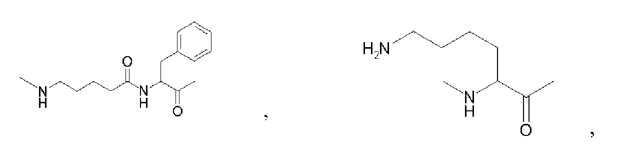 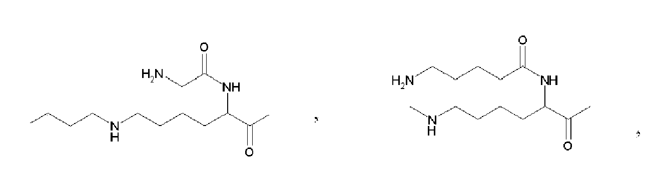 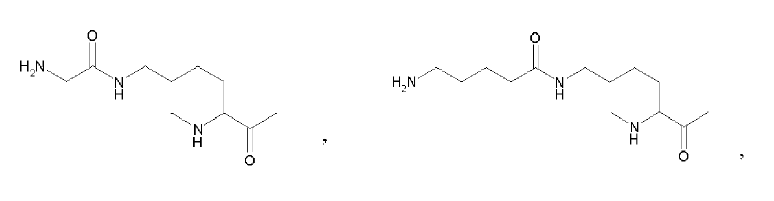 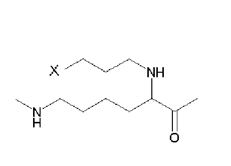 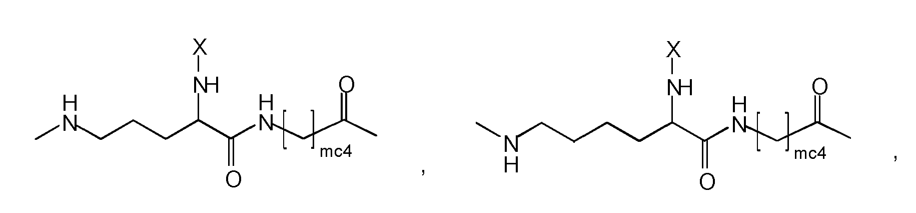 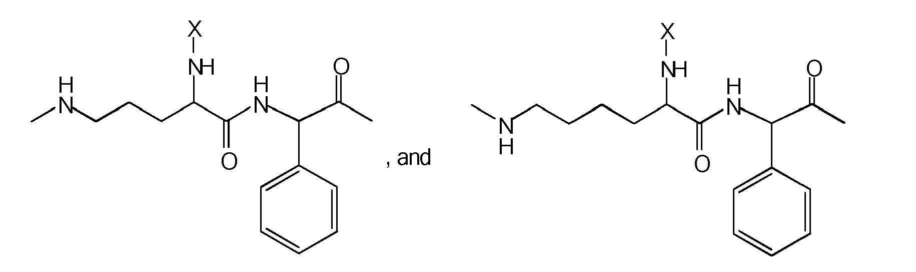 wherein
   mc1 represents an integer ranging from 1 to 11, mc2 and mc3 respectively represent an integer ranging from 1 to 5 (preferably the sum of mc2 and mc3 ranges from 4 to 7), mc4 represents an integer ranging from 1 to 5, and X represents the same meaning as mentioned above,
   or so on.
   If La is represented by 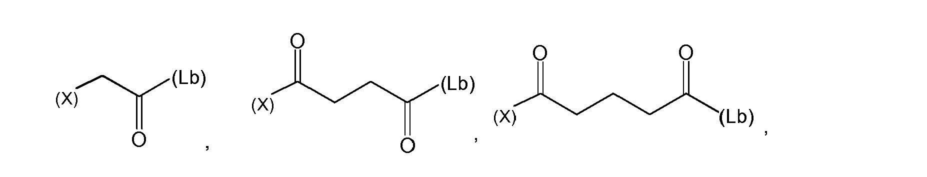 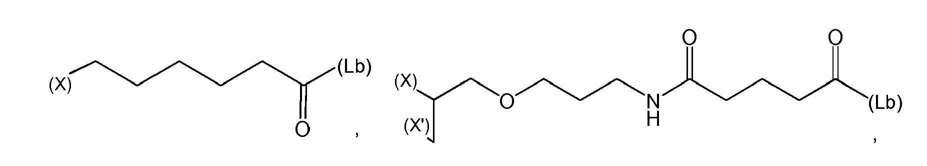 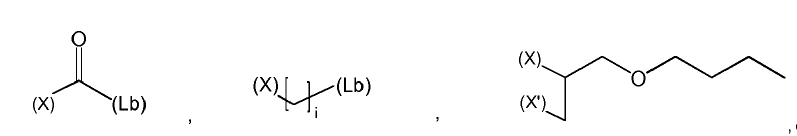 or 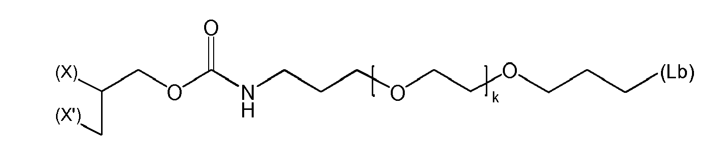 preferably, Lb is a bond.
   If La is represented by 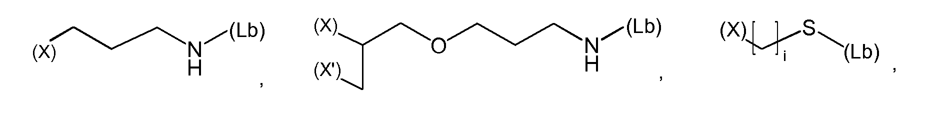 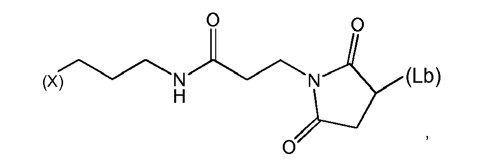 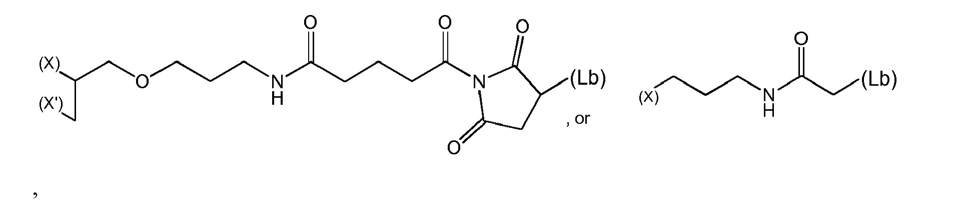 then j is 0, namely (Lc)ⱼ is a bond.

However, if La is represented by 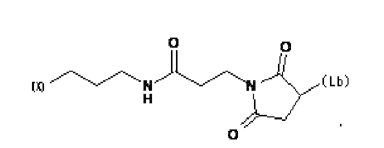 and Lb is represented by a bond, then Lc is preferably not a bond.
Further, preferably if La is represented by 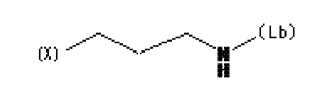 and Lb is a divalent group represented by a formula: -CO-Q^{b2}-B^{2b}-
wherein
Q^{b2} is 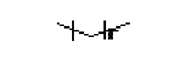 (wherein r is 2)
B^{2b} is 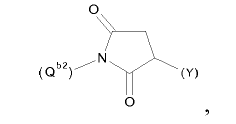 then Lc is not a bond.

A linker represented by L preferably represents a divalent group represented by a formula: 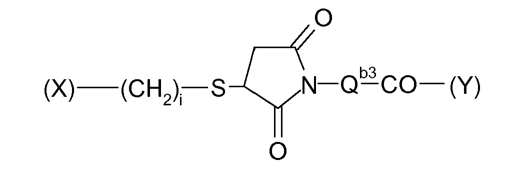 wherein i is an integer ranging from 1 to 5;
Q^{b3} represents a divalent group represented by a formula: -(CH₂)ₙ₁-Z-(CH₂)ₙ₂-
wherein
n1 represents an integer ranging from 0 to 5,
n2 represents an integer ranging from 0 to 5,
Z represents a bond, -O-CO-, -CO-NH-, -CO-O-, -NH-CO-, 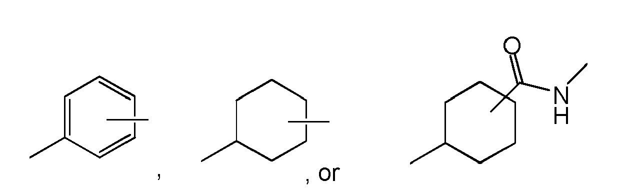 In particular, the provided is a neuromedin U derivative wherein
i is 2 and Z is a bond.

Preferably, a linker represented by L is a divalent group represented by 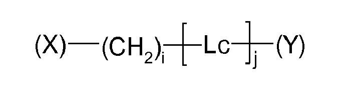 wherein
i represents an integer ranging from 1 to 5;
Lc represents
(i) a divalent group represented by a formula: -C^{a}-Q^{c}-C^{b}-
   wherein
   C^{a} represents -NH-,
   Q^{c} represents a divalent group represented by a formula: -(CH₂)ₘ₁-Z^{c}-(CH₂)ₘ₂-
   wherein
   m1 represents an integer ranging from 0 to 15,
   Z^{c} represents
   (a) a bond or
   (b) a divalent group selected from -CO-, -O-CO-, -CO-NH-, -NH-CO-, -CO-NH-CO-, -NH-CO-NH-, -CH(NH₂)-, -CH(-NHR^{Zc1})-, -CH(R^{Zc2})-, -CH(OH)-, -CH(COOH)-, -C(=NH)-, -CH(-NHX)- 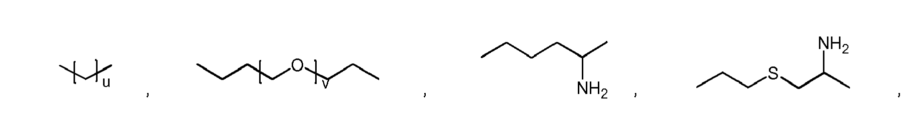 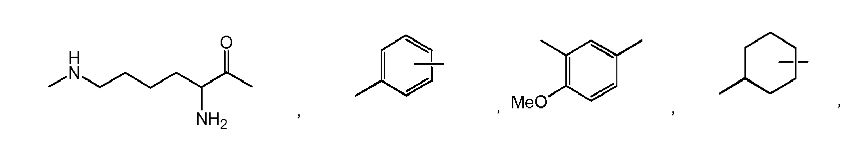 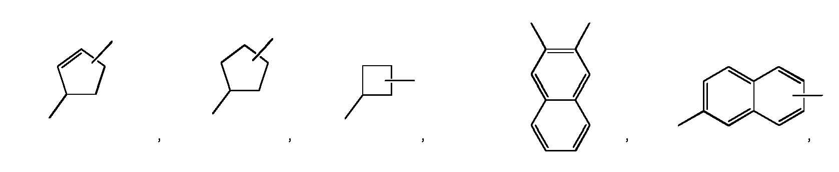 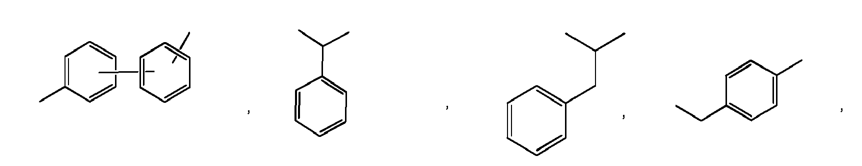 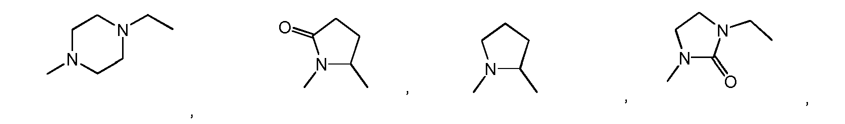 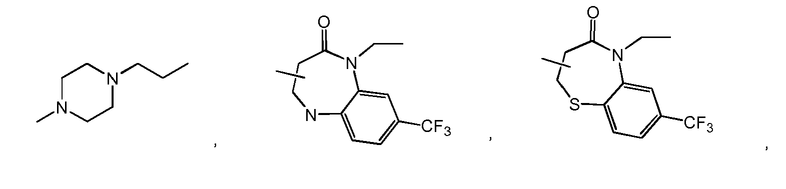 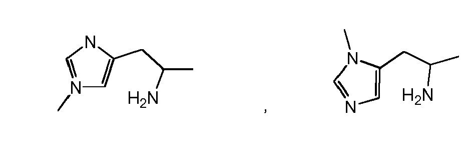 , and 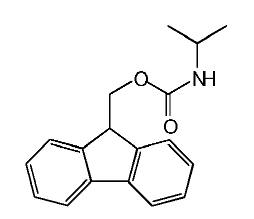 wherein
      R^{Zc1} represents an amino-straight chain C₁-₅ alkyl-carbonyl group or X -straight chain C₁-₅ alkyl group, and
      R^{Zc2} represents an amino-straight chain C₁-₅ alkyl-carbonyl amino-straight chain C₁-₅ alkyl group, and
      X represents the same as mentioned above,
      m2 represents an integer ranging from 0 to 15,
      C^{b} represents a bond, -CO- or -SO₂-, or
(ii) a divalent group represented by a formula: -Q^{c'}-C^{b'}-
   wherein
   Q^{c'} represents a formula: -(CH₂)_{m1'}-Z^{c'}-(CH₂)^{m2'}-
   wherein
   m1' represents an integer ranging from 0 to 15,
   Z^{c'} represents 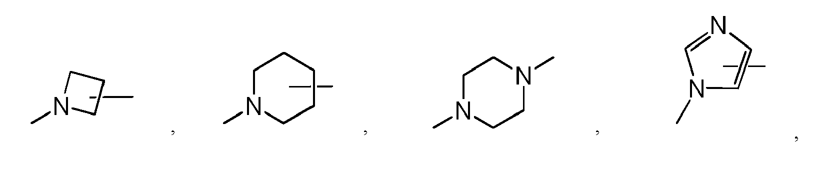 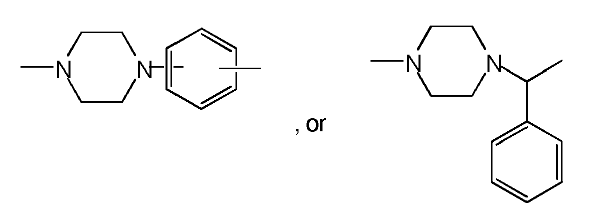 m2' represents an integer ranging from 0 to 15,
   C^{b'} represents -CO-, or -SO₂-;
   j represents an integer ranging from 1 to 3;
   Lc can be identical or different when they are repeated.

In particular, the provided is a neuromedin U derivative wherein
i is 3 and (Lc)ⱼ is a bond,
-NH-(CH₂)_{mc1}-CO-,
-NH-(CH₂)_{mc2}-CO-NH-(CH₂)_{mc3}-CO- 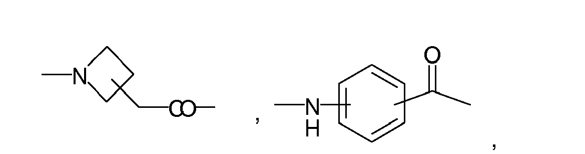 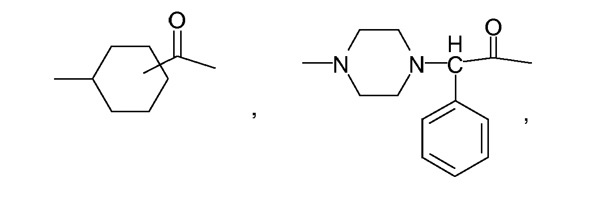 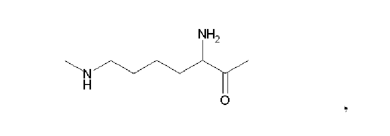 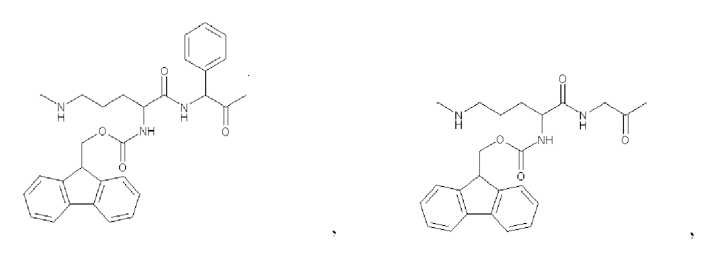 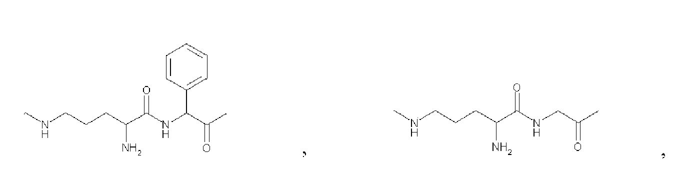 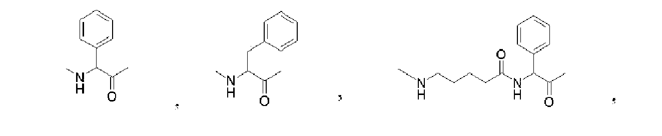 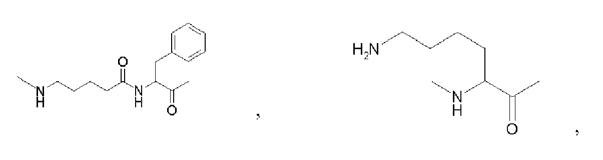 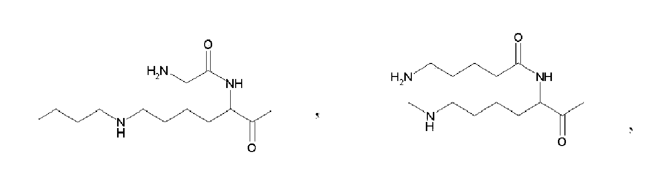 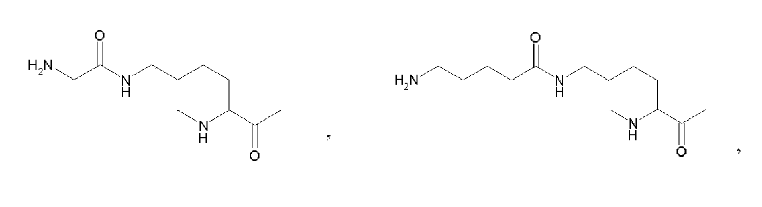 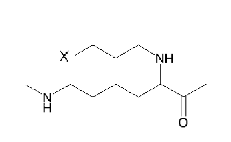 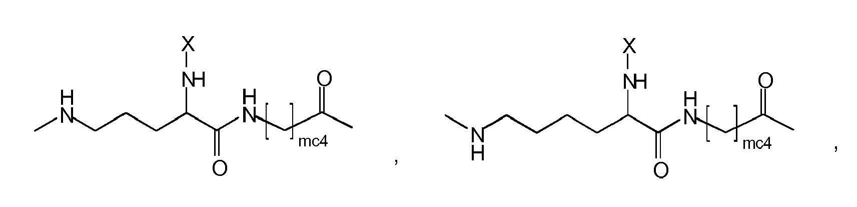 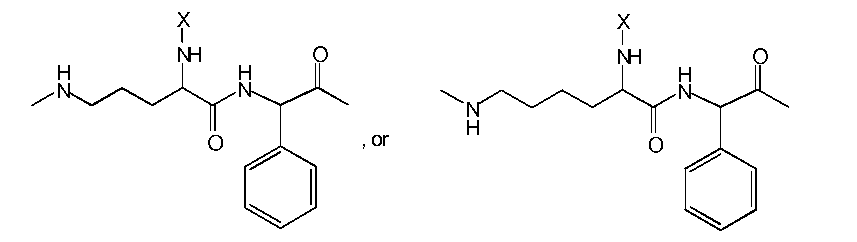 wherein
mc1 represents an integer ranging from 1 to 11, mc2 and mc3 respectively represent an integer ranging from 1 to 5 (preferably the sum of mc2 and mc3 ranges from 4 to 7), mc4 represents an integer ranging from 1 to 5, and X represents the same meaning as mentioned above.

A linker represented by L may also preferably be a divalent group represented by 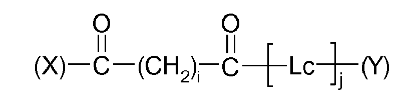 wherein
i represents an integer ranging from 1 to 5;
Lc represents
(i) a divalent group represented by a formula: -C^{a}-Q^{c}-C^{b}-
   wherein
   C^{a} represents -NH-,
   Q^{c} represents a divalent group represented by a formula: -(CH₂)m₁-Z^{c}-(CH₂)ₘ₂-
   wherein
   m1 represents an integer ranging from 0 to 15,
   Z^{c} represents
   a bond, -CO-, -O-CO-, -CO-NH-, -NH-CO-, -CO-NH-CO-, -NH-CO-NH-, -CH(NH₂)-, -CH(OH)-, -CH(COOH)-, -C(=NH)-,

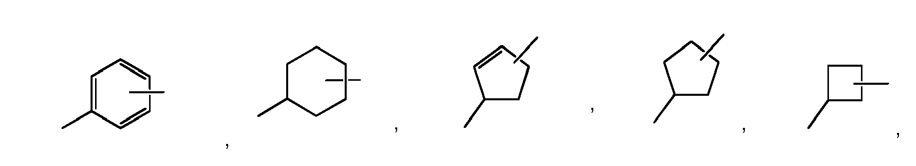

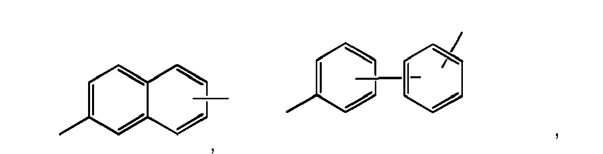

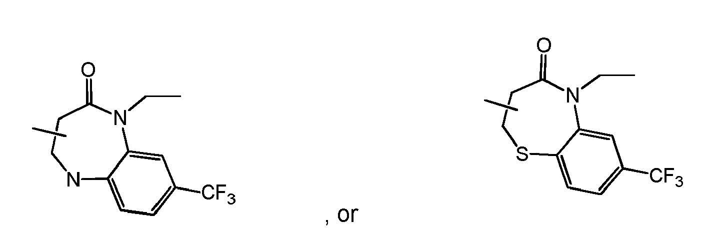

m2 represents an integer ranging from 0 to 15,
C^{b} represents -CO- or -SO₂-, or
(ii) a divalent group represented by a formula: -Q^{c'}-C^{b'}-
   wherein
   Q^{c'} represents a formula: -(CH₂)_{m1'}-Z^{c'}-(CH₂)_{m2'}-
   wherein
   m1' represents an integer ranging from 0 to 15,
   Z^{c'} represents 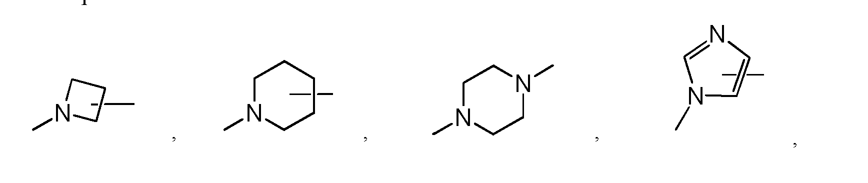 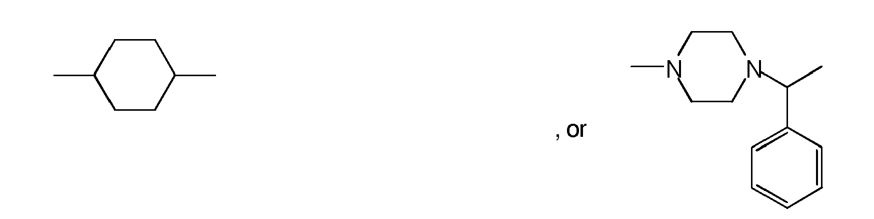 m2' represents an integer ranging from 0 to 15,
   C^{b'} represents -CO-;
   j represents an integer ranging from 1 to 3.
   Lc can be identical or different when they are repeated.

In particular, the provided is a neuromedin U derivative wherein
i is 3 and (Lc)ⱼ is a bond,
-NH-(CH₂)_{mc1}-CO-,
-NH-(CH₂)_{mc2}-CO-NH-(CH₂)_{mc3}-CO- 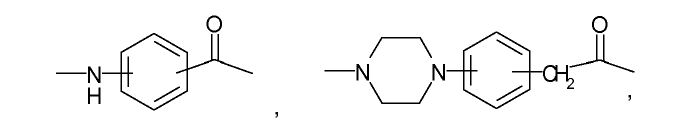 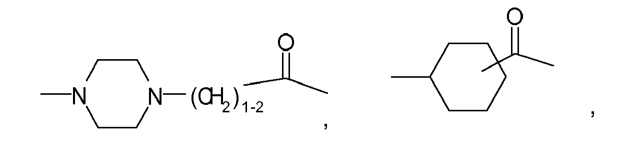 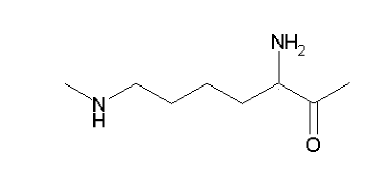 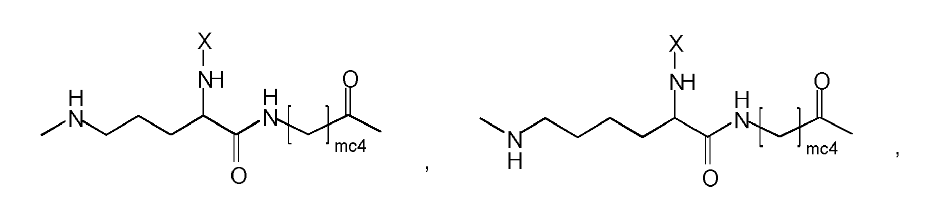 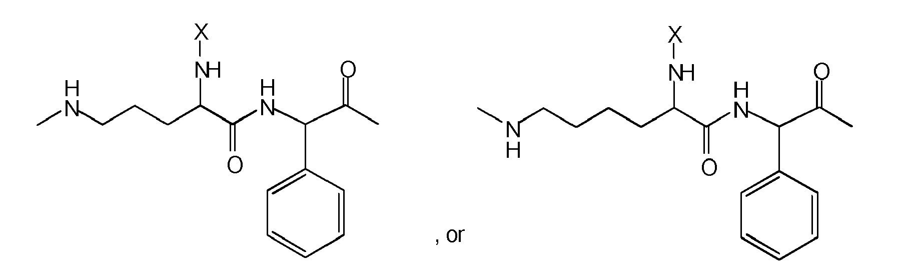 wherein
mc1 represents an integer ranging from 1 to 11, mc2 and mc3 respectively represent an integer ranging from 1 to 5 (preferably the sum of mc2 and mc3 ranges from 4 to 7), mc4 represents an integer ranging from 1 to 5, and X represents the same meaning as mentioned above.

### [Embodiment 2]

In another embodiment of the present invention, a linker represented by L represents a formula:

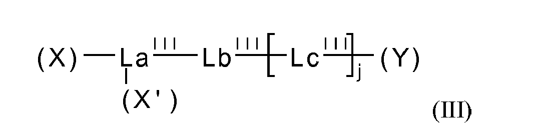

In the Embodiment 2,
La^{III} represents a divalent or trivalent group represented by a formula: 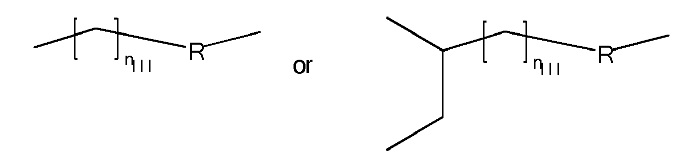 wherein
R represents a bond, -O-, -CO-O-, -O-CO-, -NH-, -CO-, -S-, -S-S-, -SO-, -SO₂-, -NH-SO₂, -SO₂-NH-, -C(=O)-NH-N=CH-, -C(=NH)-NH-, -CO-CH₂-S-, or 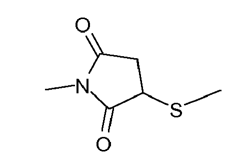 n_{III} represents an integer ranging from 0 to 5;
Lb^{III} represents -(CH₂)ᵢ-(wherein i represents an integer ranging from 1 to 5);
Lc^{III} represents
(i) a divalent group represented by a formula: -NH-Q^{cIII}-C^{bIII}-
   wherein
   Q^{cIII} represents a divalent group represented by a formula: -(CH₂)ₘ₁-Z^{cIII}-(CH₂)ₘ₂-
   wherein
   m1 represents an integer ranging from 0 to 15,
   Z^{cIII} represents
   (a) a bond or
   (b) a divalent group selected from -CO-, -O-CO-, -CO-O-, -CO-NH-, -NH-CO-, -CO-NH-CO-, -NH-CO-NH-, -CH(NH₂)-, -CH(-NHR^{Zc1})-, -CH(R^{Zc2})-, -CH(OH)-, -CH(COOH)-, -C(=NH)-, -S-, -S-S-, -SO-, -SO₂-, -NH-SO₂, -SO₂-NH-, 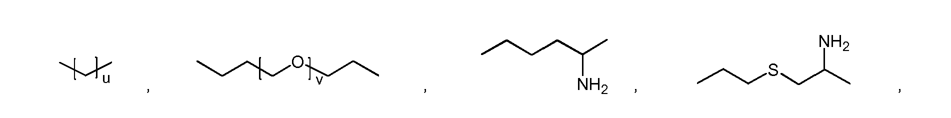 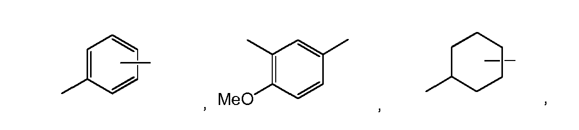 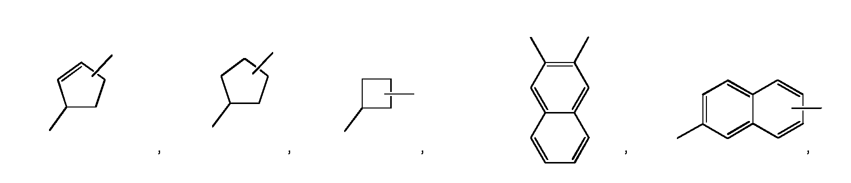 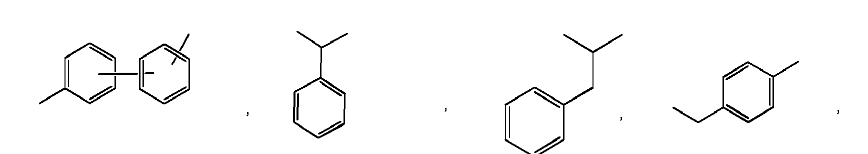 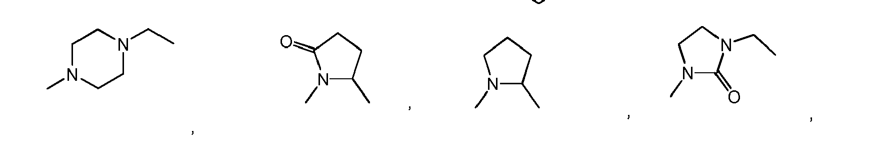 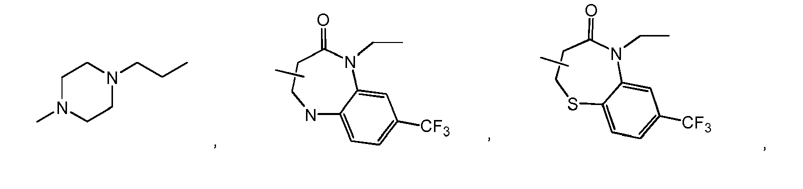 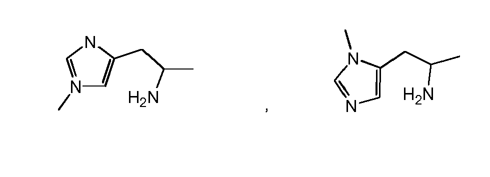 , and 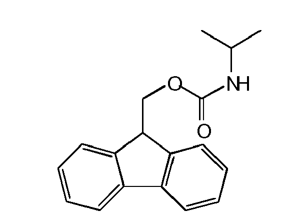 wherein
      u represents an integer ranging from 1 to 18,
      v represents an integer ranging from 1 to 12,
      R^{Zc1} represents an amino-straight chain C₁-₅ alkyl-carbonyl group or X -straight chain C₁-₅ alkyl group (X represents the same meaning as above),
      R^{Zc2} represents an amino-straight chain C₁-₅ alkyl-carbonyl amino-straight chain C₁-₅ alkyl group,
      m2 represents an integer ranging from 0 to 15,
      C^{bIII} represents a bond, -CO- or -SO₂-, or
(ii) a divalent group represented by a formula: ₋Q^{cIII'}-C^{bIII'}-
   wherein
   Q^{cIII'} represents a formula: -(CH₂)_{m1'}-Z^{cIII'}-(CH₂)_{m2'}
   wherein
   m1' represents an integer ranging from 0 to 15,
   Z^{cIII'} represents a divalent group selected from 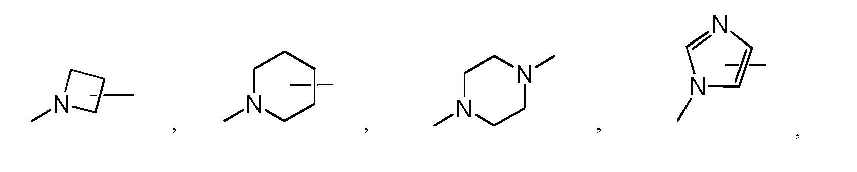 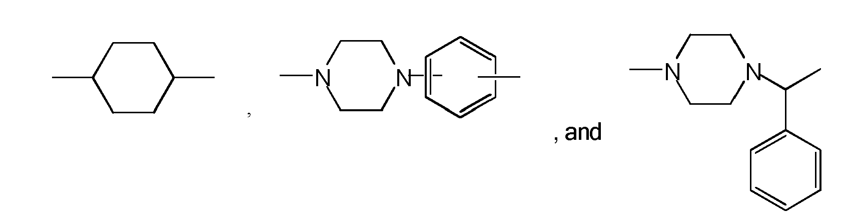 m2' represents an integer ranging from 0 to 15,
   C^{bIII'} represents -CO- or -SO₂-; and
   j^{III} represents an integer ranging from 1 to 3.

In the Embodiment 2, preferably, La^{III} represents a divalent or trivalent group represented by a formula: 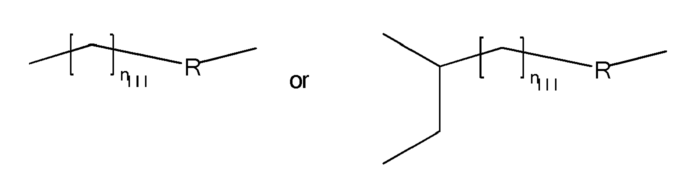 wherein
R represents -O-
n_{III} represents an integer of 1.

In the Embodiment 2, preferably, Lb^{III} represents -(CH₂)ᵢ- (wherein i represents an integer of 3).
In the Embodiment 2, preferably Lc^{III} represents
(i) a divalent group represented by a formula: -NH-Q^{cIII}-C^{bIII}-
   wherein
   Q^{cIII} represents a divalent group represented by a formula: -(CH₂)ₘ₁-Z^{cIII}-(CH₂)ₘ₂-
   wherein
   m1 represents an integer of 0,
   Z^{cIII} represents
   (a) a bond or
   (b) a divalent group selected from -CO-, -CH(-NHR^{Zc1})-, -CH(R^{Zc2})-, 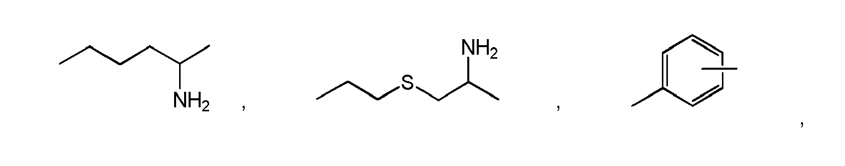 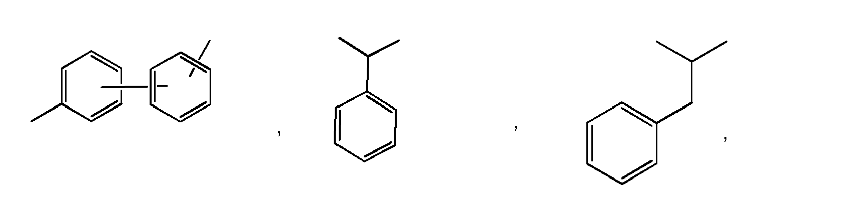 , and 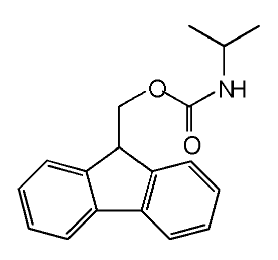 wherein
      u represents an integer ranging from 1 to 18,
      v represents an integer ranging from 1 to 12,
      R^{Zc1} represents an amino-straight chain C₁-₅ alkyl-carbonyl group or X-straight chain C₁-₅ alkyl group (X represents the same meaning as above),
      R^{Zc2} represents an amino-straight chain C₁-₅ alkyl-carbonyl amino-straight chain C₁-₅ alkyl group,
      m2 represents an integer ranging from 0 to 10,
      C^{bIII} represents a bond, -CO- or -SO₂-, or
(ii) a divalent group represented by a formula: -Q^{cIII'}-C^{bII'}-
   wherein
   Q^{cIII'} represents a formula: -(CH₂)_{m1'}-Z_{cIII'}-(CH₂)_{m2'}-
   wherein
   m1' represents 0,
   Z^{cIII'} represents a divalent group selected from 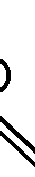 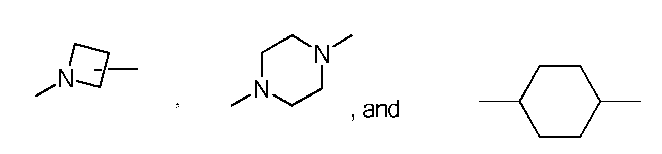 m2' represents an integer ranging from 0 to 2,
   C^{bIII} represents -CO- or -SO₂-; and
   j^{III} represents an integer of 1 to 2.

In the Embodiment 2, preferably La ^{III} is a divalent or trivalent group represented by a formula: 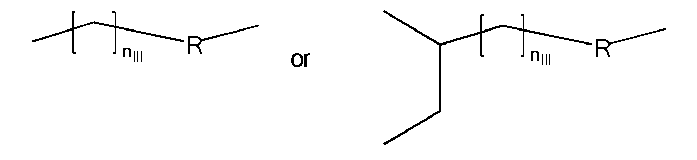 wherein
R represents -O-,
n _{III} represents an integer of 1;
Lb^{III} is -(CH₂)ⱼ- (wherein i represents an integer of 3); and
L_{c}^{III} is
(i) a divalent group represented by a formula: - NH-Q^{cIII}-C^{bIII}-
   wherein
   Q^{cIII} represents a divalent group represented by a formula: -(CH₂)ₘ₁-Z^{cIII}-(CH₂)ₘ₂-
   wherein
   m1 represents an integer of 0,
   Z^{cIII} represents
   (a) a bond or
   (b) -CO-, -CH(-NHR^{Zc1})-, -CH(R^{Zc2})-, 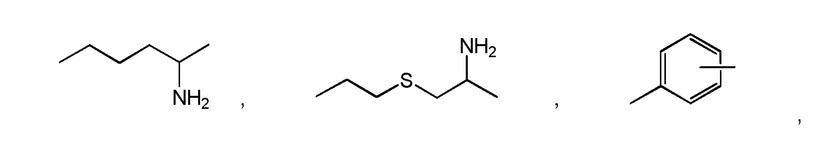 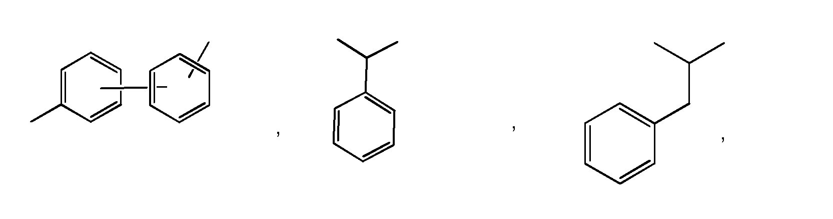 , and 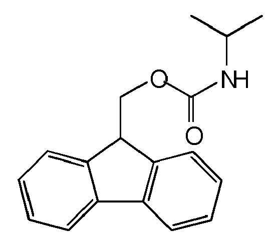 wherein
      u represents an integer ranging from 1 to 18,
      v represents an integer ranging from 1 to 12,
      R^{Zc1} represents an amino-straight chain C₁-₅ alkyl-carbonyl group or X-straight chain C₁-₅ alkyl group (X represents the same meaning as above),
      R^{Zc2} represents an amino-straight chain C₁-₅ alkyl-carbonyl amino-straight chain C₁-₅ alkyl group,
      m2 represents an integer ranging from 0 to 10,
      C^{bIII} represents a bond, -CO- or -SO₂-, or
(ii) a divalent group represented by a formula: -Q^{cIII}-C^{bIII'}-
   wherein
   Q^{cIII'} represents a formula: -(CH₂)_{m1'}-Z^{cIII'}-(CH₂)_{m2'}-
   wherein
   m1' represents 0,
   Z^{cIII'} represents a divalent group selected from 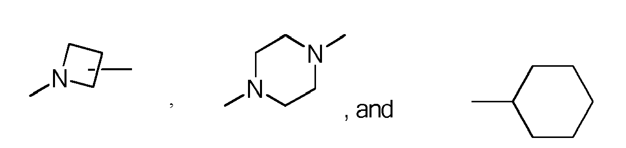 m2' represents an integer ranging from 0 to 2,
   C^{bIII} represents -CO- or -SO₂-; and
   j^{III} represents an integer of 1 to 2.

In the Embodiment 2, the distance from the nitrogen atom closest to the Lb in the Lc to the nitrogen atom of the N-terminus ofneuromedin U ranges from 3.5 to 30Å and preferably from 3.5 to 15Å

In the Embodiment 2, preferably if Lc represents
(i) a divalent group represented by a formula: -NH-Q^{cIII}-C^{bIII}-
   wherein
   Q^{cIII} represents a divalent group represented by a formula: -(CH₂)ₘ₁- (m1 is an integer ranging from 0 to 15),
   C^{bIII} represents a bond, -CO- or -SO₂-,
   The distance from the nitrogen atom ofNH in (i) the formula: -NH-Q^{cIII}-C^{bIII}- to the nitrogen atom of the N-terminus of neuromedin U ranges from 3.5 to 7.0Å.

In the Embodiment 2, preferably if Lc^{III} is
(i) a divalent group represented by a formula: -NH-Q^{cIII}-C^{bIII}-
   wherein
   Q^{cIII} represents a formula: -(CH₂)ₘ₁-Z^{cIII}-(CH₂)ₘ₂-
   wherein
   m1 represents an integer ranging from 0 to 10,
   Z^{cIII} represents a divalent group selected from
   -CO-, -O-CO-, -CO-O-, -CO-NH-, -NH-CO-, -CO-NH-CO-, -NH-CO-NH-, -CH(NH₂)-, -CH(-NHR^{Zc1})-, -CH(R^{Zc2})-, -CH(OH)-, -CH(COOH)-, -C(= NH)-, -S-, -S-S-, -SO-, -SO₂-, -NH-SO₂, -SO₂ -NH-, 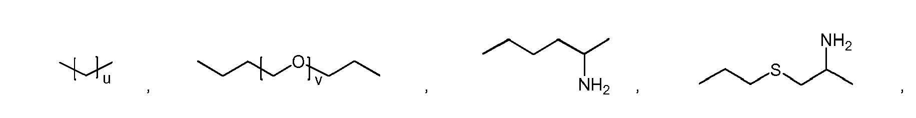 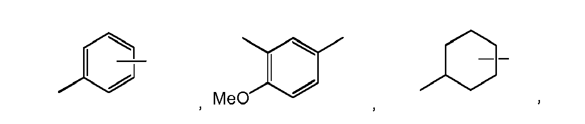 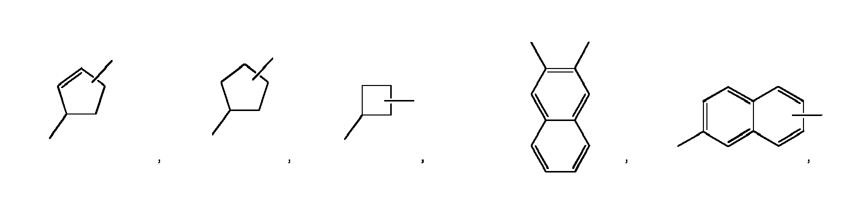 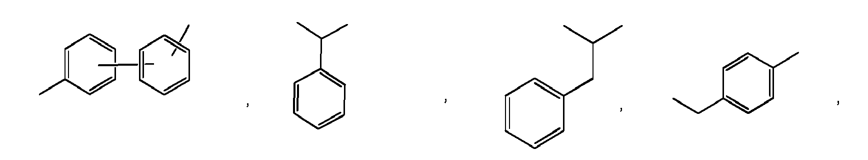 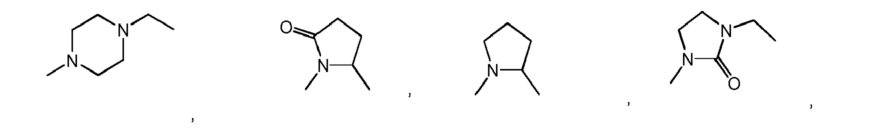 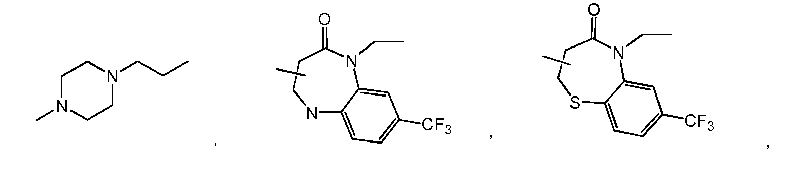 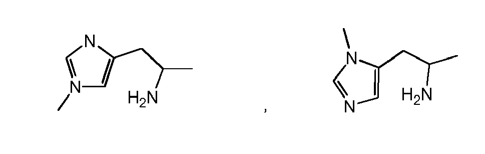 , and 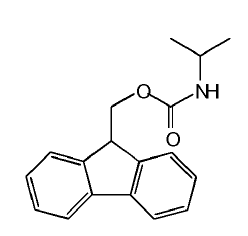 wherein
   u represents an integer ranging from 1 to 10,
   v represents an integer ranging from 1 to 10,
   R^{Zc1} represents an amino-straight chain C₁-₅ alkyl-carbonyl group or X-straight chain C₁-₅ alkyl group (X represents the same meaning as above),
   R^{Zc2} represents an amino-straight chain C₁-₅ alkyl-carbonyl amino-straight chain C₁-₅ alkyl group,
   m2 represents an integer ranging from 0 to 5,
   C^{bIII} represents a bond, -CO- or -SO₂,
   the distance from the nitrogen atom ofNH in the formula: - NH-Q^{c}-C^{b}- to the atom which is closest to the -(CH₂)ₘ₁- part in the Z^{c} ranges from 3.5 to 10 Å and the distance from the atom which is closest to the -(CH₂)ₘ₁- part in the Z^{c} to the nitrogen atom of the N terminus of neuromedin U ranges from 3.5 to 7.0 Å.

Further, in the Embodiment 2, preferably if Lc is
(ii) a divalent group represented by the formula: -NH-Q^{cIII'}-C^{bIII'}-
   wherein
   Q^{cIII'} represents a formula: -(CH₂)_{m1'}-Z^{cIII'}-(CH₂)_{m2'}-
   wherein m1' represents an integer ranging from 0 to 15,
   Z^{cIII'} represents 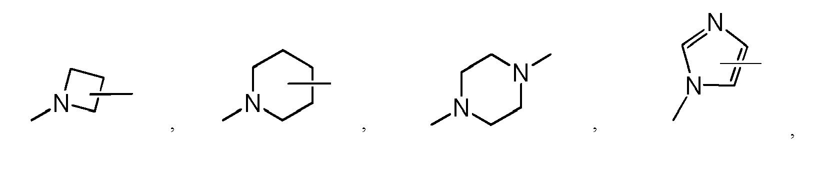 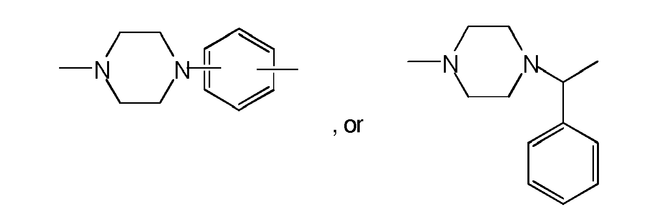 m2' represents an integer ranging from 0 to 15,
   C^{bIII'} represents a bond, -CO- or -SO₂,
   The distance from the nitrogen closest to the Lb to the nitrogen atom of the N-terminus of neuromedin U in 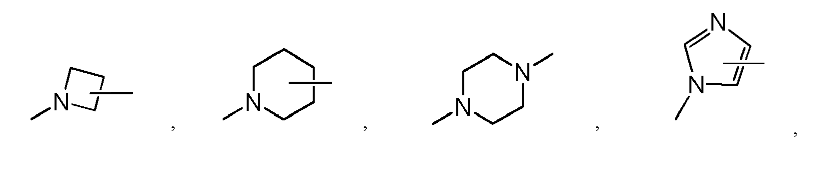 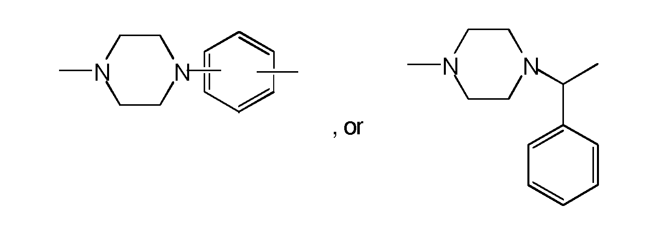 ranges from 5 to 10 Å.

In the Embodiment 2, examples of (Lc)j preferably include those listed as examples in the aforementioned Embodiment 1.

The inter-atomic distance is an inter-atomic distance in the three-dimensional stable structure that has been output when performing energy stabilization calculations as an extended structure using a compound or a three-dimensional molecular model for the compound using commercial molecular modeling and calculation software (e.g., Gaussian, MOPAC, AMBER, CHARMM, MOE, Insight, etc. that are sold by Ryoka Systems Inc.). With each software, parameters are pre-determined such that the inter-atomic distance corresponds to the estimated inter-atomic distance measured by X-ray crystal structural analysis (for example, Cambridge Structural Database, etc.). In the case of molecules consisting of approximately 20 regular heavy atoms, the error is less than 0.1 Å. (See J. Am. Chem. Soc, 106, 765-784 in the case of AMBER).

### [Embodiment 3]

In the aforementioned Embodiments 1 and 2, the linker which was branched into two branches and which can connect two methoxypolyethylene glycol molecules were explained. In one other embodiment of the present invention, the linker which was branched into many branches and by so doing, and could connect numerous methoxypolyethylene glycols was used.
For example, a four-branched linker structure can be easily designed by a branching two branched linker alkylene portion.
For example, if a neuromedin U derivative of the present invention having two branches has the following structure, 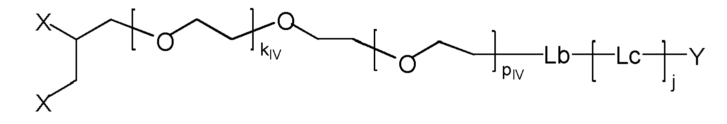 , or 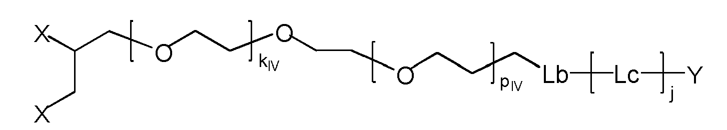 a four-branched linker structure can be designed as follows. 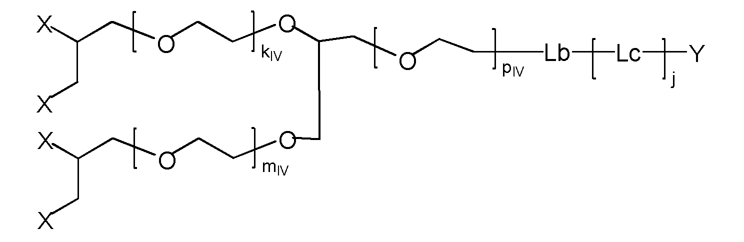 , or

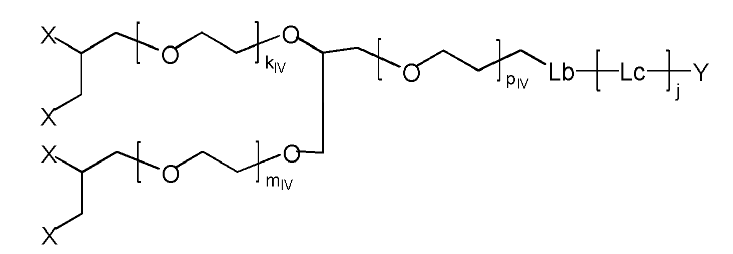

Further, if a neuromedin U derivative of the present invention having two branches has the following structure, a four-branched linker structure can be designed as follows. 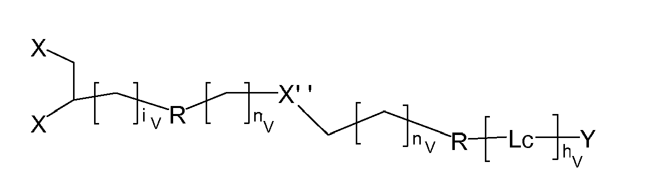 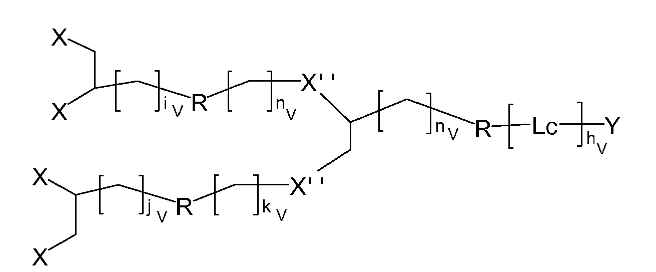 Similarly, 6-branched, 8-branched, 10-branched to 32-branched linkers can be designed. These linkers can also be used in the neuromedin U derivatives of the present invention.

A neuromedin U derivative having a 4-branched linker will be explained below.

### [Embodiment 3-1]

Another embodiment of the present invention provides a neuromedin U derivative wherein a methoxypolyethylene glycol is bound via a linker to the polypeptide which contains at least 8 amino acids of the C-terminus of an amino acid sequence of neuromedin U and which consists of the same or substantially the same amino acid sequence as the amino acid sequence of neuromedin U which is represented by a formula: 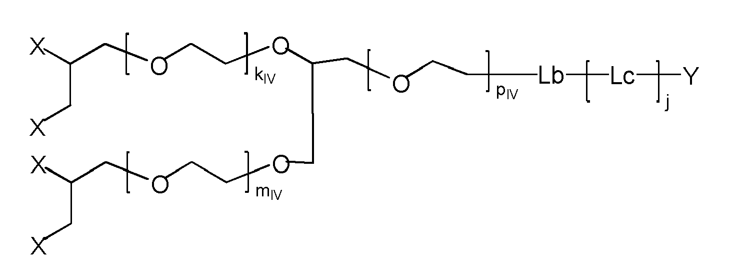 , or 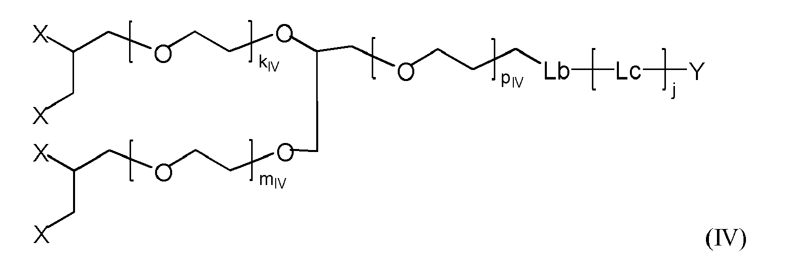 wherein
Y represents a polypeptide which contains at least 8 amino acids of an amino acid sequence at the C-terminus ofneuromedin U and which consists of the same or substantially the same amino acid sequence as that of neuromedin U; X represents a methoxypolyethylene glycol (here, methoxypolyethylene glycol represented by plural Xs can be identical or different);
Lb represents
(i) a bond,
(ii) a divalent group represented by a formula: -B^{1a}-Q^{b1}-B^{1b}-
   wherein
   B^{1a} and B^{1b} represent -CO-,
   Q^{b1} represents a divalent group selected from 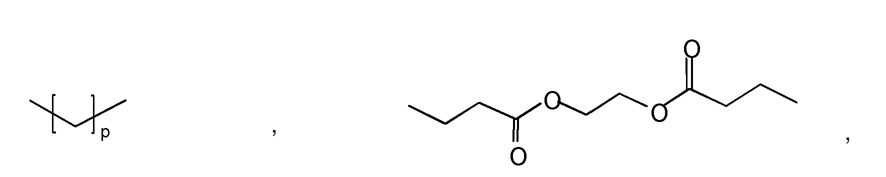 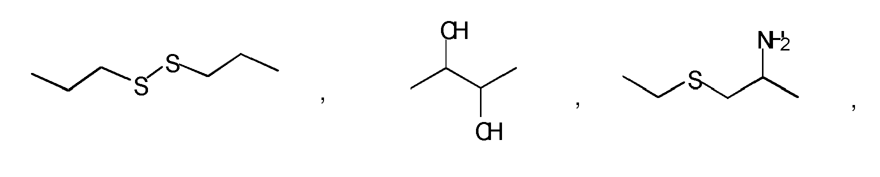 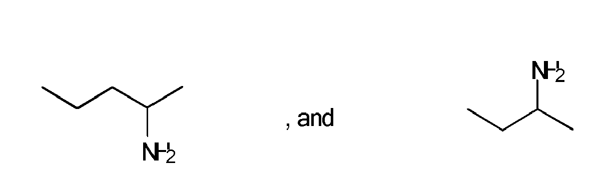 wherein p represents an integer ranging from 2 to 8,
(iii) a divalent group represented by a formula: -B^{2a}-Q^{b2}-B^{2b}-
   wherein
   B^{2a} represents -CO-,
   B^{2b} represents 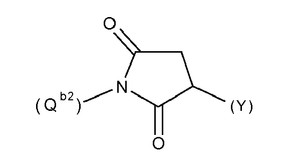 Q^{b2} represents a divalent group selected from 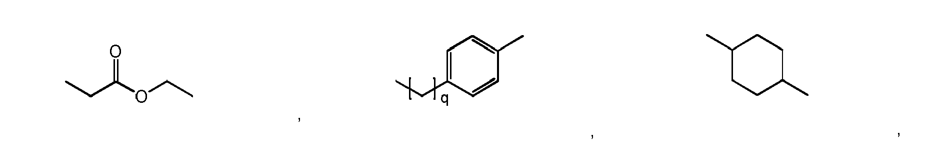 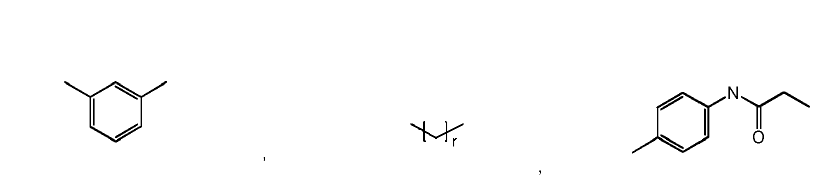 , and 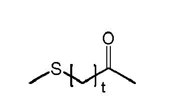 wherein q represents an integer ranging from 3 to 10, r represents an integer ranging from 1 to 10, and t represents an integer ranging from 1 to 10, or
(iv) a divalent group represented by a formula: -B^{3a}-Q^{b3}-B^{3b}-
   wherein
   B^{3a} represents 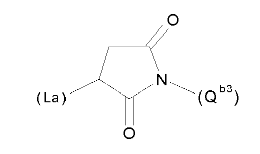 or a bond,
   B^{3b} represents -CO-,
   Q^{b3} represents a divalent group represented by -(CH₂)ₙ₁-Z-(CH₂)ₙ₂-
   wherein
   n1 represents an integer ranging from 0 to 5,
   n2 represents an integer ranging from 0 to 5,
   Z represents a bond, -O-CO-, -CO-NH-, -CO-O-, -NH-CO-, 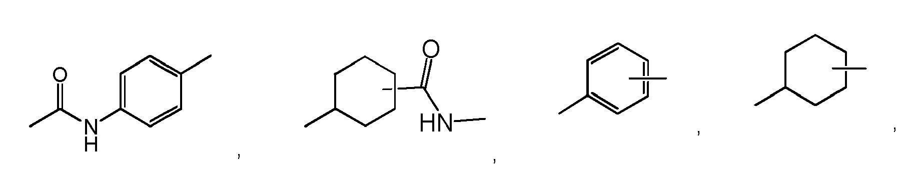 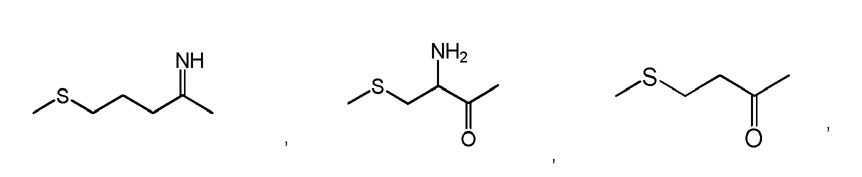 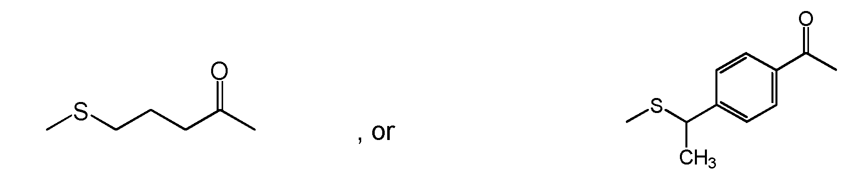
Lc represents
(i) a divalent group represented by a formula: -C^{a}-Q^{c}-C^{b}-
   wherein
   C^{a} represents -NH-,
   Q^{c} represents a divalent group represented by a formula: -(CH₂)ₘ₁-Z^{c}-(CH₂)ₘ₂-
   (wherein
   m1 represents an integer ranging from 0 to 15,
   Z^{c} represents
   (a) a bond or
   (b) a divalent group selected from -CO-, -O-CO-, -CO-O-, -CO-NH-, -NH-CO-, -CO-NH-CO-, -NH-CO-NH-, -CH(NH₂)-, -CH(-NHR^{Zc1})-, -CH(R^{Zc2})-, -CH(OH)-, -CH(COOH)-, -C(=NH)-, -CH(NHX)-, 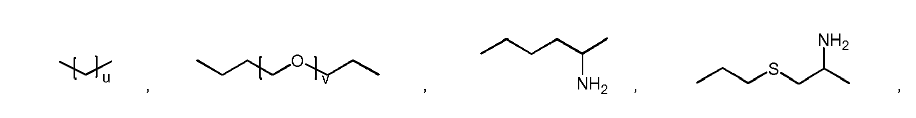 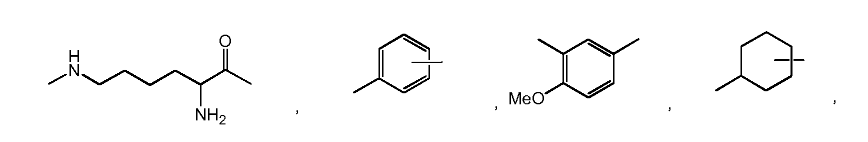 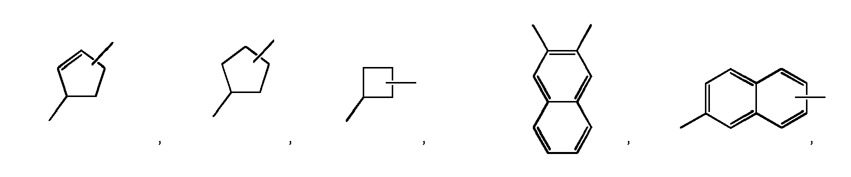 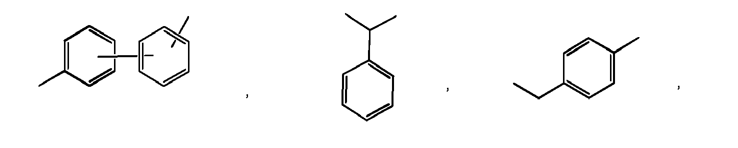 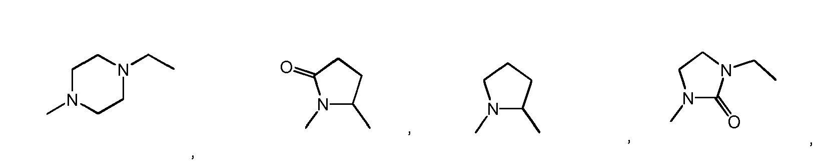 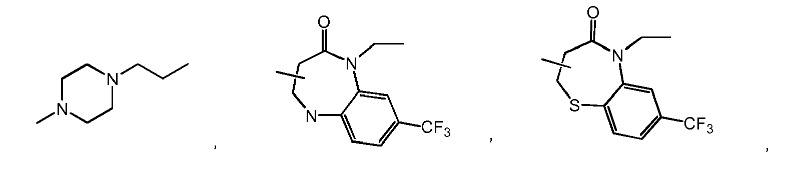 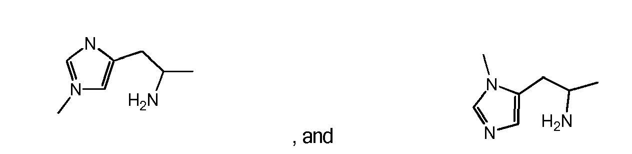
   (wherein
   u represents an integer ranging from 1 to 18,
   v represents an integer ranging from 1 to 12,
   R^{Zc1} represents an amino-straight chain C₁-₅ alkyl-carbonyl group or X-straight chain C₁-₅ alkyl group,
   R^{Zc2} represents an amino-straight chain C₁-₅ alkyl-carbonyl amino-straight chain C₁-₅ alkyl group, and
   X represents the same as mentioned above),
   m2 represents an integer ranging from 0 to 15),
   C^{b} represents a bond, -CO-, or -SO₂-, or
(ii) a formula: -Q^{c'}-C^{b'}-
   wherein
   Q^{c'} represents a divalent group represented by a formula: -(CH₂)_{m1'}-Z^{c'}-(CH₂)_{m2'}-
   (wherein
   m1' represents an integer ranging from 0 to 15,
   Z^{c'} represents 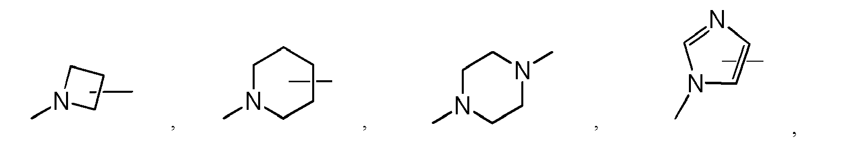 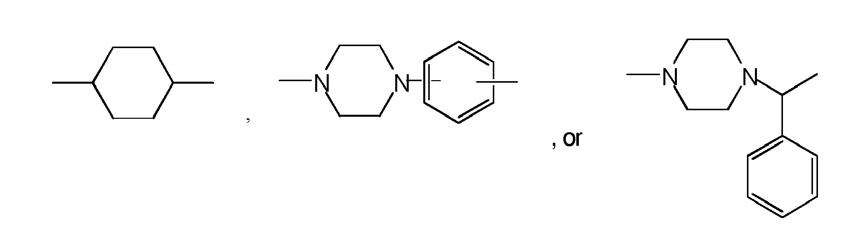 and
   m2' represents an integer ranging from 0 to 15)
   C^{b'} represents -CO- or -SO₂-;
   k_{IV} represents an integer ranging from 1 to 100;
   m_{IV} represents an integer ranging from 1 to 100;
   p_{IV} represents an integer ranging from 1 to 100; and
   j represents an integer ranging from 0 to 3.

In particular, provided is a neuromedin U derivative wherein Lb is a bond and (Lc)ⱼ is as follows:

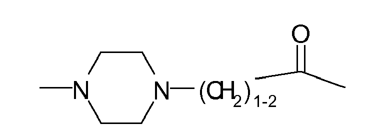

### [Embodiment 3-2]

Another embodiment of the present invention provides a neuromedin U derivative wherein a methoxypolyethylene glycol is bound via a linker to the polypeptide which contains at least 8 amino acids at the C-terminus of an amino acid sequence of neuromedin U and which consists of the same or substantially the same amino acid sequence as the amino acid sequence of neuromedin U which is represented by a formula: 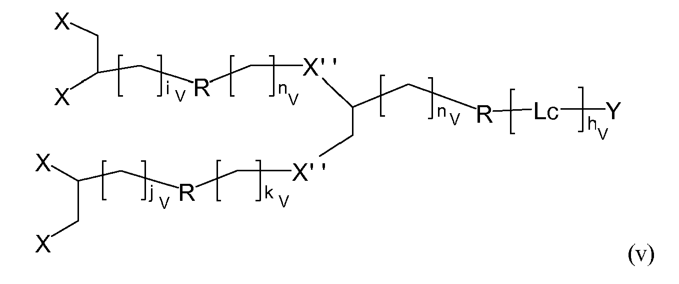 wherein
Y represents a polypeptide which contains at least 8 amino acids of an amino acid sequence at the C-terminus ofneuromedin U and which consists of the same or substantially the same amino acid sequence as that of neuromedin U;
X represents a methoxyethylene glycol (here, polyethylene glycol represented by plural Xs can be identical or different and
X" represents a polyethylene glycol (here, polyethylene glycol represented by plural X"s can be identical or different);
Lc represents a divalent group represented by
(i) a formula: -C^{a}-Q^{c}-C^{b}-
   wherein
   C^{a} represents -NH-,
   Q^{c} represents a divalent group represented by a formula: -(CH₂)ₘ₁-Z^{c}-(CH₂)ₘ₂-
   (wherein
   m1 represents an integer ranging from 0 to 15,
   Z^{c} represents
   (a) a bond or
   (b) a divalent group selected from -CO-, -O-CO-, -CO-O-, -CO-NH-, -NH-CO-, -CO-NH-CO-, -NH-CO-NH-, -CH(NH2)-, -CH(OH)-, -CH(COOH)-, -C(=NH)-, -CH(-NHX)-, -S-, -S-S-, -SO-, -SO₂-, -NH-SO₂-, -SO₂-NH-, 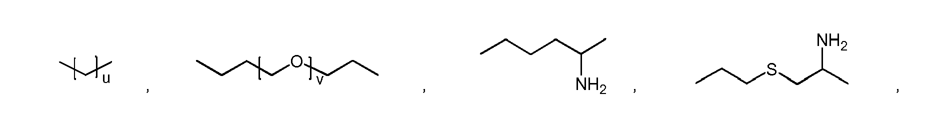 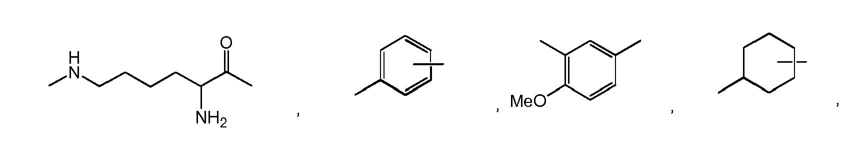 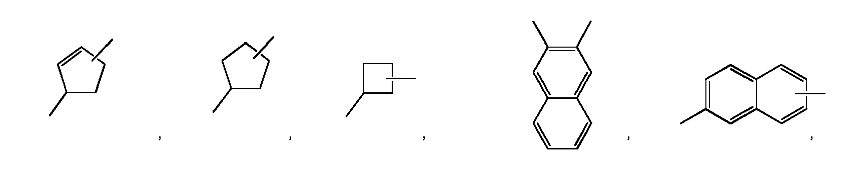 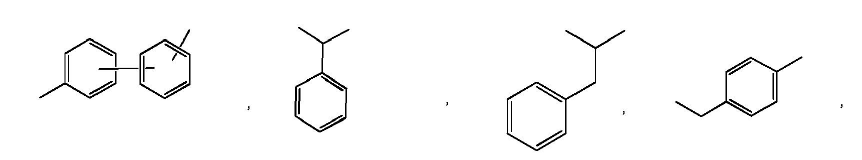 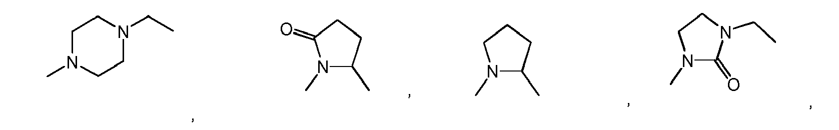 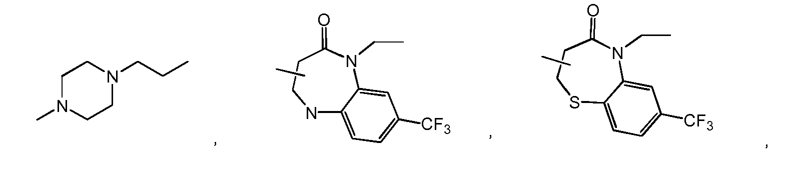 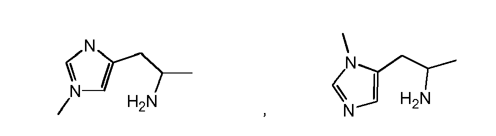 , and 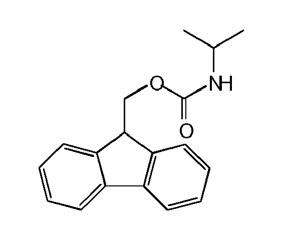 (wherein
      u represents an integer ranging from 1 to 18,
      v represents an integer ranging from 1 to 12,
      R^{Zc1} represents an amino-straight chain C₁-₅ alkyl-carbonyl group or X-straight chain C₁-₅ alkyl group,
      R^{Zc2} represents an amino-straight chain C₁-₅ alkyl-carbonyl amino-chain C₁-₅ alkyl group, and
      X represents the same as mentioned above,
      m2 represents an integer ranging from 0 to 15,
      C^{b} represents a bond, -CO-, or -SO₂-, or
(ii) a formula: -Q^{c'}-C^{b'}-
   wherein
   Q^{c'} represents a divalent group represented by a formula: -(CH₂)_{m1'}-Z^{c'}-(CH₂)_{m2'}-
   (wherein
   m1' represents an integer ranging from 0 to 15,
   Z^{c'} represents 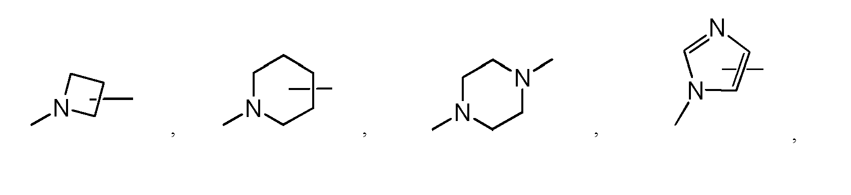 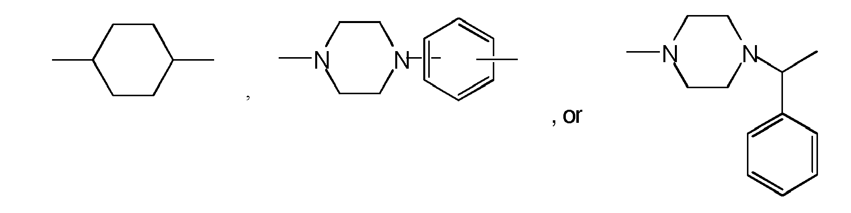 and
   m2' represents an integer ranging from 0 to 15)
   C^{b'} represents -CO- or -SO₂-;
   plural Rs are identical or different at different points of appearance, which represent a bond, 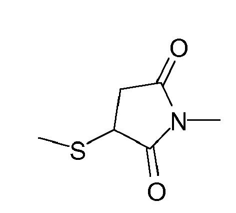 h_{V} represents an integer ranging from 0 to 3; and
   i_{V}, j_{V}, k_{V}, m_{V} and n_{V} are respectively identical or different integers ranging from 0 to 5.

In particular, examples of a linker represented by L includes the following moieties: 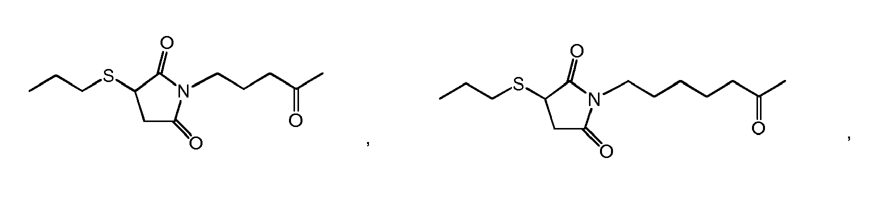 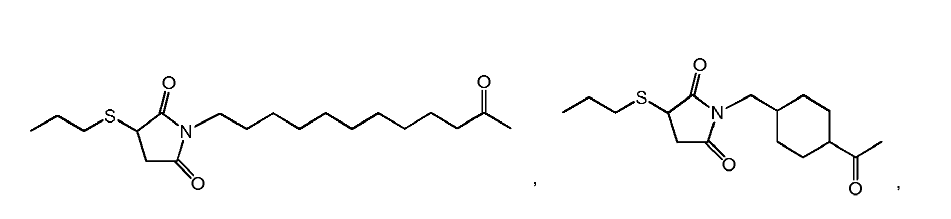 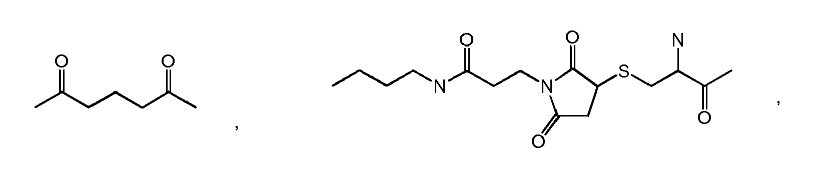 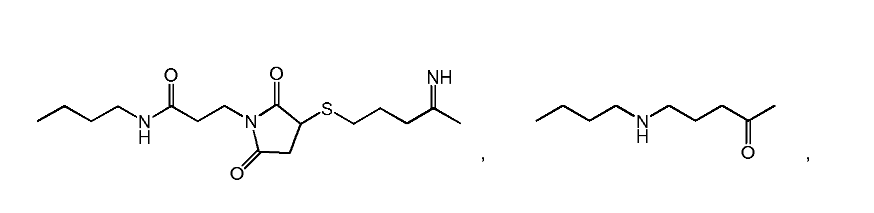 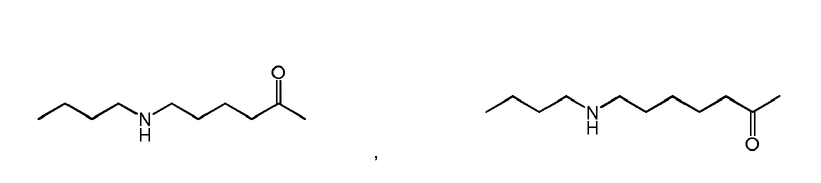 , and

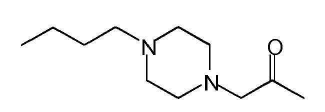

In particular, a neuromedin U derivative wherein n_{V} is 0, R is a bond and (Lc)ⱼ is the following compound is desirable:

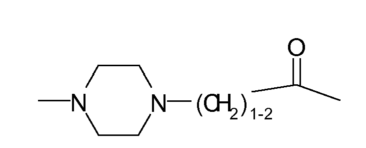

### [Production Method]

A method of producing a neuromedin derivative will be explained below.

The neuromedin derivatives of the present invention can be produced by binding a methoxypolyethylene glycol to a peptide to be used in the present invention via a linker.

The neuromedin derivative of the present invention can be prepared by the known peptide purification method from the aforementioned warm-blooded animal cells or tissues. Specifically, the tissues or cells of warm-blooded animals are homogenized and the soluble fractions are isolated and purified by chromatography such as reversed phase chromatography, ion exchange chromatography, and affinity chromatography to prepare a neuromedin derivative of the present invention.

Further, a neuromedin derivative in used the present invention can be purchased as a commercial product.

A neuromedin of the present invention can be produced according to the known peptide synthetic method.

A peptide synthetic method, for example, can be a solid phase synthetic method and a liquid phase synthetic method. A partial peptide or an amino acid that can constitute a neuromedin derivative of the present invention and a residual part are condensed and if the product has a protective group, a target peptide can be produced by dissociating the protective group.

Here, condensation and dissociation of the protective group can be performed according to the methods described in the following items (1) through (5):
(1) M. Bodanszky and M.A. Ondetti, Peptide Synthesis, Interscience Publishers, New York (1966).
(2) Schroeder and Luebke, The Peptide, Academic Press, New York (1965).
(3) Nobuo Izumiya et al. Peptide Synthesis Basics and Experiments, Maruzen (Inc.) (1975).
(4) Haruaki Yashima and Shunpei Kambara, Biochemistry Experimental Lecture Series 1, Protein Chemistry IV, 205, (1977).
(5) Edited by Haruaki Yashima, Development of Medicines (Second Edition), Vol. 14, Peptide Synthesis, Hirokawa Shoten

The neuromedin derivative of the present invention thus obtained can be isolated and purified by the known purification methods.

Further, the peptide used in the present invention can be produced by culturing a transformant containing nucleic acids coding the peptide and by isolating and purifying the peptide to be used in the present invention from the cultured product obtained.

The nucleic acids for coding the peptide used in the present invention can be DNA or RNA, or DNA/RNA chimera. Preferably, DNA can be used. Further, nucleic acids can be made of double chains or single chains. In the case of double chains, double chain DNA, double chain RNA or hybrids of DNA-RNA are available. In the case of single chains, either sense chains (namely coded chains) or antisense chains (namely non-coded chains) are available.

Examples of DNA coding a peptide to be used in the present invention includes genome DNA, cDNA originated from all cells of warm-blooded animals (e.g., humans, mice, rats, guinea pigs, hamsters, rabbits, sheep, goats, pigs, horses, roosters, cats, dogs, monkeys, chimpanzees) [e.g., pancreatic cells, neural cells, glial cells, pancreatic β-cells, bone marrow cells, mesangial cells, Langerhans cells, epidermal cells, epithelial cells, goblet cells, endothelial cells, smooth muscle cells, fibroblasts, fibrocytes, muscle cells, lipocytes, immune cells (macrophages, T-cells, B-cells, natural killer cells, mast cells, neurocytes, basophils, eosinophils, monocytes, dendrocytes), megakaryocytes, synovial cells, chondrocytes, osteocytes, osteoblasts, osteoclasts, mammary cells, hepatic cells or interstitial cells, or precursor cells, stem cells, or cancer cells of these cells], or the peptides originated from all tissues where such cells are present [e.g., brain, cerebral sites (e.g., olfactory, tonsil nucleus, cerebral basal cells, hippocampus, thalamus, hypothalamus, cerebral cortex, medulla oblongata, cerebellum), spinal cord, pituitary gland, stomach, pancreas, kidney, liver, gonad, thyroid gland, gall bladder, bone marrow, adrenal gland, skin, muscle, lung, gastrointestinal tract (e.g., colon, small intestine), blood vessel, heart, thymus gland, spleen, submandibular gland, peripheral blood, prostate gland, testis, ovary, placenta, uterus, bone, joints, adipose tissue, skeletal muscle, peritoneum], as well as a synthesized DNA.

The genome DNA and cDNA for coding the peptide to be used in the present invention can be directly amplified according to the known method, for example, the Polymerase Chain Reaction (hereinafter referred to as "PCR method") and the Reserve Transcriptase -PCR (hereinafter referred to as "RT -PCR method") using the genome DNA fractions or total RNA or mRNA fractions prepared from the aforementioned cells and tissues respectively as a template. Alternatively, the genome DNA and cDNA for coding the peptide to be used in the present invention can be respectively cloned for example by colony or plaque hybridization method or PCR method according to the known method from the genome DNA library and cDNA library that are prepared by inserting segments of genome DNA and total RNA or mRNA prepared from the aforementioned cells and tissues into an appropriate vector. Examples of the vector to be used in the library include bacteriophages, plasmids, cosmids and phagemids, but any of the above can be used.

The neuromedin derivative of the present invention can be synthesized, for example, by any of the following methods.
(1) A PEGylation reagent having an active ester (e.g., SYNBRIGHT MEGC -30TS (Product name), Nippon Yushi) is bound to an amino group of the peptide to be used in the present invention.
(2) A PEGylation reagent having an aldehyde (e.g., SYNBRIGHT ME -300AL (Product name), Nippon Yushi) is bound to an amino group of the peptide to be used in the present invention.
(3) A divalent cross-linking reagent (e.g., GMBS (Dojin Kagaku), EMCS (Dojin Kagaku), KMUS (Dojin Kagaku), SMCC (Pierce)) is bound to the peptide to be used in the present invention and subsequently a PEGylation reagent having a thiol group (e.g., SUNBRIGHT ME -300 -SH (Product name), Nippon Yushi) is bound. In this case, the linker in the neuromedin derivative of the present invention is originated from PEGylation reagents and divalent cross-linking reagents.
(4) A SH introduction agent (e.g., D -cysteine residue, L -cysteine residue, Traut's reagent) is introduced into the peptide to be used in the present invention and a PEGylation reagent having a maleimide group (e.g., SUNBRIGHT ME -300MA (Product name) Nippon Yushi) is reacted with this thiol group. In this case, the linker in the neuromedin derivative of the present invention is originated from PEGylation reagents and SH introduction agents.
(5) A SH introduction agent (e.g., D-cysteine residue, L-cysteine residue, Traut's reagent) is introduced into the peptide to be used in the present invention and a PEGylation reagent having an iodo-acetamide group (e.g., SUNBRIGHT ME -300IA (Product name) Nippon Yushi) is reacted with this thiol group. In this case, the linker in the neuromedin derivative of the present invention is originated from PEGylation reagents and SH introduction agents.
(6) ω -aminocarboxylic acid or α -amino acid is introduced as a linker to the N-terminal amino group of the peptide to be used in the present invention and a PEGylation reagent having an active ester (e.g., SUNBRIGHT MEGC -30TS (Product name), Nippon Yushi) is reacted to the amino group originated from this linker. In this case, the linker in the neuromedin derivative of the present invention is originated from PEGylation reagents and ω -aminocarboxylic acid or PEGylation reagents and ω -amino acid.
(7) ω -aminocarboxylic acid or α -amino acid is introduced as a linker to the N-terminal amino group of the peptide to be used in the present invention and a PEGylation reagent having an aldehyde group (e.g., SUNBRIGHT MEGC -30AL (Product name), Nippon Yushi) is reacted to the amino group originated from this linker. In this case, the linker in the neuromedin derivative of the present invention is originated from PEGylation reagents and ω -aminocarboxylic acid or PEGylation reagents and α -amino acid.

The aforementioned reagents can be obtained, for example, as commercial products. Each reaction can be carried out by the method known to those in the art.

The neuromedin U derivative of the present invention can be salts. Examples of such salts include salts with inorganic bases, salts with organic bases, salts with inorganic acids, salts with organic acids, salts with basic or acidic amino acids.

Favorable examples of the salts with inorganic bases include alkali metal salts such as sodium salts and potassium salts; alkali earth metal salts such as calcium salts and magnesium salts; aluminum salts and ammonium salts.

Favorable examples of the salts with organic bases include salts with trimethylamine, triethylamine, pyridine, picoline, ethanolamine, diethanolamine, triethanolamine, dicyclohexylamine, N, N-dibenzylethylenediamine.

(1)Favorable examples ofthe salts with organic bases include salts with hydrochloric acid, hydrobromic acid, nitric acid, sulfuric acid, which is mentioned as salt and phosphate.

Favorable examples of the salts with organic acids include salts with formic acid, acetic acid, trifluoroacetic acid, fumaric acid, oxalic acid, tartaric acid, maleic acid, succinic acid, malic acid, methanesulfonic acid, benzenesulfonic acid and *p-*toluenesulfonic acid.

Favorable examples of the salts with basic amino acids include salts with arginine, lysine and ornithine.

Favorable examples of the salts with acidic amino acids include salts with aspartic acid and glutamic acid.

If the neuromedin U derivative ofthe present invention is obtained in a free state according to the aforementioned synthetic method, it can be converted to a salt according to the common method. Further, if it is obtained as a salt, it can be converted to a free form or other salts according to the common method. The neuromedin U derivative of the present invention thus obtained can be isolated and purified from the reaction solution by a known means such as phase transfer, concentration, solvent extraction, fractional distillation, crystallization, recrystallization and chromatography.

If the neuromedin U derivative of the present invention is present as a configurational isomer (configurational isomer), diastereomer and conformer, if desirable, each can be isolated respectively by said separation and purification means. Further, if the neuromedin U derivative is a racemate, it can be separated into a S-form and a R-form by the ordinary optical dissolution means.

If a stereoisomer is present in the neuromedin U derivative of the present invention, the present invention includes cases when this isomer is present independently or cases when they are present as a mixture thereof.

Further, the neuromedin U derivative of the present invention can be a hydrate or non-hydrate. Further, the neuromedin U derivative of the present invention can be a solvate or a non-solvate.

The neuromedin U derivative of the present invention can be labeled with isomers (e.g., ³H, ¹⁴C, ³⁵S). Further, the neuromedin U derivative of the present invention can converted by deuterium.

The neuromedin U derivative of the present invention is useful as an antifeedant, or as an agent for preventing or treating obesity.

The neuromedin U derivative of the present invention is used as a medical composition obtained by formulation according to the known method (e.g., methods described in the Japanese Pharmacopoeia) along with a pharmacologically acceptable carrier.

As a pharmacologically acceptable carrier, various kinds of organic or inorganic carrier substances that are commonly used can be used as formulation raw materials. As specific examples, vehicles, lubricants, binders, disintegrants are included in the solid formulations; solvents, solubilizing agents, suspending agents, isotonization agents, buffers and soothing agents are included in the liquid formulations. When formulating, if desirable, formulation additives such as antiseptics, antioxidants, colorants and sweeteners can be added.

Favorable examples of vehicles include lactose, sucrose, D-mannitol, D-sorbitol, starch, α-starch, dextrin, crystalline cellulose, low-substituted hydroxypropyl cellulose, sodium carboxymethylcellulose, gum Arabic, pullulan, light silicic anhydride, synthetic aluminum silicate and magnesium metasilicic aluminate, xylitol, sorbitol and erythritol.

Favorable examples of lubricants include magnesium stearate, calcium stearate, talc, colloidal silica and polyethylene glycol 6000.

Favorable examples of binders include α-starch, sucrose, gelatin, gum Arabic, methylcellulose, carboxymethylcellulose, sodium carboxymethylcellulose, crystalline cellulose, sucrose, D-mannitol, trehalose, dextrin, pullulan, hydroxypropyl cellulose, hydroxypropyl methylcellulose and polyvinylpyrrolidone.

Favorable examples of disintegrants are as follows: lactose, sucrose, starch, carboxymethylcellulose, calcium carboxymethylcellulose, croscarmellose sodium, sodium carboxymethyl starch, low-substituted hydroxypropylcellulose, light silicic anhydride and calcium carbonate.

Favorable examples of solvents include water for injection, physiological saline, Ringer's solution, alcohol, propylene glycol, polyethylene glycol, sesame oil, corn oil, olive oil and cottonseed oil.

Favorable examples of solubilizing agents include polyethylene glycol, propylene glycol, D-mannitol, trehalose, benzylbenzoate, ethanol, tris-aminomethane, cholesterol, triethanolamine, sodium carbonate, sodium citrate, sodium salicylate and sodium acetate.

Favorable examples of suspending agents include surfactants such as stearyl triethanolamine, sodium laurylsulfate, lauryl amino propionic acid, lecithin, benzalkonium chloride, benzethonium chloride, and glycerin monostearate; hydrophilic polymers such as polyvinyl alcohol, polyvinyl pyrrolidone, sodium carboxymethylcellulose, methylcellulose, hydroxymethylcellulose, hydroxyethylcellulose and hydroxypropylcellulose; polysorbates, and polyoxyethylene-hardened castor oil.

Favorable examples of isotonization agents include sodium chloride, glycerin, D-mannitol, D-sorbitol, glucose, xylitol and fructose.

Favorable examples of buffers include buffer solutions of phosphates, acetates, carbonates and citrates.

Favorable examples of soothing agents include propylene glycol, lidocaine hydrochloride and benzyl alcohol.

Favorable examples of antiseptics include p-oxybenzoic acid esters, chlorobutanol, benzyl alcohol, phenethyl alcohol, dehydroacetic acid and sorbic acid.

Favorable examples of antioxidants include sulfites and ascorbates.

Favorable examples of colorants include water soluble edible tar dyes (e.g., edible dyes such as Food Red No. 2 and No. 3, Food Yellow No. 4 and No. 5, Food Blue No. 1 and 2); insoluble lake dyes (e.g., aluminum salts of the aforementioned water soluble edible tar dyes), natural dyes (e.g., β-carotene, chlorophyll, colcothar).

Favorable examples of sweeteners include sodium saccharin, dipotassium glycyrrhizate, aspartame and stevia.

Examples of form of the aforementioned medical composition include oral agents such as tablets (including sublingual tablets, orally-disintegrating tablets), capsules (including soft capsules and micro capsules), granular agents, powder agents, troches, syrups, emulsions and suspensions; non-oral agents such as injection solutions (e.g., subcutaneous injection agents, intravenous injection solutions, intramuscular injection agents, intraperitoneal injection agents, drip infusion agents), external agents (e.g., transcutaneous formulations and ointments), suppositories (e.g., rectal suppositories and vaginal suppositories), pellets, transnasal agents, transpulinonary agents (inhalation powder), eye drops. These formulations can be provided as controlled-release formulations such as quick release formulations or slow-release formulations (e.g., slow-release microcapsules).

The content of the neuromedin U derivative in the aforementioned medical compositions, for example, ranges from 0.1 to 100 wt%.

A method of producing oral agents and non-oral agents will be explained specifically below. Oral agents can be produced by adding a vehicle (e.g., lactose, sucrose, starch, D -mannitol, xylitol, sorbitol, erythritol, crystalline cellulose, light silicic anhydride), a disintegrant (e.g., calcium carbonate, starch, carboxymethylcellulose, calcium carboxymethylcellulose, low -substituted hydroxypropylcellulose, croscarmellose sodium, sodium carboxymethyl starch, light silicic anhydride), a binder (e.g., α -starch, gum Arabic, carboxymethylcellulose, hydroxypropylcellulose, hydroxypropyl methylcellulose, polyvinylpyrrolidone, crystalline cellulose, methylcellulose, sucrose, D-mannitol, trehalose, dextrin) or a lubricant (e.g., talc, magnesium stearate, calcium stearate, colloidal silica, polyethylene glycol 6000) to an active component followed by compression molding.

Furthermore, for the purpose of masking tastes, enteric solubilization or slow - release, coating can be applied to the oral agent by a known method. As a coating agent, for example, enteric -soluble polymers (e.g., acetic acid phthalic acid cellulose, methacrylic acid copolymer L, methacrylic acid copolymer LD, methacrylic copolymer S, hydroxypropyl methylcellulose phthalate, hydroxypropyl methylcellulose acetate succinate, and carboxymethyl ethyl cellulose), gastric -soluble polymers (e.g., polyvinyl acetal diethylamino acetate and aminoalkyl methacrylate copolymer E), water -soluble polymers (e.g., hydroxypropyl cellulose and hydroxypropyl methylcellulose), water-insoluble polymers (e.g., ethylcellulose, aminoalkyl methacrylate copolymer RS and ethyl acrylate/methyl methacrylate copolymer), and wax can be used. When applying a coating, a plasticizer such as polyethylene glycol; and a light-shielding agent such as titanium oxide and iron sesquioxide can be used along with the aforementioned coating agent.

Injection solutions can be produced by dissolving, suspending or emulsifying an active component along with a dispersant (e.g., Tween 80 (by Atraspowder Corporation, USA), HCO 60 (by Nikko Chemicals), Polyethylene glycol, carboxymethylcellulose and sodium arginate), a preservative (e.g., methylparaben, propylparaben, benzyl alcohol, chlorobutanol and phenol), an isotonization agent (e.g., sodium chloride, glycerin, D- sorbitol, D-mannitol, xylitol, glucose and fructose) in an aqueous solvent (e.g., distilled water, physiological saline and Ringer's solution) or an oil-base solvent (e.g., vegetable oils such as olive oil, sesame oil, cottonseed oil, corn oil; propylene glycol, macrogol, tricaprylin). In this case, if desirable, the following additives can be added: a solubilizing agent (e.g., sodium salicylate, sodium acetate, polyethylene glycol, propylene glycol, D-mannitol, trehalose, benzyl benzoate, ethanol, tris-aminomethane, cholesterol, ethanolamine, sodium carbonate, and sodium citrate), a suspending agent (e.g., surfactants such as stearyl triethanolamine, sodium laurylsulfate, lauryl amino propionic acid, lecithin, benzalkonium chloride, Benzethonium chloride and glycerin monostearate; hydrophilic polymers such as polyvinyl alcohol, polyvinyl pyrrolidone, sodium carboxymethylcellulose, methylcellulose, hydroxymethylcellulose, hydroxyethylcellulose and hydroxypropylcellulose), a buffer (e.g., buffer solutions such as phosphates, acetates, carboxylates and citrates), a stabilizer (e.g., human serum albumin), a soothing agent (e.g., propylene glycol, lidocaine hydrochloride and benzyl alcohol), an antiseptic (e.g., para-oxybenzoic acid esters, chlorobutanol, benzyl alcohol, phenethyl alcohol, dehydroacetic acid and sorbic acid).

External agents can be produced by preparing an active component as solid, semi-solid or liquid compositions. For example, the aforementioned solid compositions can be produced directly from the active component or by adding a vehicle (e.g., lactose, D-mannitol, starch, crystalline cellulose and sucrose) and a thickening agent (e.g., natural gums, cellulose derivatives, acrylic acid polymers) or by blending to form a powder. The aforementioned liquid compositions can be produced almost similarly to the case of injection solutions. Semi-solid compositions are preferably prepared in aqueous or oil-base gelatin agents or in an ointment form. Further, all of these compositions can contain a pH regulator (e.g., phosphoric acid, citric acid, hydrochloric acid, sodium hydroxide), and an antiseptic (e.g., p-oxybenzoic acid esters, chlorobutanol, benzalkonium chloride, benzyl alcohol, phenethyl alcohol, dehydroacetic acid and sorbic acid). Suppositories can be produced by preparing an active component as oil-base or aqueous solid, semi-solid or liquid compositions. Examples of oily bases used when producing such compositions, for example, include higher fatty acid glycerides [e.g., cacao fat, Witepsol], intermediate fatty acid triglycerides [e.g., miglyols], vegetable oils (e.g., sesame oil, soybean oil, cottonseed oil). Examples of aqueous bases include, for example, polyethylene glycols and propylene glycol. Further, examples of aqueous gel bases include, for example, natural gums, cellulose derivatives, vinyl polymers and acrylic acid polymers.

The dosage of the neuromedin U derivative of the present invention can be selected appropriately based on the subjects of administration, administration routes, target diseases and symptoms. A dosage when a medical composition containing the neuromedin U derivative ofthe present invention as an active ingredient is subcutaneously injected in adult patients generally ranges from approximately 5 to 5000 µg/human and preferably from approximately 5 to 500 µg/human as a single dose. It is desirable to administer this dosage 1 to 3 times daily.

The neuromedin U derivative of the present invention (hereinafter simply called a compound of the present invention) can be used concomitantly with a concomitant drug having no adverse effects on the compound of the present invention for the purpose of enhancing the activity (e.g., antifeedant effect, preventive or therapeutic effect on obesity) and reducing the amount of the compound of the present invention to be used. Examples of such concomitant drugs include for example, "diabetes treatment drugs", "diabetes complication treatment drugs", "anti-obesity drugs", and "hyperlipidemia treatment drugs". Two or more concomitant drugs can be combined at an appropriate ratio.

Examples of the aforementioned "diabetes treatment drugs" include the following drugs: insulin formulations (e.g., animal insulin formulations extracted from bovine and pig pancreas; human insulin formations synthesized by genetic engineering using E. coli and yeast; insulin zinc; protamine insulin zinc; insulin fragment or derivatives (e.g., INS1), oral insulin formulations), insulin resistance improvement agents (pioglitazone or its salts (preferably hydrochloride), rosiglitazone or its salts (preferably maleates), Tesaglitazar, Ragaglitazar, Muraglitazar, Edaglitazone, Metaglidasen, Naveglitazar, AMG-131, THR-0921), α-glycosidase inhibitor (e.g., voglibose, acarbose, miglitol, emiglitate), biguanide agents (e.g., metformin, buformin or their salts (e.g., hydrochloride, fumarates, succinates)), insulin secretion accelerators [sulfonylurea agents (e.g., tolbutamide, glybenclamide, gliclazide, chlorpropamide, tolazamide, acetohexamide, glyclopyramide, glimepiride, glipizide, glybuzole), repaglinide, nateglinide, mitiglinide or its calcium salt hydrate], dipeptidyl peptidase IV inhibitor (e.g., Vildagliptin, Sitagliptin, Saxagliptin, T-6666, TS-021), β3 agonist (e.g., AJ-9677), GPR40 agonist, GLP-1 receptor agonist [e.g., GLP-1, GLP-1MR agent, NN-2211, AC-2993 (exendin-4), BIM-51077, Aib (8,35) hGLP-1 (7, 37) NH2, CJC-1131], amyrin agonists (e.g., Pramlintide), phosphotyrosine phosphorylase inhibitor (e.g., sodium vanadate), sugar generation inhibitors (e.g., glycogen phosphorylase inhibitor, glucose-6-phosphatase inhibitor, glucagon antagonist), SGLUT (sodium-glucose cotransporter) inhibitor (e.g., T -1095), 11β-hydroxysteroid dehydrogenase inhibitor (e.g., BVT -3498), adiponectin or its acting drug, IKK inhibitor (e.g., AS -2868), Leptin resistance improving drug, Somatostatin receptor acting drug, glucokinase activation drug (e.g., Ro-28-1675), GIP (Glucose -dependent Insulinotropic Peptide).

Examples of the aforementioned "diabetes complication treatment drugs" include the following drugs: aldose reductive enzyme inhibitor (e.g., tolrestat, epalrestat, zenarestat, zopolrestat, minalrestat, fidarestat, Neurotrophin production/secretion accelerator described in WO01/14372 (e.g., 4 -(4 -chlorophenyl)-2-(2-methyl-1-imidazolyl)-5-[3-(2-methylphenoxy) propyl]oxazol)), PKC inhibitors (e.g., ruboxistaurin mesylate)), AGE inhibitors (e.g., ALT946, Pimagedine, N-phenacylthiazolium bromide, EXO -226, Pyridorin), Pyridoxamine), active enzyme deleting drug (e.g., thioctic acid), brain blood vessel dilator (e.g., tiapride, mexiletine), somatostatin receptor acting drug (e.g., (BIM23190), apoptosis signal regulating kinase (ASK-1) inhibitor neuro generation accelerator (e.g., Y-128, VX-853, prosaptide).

Examples of the aforementioned "anti-obesity drug" include central anti-obesity drug (e.g., dexfenfluramine, fenfluramine, phentermine, Sibutramine, amfepramon, dexamfetamine, mazindol, phenylpropanolamine, clobenzorex; neuropeptide Y antagonists (e.g., CP -422935); cannabinoid receptor antagonists (e.g., SR -141716, SR - 147778); ghrelin antagonists; 11β-hydroxysteroid dehydrogenase inhibitors (e.g., BVT - 3498), pancreatic lipase inhibitors (e.g., orlistat, cetilistat, β3 agonist (e.g., AJ -9677), peptide antifeedant (e.g., leptin, CNTF (Ciliary Neurotrophic Factor), cholecystokinin agonists (e.g., lintitript, FPL -15849), and antifeedant (e.g., P -57).

Examples of the aforementioned "hyperlipidemia treatment drug" include HMG-CoA reductase inhibitors (e.g., pravastatin, simvastatin, lovastatin, atorvastatin, fluvastatin, pitavastatin or their salts (e.g., sodium salts, calcium salts)), squalene synthase inhibitors (e.g., Compounds described in WO97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid), fibrate compounds (e.g., bezafibrate, clofibrate, simfibrate, clinofibrate), ACAT inhibitors (e.g., Avasimibe), Eflucimibe)), cation exchange resins (e.g., cholestyramine), probucol, nicotinic acid drugs (e.g., nicomol), niceritrol)), ethyl icosapentate, and plant sterols (e.g., soysterol, γ-oryzanol))

Timing of administration of said concomitant drugs is not limited. The compound of the present invention and the concomitant drugs can be administered at the same time to the administration subjects, or can be administered at different times. The dosages of concomitant drugs are determined in compliance with the dosages applied clinically. It can be selected appropriately based on the administering subjects, administration routes, diseases and combinations.

The form of administration of concomitant drugs with the compound of the present invention is not particularly limited and is acceptable as long as the compound of the present invention is combined with concomitant drugs at the time of administration. Examples of such forms of administration are as follows:
(1) Administration of a single formula obtained by simultaneous formulation of the compound of the present invention with a concomitant drug,
(2) Simultaneous administration via the same administration route for two kinds of formulas obtained by independent formulations of the compound of the present invention and a concomitant drug,
(3) Administrations at different times via the same administration route for two kinds of formulas obtained by independent formulations of the compound of the present invention and a concomitant drug,
(4) Simultaneous administration via different administration routes for two kinds of formulas obtained by independent formulations of the compound of the present invention and a concomitant drug,
(5) Administrations at different times via different administration routes for two kinds of formulas obtained by independent formulations of the compound of the present invention and a concomitant drug. (For example, administration in the order of the composition of the present invention and a concomitant drug, or administration in the reverse order).

The mixing ratio between the compound of the present invention and a concomitant drug can be selected appropriately based on the administration subjects, administration routes and diseases.

The compound of the present invention can be used along with dietary therapies (e.g., dietary therapies for diabetes) and physical therapies.

When amino acids are displayed by abbreviations in the present specification, they are based on the abbreviations according to the IUPAC-IUB Commission on Biochemical Nomenclature or common abbreviations used in this field and examples will be shown below. If optical isomers are possibly present with regard to amino acids, they represent a L-form unless otherwise specifically mentioned.

- Gly:: Glysine
- Ala:: Alanine
- Val:: Valine
- Leu:: Leucine
- Ile:: Isoleucine
- Ser:: Serine
- Thr:: Threonine
- Cys:: Cysteine
- Met:: Methionine
- Glu:: Glutamic acid
- Asp:: Aspartic acid
- Lys:: Lysine
- Arg:: Arginine
- His:: Histidine
- Phe:: Phenylalanine
- Tyr:: Tyrosine
- Trp:: Tryptophan
- Pro:: Proline
- Asn:: Asparagine
- Gln:: Glutamine
- pGlu:: Pyroglutamic acid
- Sec:: Selenocysteine

The sequence numbers in the sequence list in the specification of this application represent the following sequences.
(Sequence No. 1) represents an amino acid sequence of porcine NMU-25.
(Sequence No. 2) represents an amino acid sequence of porcine NMU-8.
(Sequence No. 3) represents an amino acid sequence of human NMU-25.
(Sequence No. 4) represents an amino acid sequence of C-terminus 8 amino acid residue (human NMU-8) of human NMU-25.
(Sequence No. 5) represents an amino acid sequence of rat NMU-23.
(Sequence No. 6) represents an amino acid sequence of C-terminus 8 amino acid residue (rat NMU-8) of rat NMU-25.
(Sequence No. 7) represents an amino acid sequence of human NMUR1.
(Sequence No. 8) represents an amino acid sequence of human NMUR2
(Sequence No. 9) represents an amino acid sequence of rat NMS.
(Sequence No. 10) represents an amino acid sequence of human NMS.
(Sequence No. 11) represents an amino acid sequence of C-terminus 8 amino acid residue (human NMS-8) of human NMS.
The C terminals in sequence numbers 1 through 6, 9 through 11 amidated.

### [Brief Explanation of the Drawing]

[Fig. 1] A graph showing an antifeedant activity of NMU-23 and NMS.
[Fig. 2] A graph showing an antifeedant activity of NMU-23 and NMU-8.
[Fig. 3A] A graph showing the equilibrium bond of ¹²⁵I-NMU8 in the FM3 membrane fraction by Scatchard plot analysis.
[Fig. 3B] A graph showing the equilibrium bond of ¹²⁵I-NMU8 in the TGR1 membrane fraction by Scatchard plot analysis.
[Fig. 4 A] A graph showing a mode of bond inhibition of ¹²⁵I-NMU8 when adding a NMU derivative and a PEG conjugate with changes in concentration relative to the FM3 membrane fraction.
[Fig. 4 B] A graph showing a mode of bond inhibition of ¹²⁵I-NMU8 when adding a NMU derivative and a PEG conjugate with changes in concentration relative to the TGR1 membrane fraction.
[Fig. 4 C] A graph showing a mode of bond inhibition of ¹²⁵I-NMU8 when adding a NMU derivative and a PEG conjugate with changes in concentration relative to the FM3 membrane fraction.
[Fig. 4 D] A graph showing a mode of bond inhibition of ¹²⁵I-NMU8 when adding a NMU derivative and a PEG conjugate with changes in concentration relative to the TGR1 membrane fraction.
[Fig. 5] A graph showing antifeedant activity of a NMU-8 PEG conjugate in mice.
[Fig. 6] A graph showing antifeedant activity of a NMU-8 PEG conjugate in mice.
[Fig. 7] A graph showing antifeedant activity of a NMU-8 PEG conjugate in mice.
[Fig. 8] A graph showing antifeedant activity of a NMU-8 PEG conjugate in mice.
[Fig. 9] A graph showing antifeedant activity of a NMU-8 PEG conjugate in mice.
[Fig. 10] A graph showing the food intakes of diet-induced obesity mice and bodyweight changes when a NMU-8 PEG conjugate was administered repeatedly subcutaneously for 1 week.
[Fig. 11] A graph showing the antifeedant activity of a NMU-8 PEG conjugate in mice.
[Fig. 12] A graph showing anti-obesity activity of a NMU-8 PEG conjugate.

### [Examples]

This invention will be further explained in detail below with reference to the following Test Examples, Reference Examples and Embodiments.

### Test Example 1

### Antifeedant Activity of NMU -23 and NMS in mice.

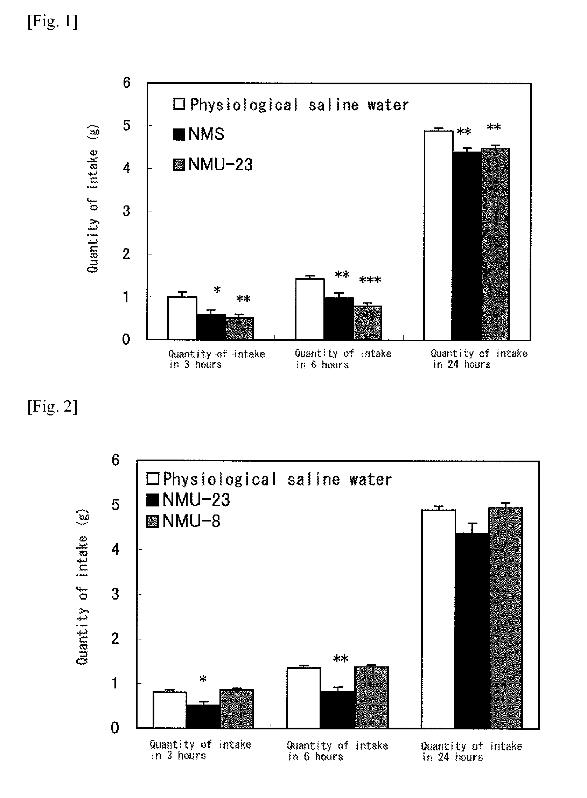

7 weeks old male C57BL/6J mice (mean bodyweight: 24 g) delivered from the Japan Charles River Company were raised for 5 to 7 days after the delivery under a feeding environment regulated for temperature and humidity with lighting time (25°C, 12 hours of lighted period and 12 hours of dark period, light was lit at 8:00). Four animals were placed in one cage. After handling the mice for 5 to 8 days, the animals were housed singly in each cage where a floor mesh was spread and acclimated by intraperitoneal injection for 3 days prior to the administration of a peptide. The animals acclimated were fasted for 16 hours from 18:00 prior to the administration of the peptide. However, the animals had free access to drinking water. Subsequently, a peptide, namely 720 µg/ml rat NMU -23 (peptide Institution) [Sequence No. 5], or rat NMS (Bachem) [Sequence No. 9] dissolved in physiological saline solution was intraperitoneally injected to the mice such that the dosage of each solution (100 µl) was 3 mg/kg at 10:00 on the day of administration. After the injection of the peptide solution, MF feed which had been weighed (Oriental Yeast Industry) was given freely to the mice. Further, the residual amount of the feed was measured after 3, 6 and 24 hours. The food intakes at 3, 6 and 24 hours were calculated by subtracting the residual amount of the feed after 3, 6 and 24 hours from the amount of the feed that was originally given. The results are shown in Fig. 1. As clearly shown in Fig. 1, both rat NMU -23 and rat NMS significantly suppressed the intakes of the feed at 3, 6 and 24 hours.
[In Fig. 1, *, ** and *** tested by T-tests indicated to be lower at the levels of significance of 0.05, 0.01 and 0.001 (P<0.05, P< 0.01, P<0.001)]

### Test Example 2

### Antifeedant Activity of NMU -23 and NMU-8 in mice

7 weeks old male C57BL/6J mice (mean bodyweight: 25 g) delivered from the Japan Charles River Company were raised for 5 to 10 days after delivery under a feeding environment regulated for temperature and humidity with lighting time (25°C, 12 hours of lighted period and 12 hours of dark period, light was lit at 8:00). Four animals were placed in one cage. After handling the mice for 5 to 8 days, the animals were housed singly in each cage where a floor mesh was spread and acclimated by intraperitoneal injection for 3 days prior to the administration of a peptide. The animals acclimated were fasted for 16 hours from 18:00 prior to the administration of the peptide. However, the animals had free access to drinking water. Subsequently, a peptide, namely 750 µg/ml rat NMU -23 (peptide Institution) [Sequence No. 5], or porcine NMU-8 (Bachem) [Sequence No. 2] dissolved in physiological saline solution was intraperitoneally injected to the mice such that the dosage of each solution (100 µl) was 3 mg/kg at 10:00 on the day of administration. After the injection of the peptide solution, MF feed which had been weighed (Oriental Yeast Industry) was given freely to the mice. Further, the residual amount of the feed was measured after 3, 6 and 24 hours. The food intakes at 3, 6 and 24 hours were calculated by subtracting the residual amount of the feed after 3, 6 and 24 hours from the amount of the feed that was originally given. The results are shown in Fig. 2. As clearly shown in Fig. 2, both rat NMU -23 significantly suppressed the intakes of the feed at 3 and 6 hours.
[In Fig. 2, * and ** tested by T-tests indicated to be lower at the levels of significance of 0.05 and 0.01 (P<0.05 and P< 0.01]

### Embodiment 1

### Preparation of a NMU-8 PEG conjugate using PEG -SH

Porcine NMU-8 (Bachem) [Sequence No. 2] 3.5 µmol (=3.85 mg) was dissolved in 500 µl of dimethylformamide and further 5 µmol (10 to 13 mg) of divalent cross-linking reagent [GMBS, EMCS, KMUS (Dojin Kagaku), or SMCC (Pierce)], and 2.5 µl (18 µmole) of 7.2 M triethylamine were dissolved in the solution to be used as a reaction solution and the reaction was carried out overnight at room temperature while light shielded. Each reaction solution was diluted with Solution A (0.1% trifluoroacetic acid/distilled water) by 20 times and injected at a flow rate of 4.5 ml/min. into a CAPCELL PAK, ODS column MGII, 10 x 250 mm, Shiseido) which was equilibrated with Solution A 100% - Solution B (0.1 % trifluoroacetic acid/80% acetonitrile) 0%. The concentration of Solution B was slowly increased from Solution A 100% - Solution B 0% in order to separate the NMU-8 wherein each divalent cross-linking reagent was introduced from the unreacted NMU-8 and excess divalent cross-linking reagent to be eluted, and purified NMU-8 containing each divalent cross-linking agent was fractionated and treated by freeze drying.
Each freeze-dried product obtained was dissolved in 10 ml of 25% acetonitrile - 75% distilled water. Further, PET (SUNBRIGHT ME300 -SH, Nippon Yushi) 180 mg (6 µmole) containing thiol with a mo lecular weight of 3 0 k was dissolved in each NMU-8 solution containing each divalent cross-linking reagent and the reaction was carried out through two nights at 4°C while light shielded. Acetic acid was added in such amount that the final concentration became 0.1 M and then loaded into a SP-Sephadex C 50 ion exchange column (capacity 5 to 10 ml). After rinsing the column initially with 0.1 M acetic acid and subsequently with 10 mM ammonium formate/0.1 M acetic acid, each NMU-8 PEG conjugate was eluted using 2 M ammonium formate. Namely, PEG 30 k (GMBS) -NMU-8 [1], PEG 30k (EMCS) -NMU-8 [2], PEG 30k (KMUS) -NMU-8 [3], and PEG 30 k (SMCC) -NMU-8 [4] were eluted from the column.

Each eluate obtained was injected at a flow rate of 4.5 ml/min. into the CAPCELL PAK column C8 column (SG300, 10 x 250 mm, Shiseido) which was equilibrated with Solution 100% - Solution 0%. After sharp elevation to Solution A 55% - Solution B 45%, further the concentration was elevated linearly to Solution A 40% - Solution B 60% during a period of 40 min. in order to elute each NMU-8 PEG conjugate respectively and a peak of each NMU-8 PEG conjugate was fractionated, further treated by freeze-drying. Each NMU-8 PEG conjugate obtained, namely PEG 30 k (GMBS) -NMU-8 [1], PEG 30 k (EMCS) -NMU-8 [2], PEG 30 k (KMUS) -NMU-8 [3], and PEG 30 k (SMCC) -NMU-8 [4] freeze-dried product was dissolved in distilled water. The peptide concentration
was measured by amino acid analysis.

**[Table 1]**

| | Structure | Conjugate name |
|---|---|---|
| [1] | 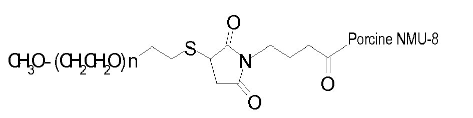 | PEG30k(GMBS)-NMU-8 |
| [2] | 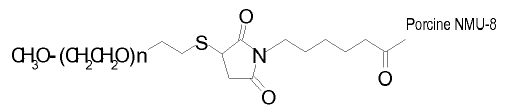 | PEG30k(EMCS)-NMU-8 |
| [3] | 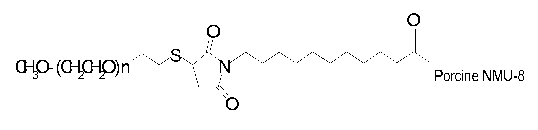 | PEG30k(KMUS)-NMU-8 |
| [4] | 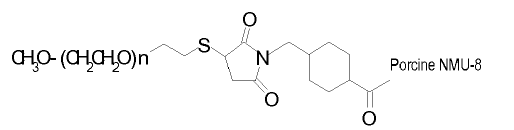 | PEG30k(SMCC)-NMU-8 |

(Porcine NMU-8 was bound to a linker at α-amino group.)

### Embodiment 2

### Preparation of a NMU-8 PEG conjugate using PEG -NHS

Porcine NMU 8 (Bachem) [Sequence No. 2] 7.2 µmol (8.0 mg) was dissolved in 500 µl of dimethylformamide and further 22 µmol (approximately 650 mg) of PEG containing n-hydroxysuccimide (SUNBRIGHT MEGC -30TS, Nippon Yushi) was dissolved in 7 ml of dimethylsulfoxide and subsequently 2.5 µl of triethylamine was added. The reaction was carried out at room temperature for 4 to 6 hours. After acetic acid was added to the reaction solution such that the final concentration became 0.1 M and the reaction solution was diluted with 42 ml of 0.1 M acetic acid, the solution was into a SP-Sephadex C50 ion exchange column (5 to 10 ml). After rinsing the column initially with 0.1 M acetic acid and subsequently with 10 mM ammonium formate/0.1 M acetic acid, a NMU-8 PEG conjugate namely PEG 30 k -NMU-8 [5] was eluted from the column using initially 2 M ammonium formate/20% acetonitrile and subsequently 3.2 M ammonium formate/20% acetonitrile.
Each eluate obtained was injected at a flow rate of 4.5 ml/min. into the CAPCELL PAK column C8 column (SG300, 10 x 250 mm, Shiseido) which was equilibrated with Solution A 100% - Solution B 0%. After sharp elevation to Solution A 60% - Solution B 40%, the concentration was further elevated linearly to Solution A 30% - Solution B 70% during a period of 40 min. in order to elute a PEG 30k -NMU-8 [5]. A peak of PEG 30 k -NMU-8 [5] was fractionated, and further treated by freeze drying. The PEG 30 k - NMU-8 [5] freeze-dried product obtained was dissolved in distilled water and the peptide concentration was measured by amino acid analysis.

**[Table 2]**

| | Structure | Conjugate name |
|---|---|---|
| [5] | 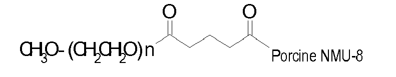 | PEG30k-NMU-8 |

(Porcine NMU-8 was bound to the linker originated from the PEGylation reagent at the α-amino group.)

### Embodiment 3

### Preparation of a NMU-8 PEG conjugate using PEG maleimide (1)

Rat NMU-8 (Anygen) containing a L-Cys residue at the N-terminal [Sequence No. 6] 7.6 µmol (= 8.9 mg) was dissolved in 10 ml of 10 mM phosphoric acid buffer/25% acetonitrile and then 20.9 µmol (627 mg) of PEG-maleimide (SUNBRIGHT ME - 300MA, Nippon Yushi) was added. The reaction was carried out overnight at 4°C while light shielded. Acetic acid was added to each reaction solution such that the final concentration became 0.1 M and the reaction solution was then loaded into a SP - Sephadex C 50 ion exchange column (capacity ranging from 5 to 10 ml). After washing the column with 0.1 M acetic acid, and subsequently with 10 mM ammonium formate/0.1 M acetic acid, a NMU-8 PEG conjugate, namely PEG 30k -Cys -NMU-8 [6] was eluted from the column using 2 M ammonium formate/20% acetonitrile and subsequently 3.2 M ammonium formate/20% acetonitrile.
The eluate obtained was injected at a flow rate of 4.5 ml/min. into the CAPCELL PAK column C8 column (SG300, 10 x 250 mm, Shiseido) equilibrated with Solution A 100% - Solution B 0%. After sharp elevation of the concentration to Solution A 60% - Solution B 40%, the concentration was further elevated linearly to Solution A 30% - Solution B 70% during a period of 40 min. to elute PEG 30 k -Cys -NMU-8 [6]. A peak of PEG 30 k -Cys -NMU-8 [6] was fractionated and further treated by freeze-drying. The freeze-dried product of PEG 30 k -Cys -NMU-8 [6] was dissolved in distilled water and the peptide concentration was measured by amino acid analysis. It is noted that, in the name of conjugate, the expression ofL-indicating the L-form of amino acid may be omitted.

**[Table 3]**

| | Structure | Conjugate name |
|---|---|---|
| [6] | 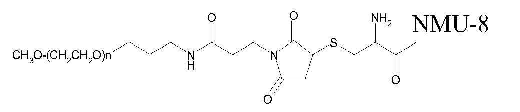 | PEG30k-Cys-NMU-8 |

(Rat NMU-8 is bound to the L -Cys which is a part of the linker at α amino group.)

### Embodiment 4

### Preparation of a NMU-8 PEG conjugate using PEG-maleimide (2)

Porcine NMU-8 (Anygen) [Sequence No. 2] 7.2 µmol (8.1 mg) was dissolved in 500 µl of dimethylformamide. Further, 700 mM triethylamine 31 µm (21.6 µmol) and 60 mM Traut's solution prepared by dissolving 20 mg of Traut's reagent (Pierce) in 2400 µl dimethylformamide 600 µl (36 µmol) were added to prepare a reaction solution. The reaction was carried out for 4 hours at room temperature while light shielded. The reaction solution was diluted with Solution A (0.1 % trifluoroacetic acid/distilled water) by 20 times and then injected at a flow rate of 9.0 ml/min into a CAPCELL PAK column C 18 column (MGII, 20 x 250 mm, Shiseido) which was equilibrated with Solution 100% -Solution B (0.1% trifluoroacetic acid/80% acetonitrile) 0%. After the sharp elevation to Solution A 75% -Solution B 25%, the concentration was further elevated linearly Solution A 60% - Solution B 40% during a period of 40 min. in order to elute Traut's - NMU-8. The peak was fractionated and treated by freeze drying.
Each freeze-dried product obtained was dissolved in 10 ml of 10 mM phosphoric acid rubber/25% acetonitrile and further 21.6 µmol (720 mg) of PEG-maleimide (Nippon Yushi) was added to the solution and the reaction was carried out overnight at 4 °C with light shielded. Acetic acid was added to each reaction mixture such that the final concentration became 0.1M and the reaction solution was loaded into a SP-Sephadex C50 ion exchange column (capacity ranging from 5 to 10 ml). After washing the column initially with 0.1 M acetic acid and subsequently with 10 mM ammonium formate/0.1 M acetic acid, a NMU-8 PEG conjugate, namely PEG 30 k (Traut) -NMU-8 [7] was eluted from the column initially using 2 M ammonium formate/20% acetonitrile and subsequently 3.2 M ammonium formate/20% acetonitrile.
The eluate obtained was injected at a flow rate of 4.5 ml/min into a CAPCELL PAK column C8 column (SG300, 10 x 250 mm, Shiseido) which was equilibrated with Solution A 100% -Solution B 0%. After the sharp elevation to Solution A 60% -Solution B 40%, the concentration was further elevated linearly to Solution A 30% -Solution B 70% during a period of 40 min. in order to elute PEG 30k (Traut) -NMU-8 [7]. The peak of PEG 30k (Traut) -NMU-8 [7] was fractionated and treated by freeze-drying. The freeze-dried PEG 30 k (Traut) -NMU-8 [7] obtained was dissolved in distilled water and the peptide concentration was measured by amino acid analysis.

**[Table 4]**

| | Structure | Conjugate name |
|---|---|---|
| [7] | 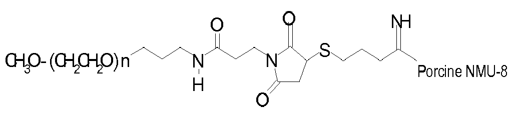 | PEG30k(Traut)-NMU-8 |

(Porcine NMU-8 is bound to a linker at α amino group.)

### Embodiment 5

### Preparation of a NMU-8 PEG conjugate using PEG-NHS (1)

A porcine NMU-8 (Bachem) [Sequence No. 2] 18 µmol (20.0 mg) was dissolved in 500 µl of dimethylformamide. Separately, 27 µmol of diethyl cyanophosphate and 54 µmol of triethylamine were added to a solution prepared by dissolving 27 µmol equivalent Boc-ω amino carboxylic acid [Boc -Aze (2) -OH, Boc -Aze (3) -OH, Boc -A (4) -OH, Boc-(4) Ambz -OH, Boc -β -Ala -OH, Boc -Abu (4) -OH, Boc-(4) Ambz -OH, Boc -Ape (5) -OH, Boc -Acp (6)-OH, Boc -Aoc (8) -OH, Boc -11 -Amino undecanoic-Acid, Boc -12 -Amino dodecanoic -Acid (All the above by Watanabe Kagaku Kogyo K.K.), 500 µl of dimethylformamide, 2 -(4 -Boc -piperazinyl) -2-phenylacetic -Acid (ALDRICH Chemical Company), 3 -(4 -Boc -piperazine -1 -yl) propionic -Acid, and 2 - (4 -Boc -piperazine -Y -YL) Acetic -Acid -Hydrate (All the above-by Fluorochem. Ltd.), 4' -[Boc -Amino] -[1, 1' -biphenyl] -4 -carboxylic acid, or 3'- [Boc - amino] [1, 1'-biphenyl] -4 -carboxylic acid (All the above by Bio-Farma)) in 500 µl dimethylformamide and the reaction was carried out at room temperature for 1 hour. Each reaction solution was diluted by 20 times by Solution A (0.1% trifluoroacetic acid/distilled water). The solution was injected at a flow rate of 9.0 ml/min into the CAPCELL PAR: C18 column (MGII: 20 x 250 mm, Shiseido) which was equilibrated with Solution A 100% - Solution B (0.1 % trifluoroacetic acid/80% acetonitrile) 0% (Boc -11 -Amino undecanoic -Acid and Boc -12 -Amino dodecanoic -Acid (CAPCELL PAK, C1 column (UG120, 20 x 25; 0 mm, Shiseido)). After elevating sharply to Solution A 60% - Solution B 40% and then linearly to Solution A 30% - Solution B 70% during a period of 60 min. NMU-8 introduced with each ω-Boc was separated and eluted from unreacted NMU-8 and excess Boc - ω amino carboxylic acid. The purified NMU-8 introduced with ω amino carboxylic were each fractionated and the product was freeze-dried.

Each freeze-dried product was dissolved in 200 µl of distilled water. 2 ml of trifluoroacetic acid was added and the reaction was carried out at room temperature for 45 min. in order to remove the Boc group. The reaction solution was diluted with diethylether by 10 times and after mixing thoroughly, the mixture was centrifuged at 4°C, at 9500 rpm for 15 min. The supernatant was discarded by decantation and 5 ml of diethylether was added to the pellets and mixed thoroughly. The same procedure was repeated. After drying the pellets obtained at room temperature, they were dissolved in 6 ml of 0.1M column C18 column (MG11, 20 x 250 mm, acetic acid. The solution was injected at a flow rate of 9.0 ml/min. into a CAPCELL Pak column which was
equilibrated with Solution A 100%/Solution B 0%. After the quick elevation to Solution A 75% - Solution B 25%, the concentration was linearly elevated further to Solution A 45 % and Solution B 55% during a period of 60 min. in order to elute and fractionate each ω amino carboxylic acid -NMU 8 conjugate. The product was freeze-dried.

The ω amino carboxylic acid -NMU 8 conjugate (equivalent to 2.0 µmol) obtained was dissolve in 500 µl of dimethylsulfoxide. Subsequently, 6.0 µmol (approximately 180 mg) of n-hydroxysuccimide-introduced PEG (SUNBRIGHT MEGC -30TS, Nippon Yushi) were dissolved in 1 ml dimethylsulfoxide and added to this solution. Subsequently 6.0 µmol of triethylamine was added and the reaction was carried out at room temperature for 2 hours. Acetic acid was added to the reaction solution in such an amount that the final concentration was 0.1 M. Further the solution was diluted with 40 ml of 0.1 M acetic acid and loaded into a SP-Sephadex C50 ion exchange column (capacity: 5 to 10 ml). After rinsing the column with 0.1 M acetic acid and then 10 mM ammonium formate/0.1 M acetic acid, the NMU-8 PEG conjugate was eluted from the column with 2 M ammonium formate/20% acetonitrile and then with 3.2 M ammonium formate/20% acetonitrile.
The eluate obtained was injected into a CAPCELL PAK column C8 column (SG300, 20 x 250 mm, Shiseido) which was equilibrated with Solution A 100%/Solution B 0% at a flow rate of 9.0 ml/min. After the quick elevation to Solution A 55%/Solution B 45%, the concentration was further elevated linearly to Solution A 25%/Solution B 75% during a period of 60 min. in order to elute the NMU-8 PEG conjugate [8 -22]. The peaks of the NMU-8 conjugate were fractionated and the product was freeze-dried. The freeze-dried product of the obtained NMU-8 conjugate [8] -[22] was dissolved in distilled water and the peptide concentration was determined by amino acid analysis.

**[Table 5 -1]**

| | Structure | Conjugate name |
|---|---|---|
| [8] | 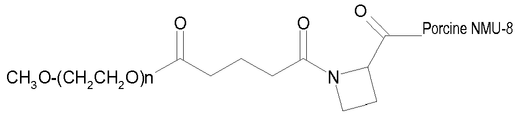 | PEG30k(Aze(2))-NMU-8 |
| [9] | 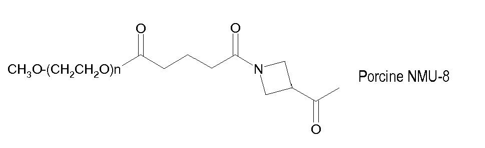 | PEG30k(Aze(3))-NMU-8 |
| [10] | 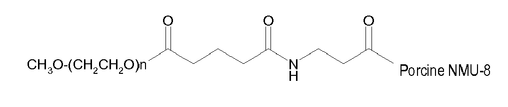 | PEG30k(βAla)-NMU-8 |
| [11] | 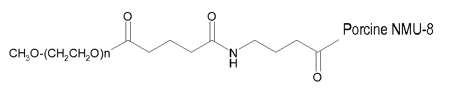 | PEG30k(AB)-NMU-8 |
| [12] | 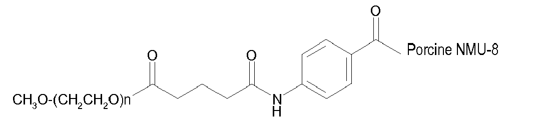 | PEG30k(AMBz)-NMU-8 |
| [13] | 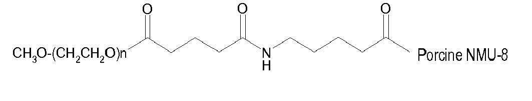 | PEG30k(AP)-NMU-8 |
| [14] | 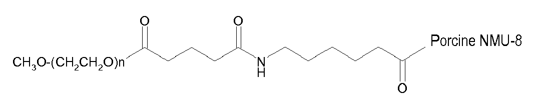 | PEG30k(ACP)-NMU-8 |
| [15] | 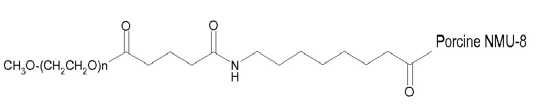 | PEG30k(AO)-NMU-8 |
| [16] | 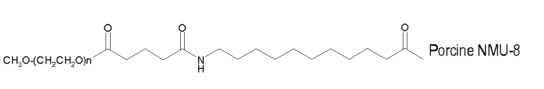 | PEG30k(AU)-NMU-8 |

**[Table 5 -2]**

| | | |
|---|---|---|
| [17] | 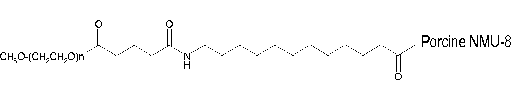 | PEG30k(AD)-NMU-8 |
| [18] | 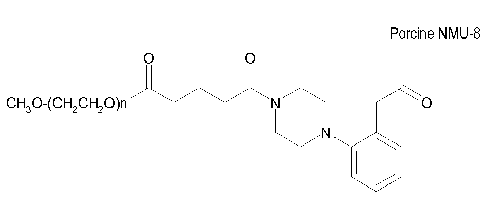 | PEG30k(PIP-P2A)-NMU-8 |
| [19] | 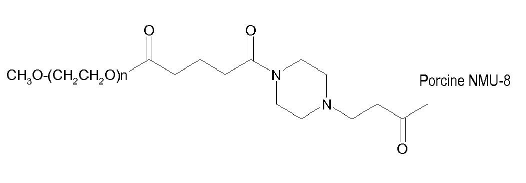 | PEG30k(PIP-PR)-NMU-8 |
| [20] | 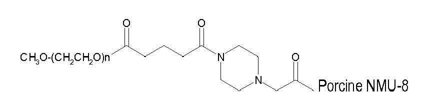 | PEG30k(PIP-AC)-NMU-8 |
| [21] | 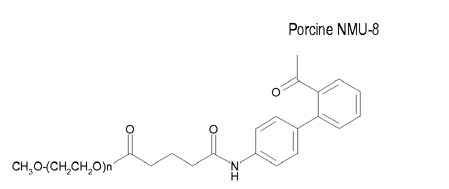 | PEG30k(BIP-4',2)-NMU-8 |
| [22] | 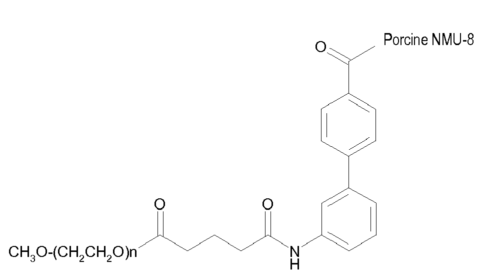 | PEG30k(BIP-3',4)-NMU-8 |

### Embodiment 6

### Preparation of a NMU-8 PEG conjugate using PEG-NHS (2)

The ω amino carboxylic acid -NMU-8 conjugate obtained by the same method as in Embodiment 5 or a NMU-8 itself in an amount equal to 2.0 µmol was dissolved in 500 µl dimethylsulfoxide. To the solution, added were 6.0 µmol (approximately 240 mg) of n-hydroxysuccimide introduced branched PEG (SUNBRIGHT GC2 -400GS2, Nippon Yushi) dissolved in 1 ml of dimethylsulfoxide, and subsequent 6.0 µmol of triethylamine. The reaction was carried out at room temperature for 2 hours. Acetic acid was added to the reaction solution in such an amount that the final concentration was 0.1 M. Further the solution was diluted with 40 ml of 0.1 M acetic acid and loaded into a SP - Sephadex C50 ion exchange column (capacity: 5 to 10 ml). After rinsing the column with 0.1 M acetic acid and then 10 mM ammonium formate/0.1 M acetic acid, the NMU-8 PEG conjugate was eluted from the column with 2 M ammonium formate/20% acetonitrile and then with 3.2 M ammonium formate/20% acetonitrile.

The eluate obtained was injected into a CAPCELL PAK column C8 column (SG300, 20 x 250 mm, Shiseido) at a flow rate of 9.0 ml/min. The concentration was further elevated linearly to Solution A 55%/Solution B 45% during a period of 60 min. in order to elute the NMU-8 PEG conjugate [23] -[37]. The peaks of the NMU-8 conjugate were fractionated and the product was freeze-dried. The freeze-dried product of the obtained NMU-8 conjugate [23 -38] was dissolved in distilled water and the peptide concentration was determined by amino acid analysis.

**[Table 6-1]**

| | Structure | Conjugate name |
|---|---|---|
| [23] | 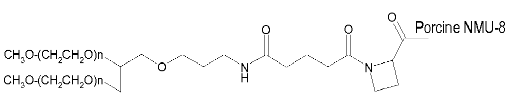 | (PEG20k)₂(Aze(2))-NMU-8 |
| [24] | 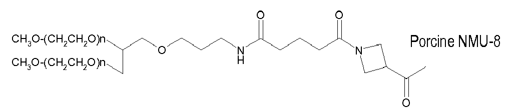 | (PEG20k)₂(Aze(3))-NMU-8 |
| [25] | 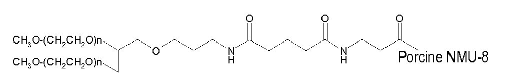 | (PEG20k)₂(βAla)-NMU-8 |
| [26] | 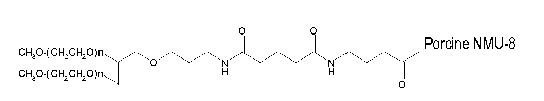 | (PEG20k)₂(AB)-NMU-8 |
| [27] | 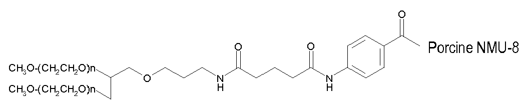 | (PEG20k)₂(AMBz)-NMU-8 |
| [28] | 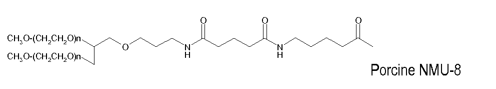 | (PEG20k)₂(AP)-NMU-8 |
| [29] | 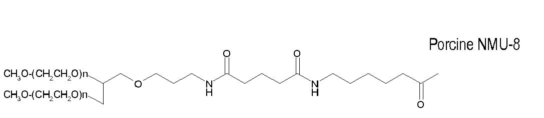 | (PEG20k)₂(ACP)-NMU-8 |
| [30] | 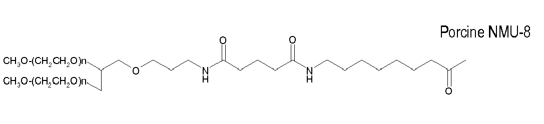 | (PEG20k)₂(AO)-NMU-8 |
| [31] | 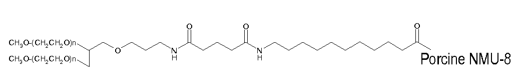 | (PEG20k)₂(AU)-NMU-8 |
| [32] | 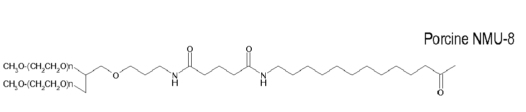 | (PEG20k)₂(AD)-NMU-8 |

**[Table 6 -2]**

| | | |
|---|---|---|
| [33] | 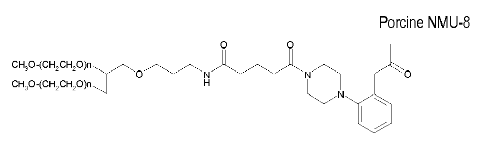 | (PEG20k)₂(PIP-P2A)-NMU-8 |
| [34] | 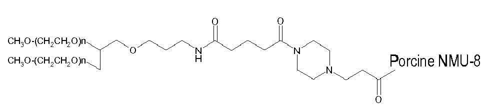 | (PEG20k)₂(PIP-PR)-NMU-8 |
| [35] | 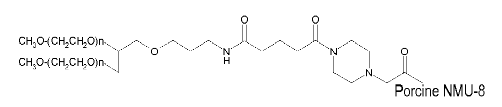 | (PEG20k)₂(PIP-AC)-NMU-8 |
| [36] | 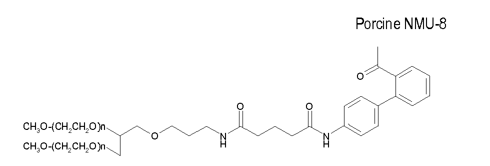 | (PEG20k)₂(BIP-4',2)-NMU-8 |
| [37] | 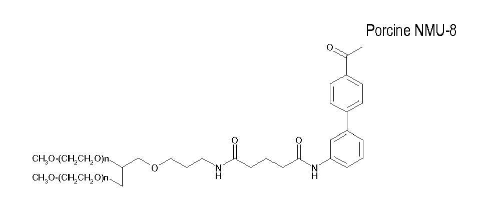 | (PEG20k)₂(BIP-3',4)-NMU-8 |
| [38] | 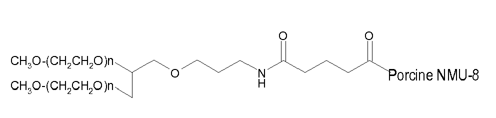 | (PEG20k)₂-NMU-8 |

### Embodiment 7

### Preparation of a NMU-8 PEG conjugate using PEG - Aldehyde

The ω aminocarboxylic acid -NMU-8 conjugate obtained by the same method as in Embodiment 5 or a NMU-8 itself in an amount equal to 1.0 µmol and 3.0 µmol of an Aldehyde group introduced PEG (SUNBRIGHT ME-300AL, Nippon Yushi) (approximately 100 mg) were dissolved in 1000 µl dimethylformaldehyde and further sodium cyanotrihydroborate in an amount equivalent to 20 µmol. The reaction was carried out at room temperature for 2 hours.

Acetic acid was added to the reaction solution in such an amount that the final concentration was 0.1 M. Further the solution was diluted with 40 ml of 0.1 M acetic acid and loaded into a SP - Sephadex C50 ion exchange column (capacity: 5 to 10 ml). After rinsing the column with 0.1 M acetic acid and then 10 mM ammonium formate/20% acetonitrile, the NMU-8 PEG conjugate was eluted from the column with 2 M ammonium formate/20% acetonitrile and then with 3.2 M ammonium formate/20% acetonitrile.

The eluate obtained was injected into a CAPCELL PAK column C8 column (SG300, 20 x 250 mm, Shiseido) which was equilibrated with Solution A 100% - Solution B 0% at a flow rate of 9.0 ml/min. After the concentration was further elevated abruptly to Solution A 55%/Solution B 45% during a period of 60 min., further elevated linearly to Solution A 25% - Solution B 75% in order to elute the NMU-8 PEG conjugate [39] -[54]. The peaks of the NMU-8 conjugate were fractionated and the product was freeze-dried.

The freeze-dried product of the obtained NMU-8 conjugate [39] -[54] was dissolved in distilled water and the peptide concentration was determined by amino acid analysis.

**[Table 7-1]**

| | Structure | Conjugate name |
|---|---|---|
| [39] | 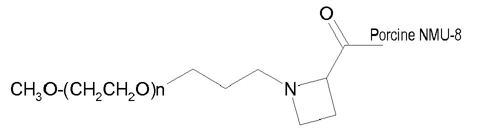 | PEG30k(NH-Aze(2))-NMU- 8 |
| [40] | 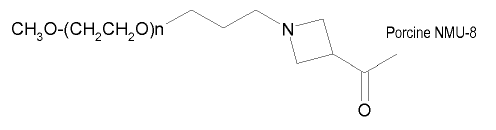 | PEG30k(NH-Aze(3))-NMU- 8 |
| [41] | 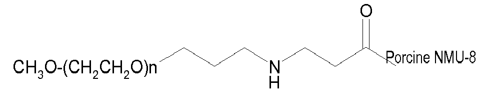 | PEG30k(NH-βAla)-NMU-8 |
| [42] | 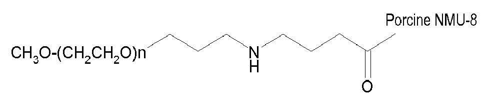 | PEG30k(NH-AB)-NMU-8 |
| [43] | 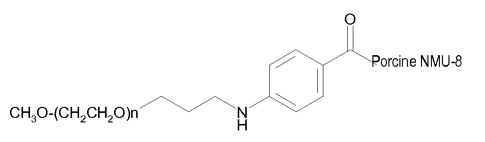 | PEG30k(NH-AMBz)-NMU- 8 |
| [44] | 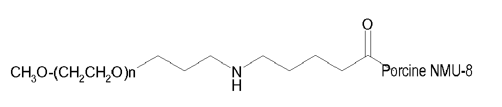 | PEG30k(NH-AP)-NMU-8 |
| [45] | 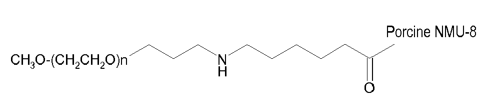 | PEG30k(NH-ACP)-NMU-8 |
| [46] | 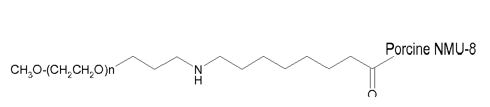 | PEG30k(NH-AO)-NMU-8 |
| [47] | 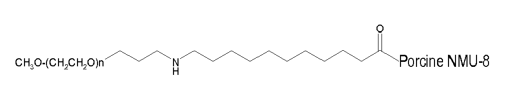 | PEG30k(NH-AU)-NMU-8 |

**[Table 7 -2]**

| | | |
|---|---|---|
| [48] | 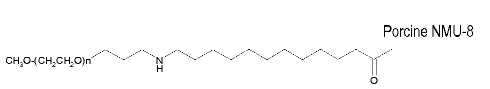 | PEG30k(NH-AD)-NMU-8 |
| [49] | 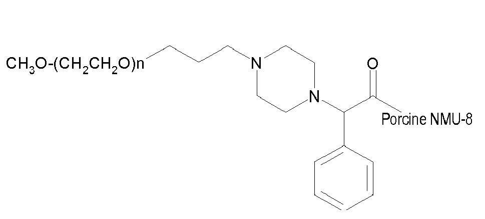 | PEG30k(N-PIP-P2A)-NMU-8 |
| [50] | 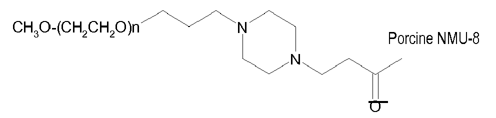 | PEG30k(N-PIP-PR)-NMU-8 |
| [51] | 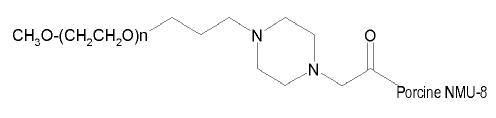 | PEG30k(N-PIP-AC)-NMU-8 |
| [52] | 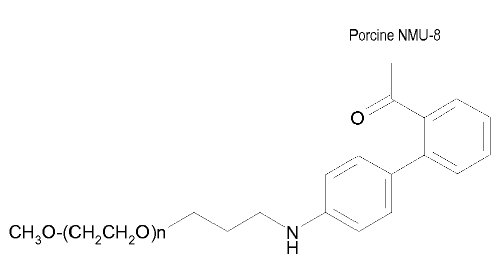 | PEG30k(NH-BIP-4',2)-NMU-8 |
| [53] | 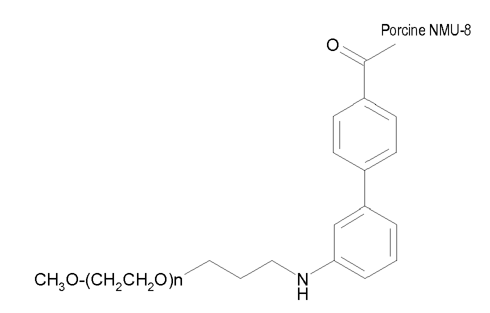 | PEG30k(NH-BIP-3',4)-NMU-8 |
| [54] | 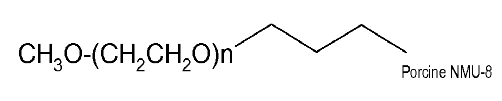 | PEG30k-NH-NMU-8 |

### Embodiment 8

### Preparation of a NMU-8 PEG conjugate using PEG -iodoacetamide

Rat NMU-8 (Anygen) [Sequence No. 6] wherein the Cys residue was introduced to the N-terminal (1.4 µmol = 1.7 mg) and 2.1 µmol (=125 mg) of iodoacetamide-induced PEG (SUNBRIGHT ME -3001A) were dissolved in 1 ml of 50 mM Tris -HCL (pH 8.5) and 5 mM EDTA and the reaction was carried out by gently rotating the light-shield reactor at room temperature for 2 hours. Acetic acid was added to the reaction solution in such an amount that the final concentration was 0.1 M. Further the solution was diluted with 40 ml of 0.1 M acetic acid and loaded into a SP - Sephadex C50 ion exchange column (capacity: 5 to 10 ml). After rinsing the column with 0.1 M acetic acid and then 10 mM ammonium formate/0.1 M acetic acid, the NMU-8 PEG conjugate was eluted from the column with 2 M ammonium formate/20% acetonitrile and then with 3.2 M ammonium formate/20% acetonitrile.

The eluate obtained was injected into a CAPCELL PAK column C8 column (SG300, 20 x 250 mm, Shiseido) which was equilibrated with Solution A 100% - Solution B 0% at a flow rate of 9.0 ml/min. After the concentration was further elevated abruptly to Solution A 55%/Solution B 45% during a period of 60 min., it was further elevated linearly to Solution A 25% - Solution B 75% in order to elute the NMU-8 PEG conjugate [55]. The peaks of the NMU-8 conjugate were fractionated and the product was freeze-dried.

The freeze-dried product of the obtained NMU-8 conjugate [55] was dissolved in distilled water and the peptide concentration was determined by amino acid analysis.

**[Table 8]**

| | Structure | Conjugate name |
|---|---|---|
| [55] | 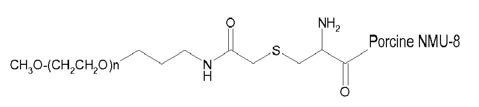 | PEG30k(NHAc)-Cys-NMU-8 |

### Test Example 3

### Receptor binding study of NMU-8 PEG conjugate

Human FM3 represented CHO cells (dhfr-) and human TRI represented CHO cells (dhfr-) were cultured under 5% carbon dioxide conditions at 37°C using a 10% dialyzed FBS-containing MEMα (Invitrogen) culture. The adhered cells were peeled with 10 ml of 0.1 mM EDTA containing DPB - S (Invitrogen) and cells were recovered by centrifugal separation at 4°C at 1000 rpm for 10 min. 15 ml of homogenizer buffer (10 mM NaHCO₃ (pH 7.4), 5 mM EDTA, Protein Inhibitors = 0.5 mM PMSF, 10 µg/ml Pepstatin A, 20 µg/ml Leupeptin, 4 µg/ml E -64) were added to the cell pellets obtained. The cellular membrane was destroyed using a polytron homogenizer (Kinematica GmbH) and the supernatant was collected by centrifugal separation under the following conditions: 4°C at 1000g, 10 min. The process was repeated twice. After the ultra centrifugation under the conditions: 4°C, 30,000 rpm, 60 min., 8 ml of homogenizer buffer was added to the pellets and suspended homogeneously to prepare a membrane fraction. A protein concentration of the FM3 represented CHO cell membrane fraction was 1.2 mg/ml and a protein concentration of the TGRI represented CHO cell membrane fraction was 1.1 mg/ml.

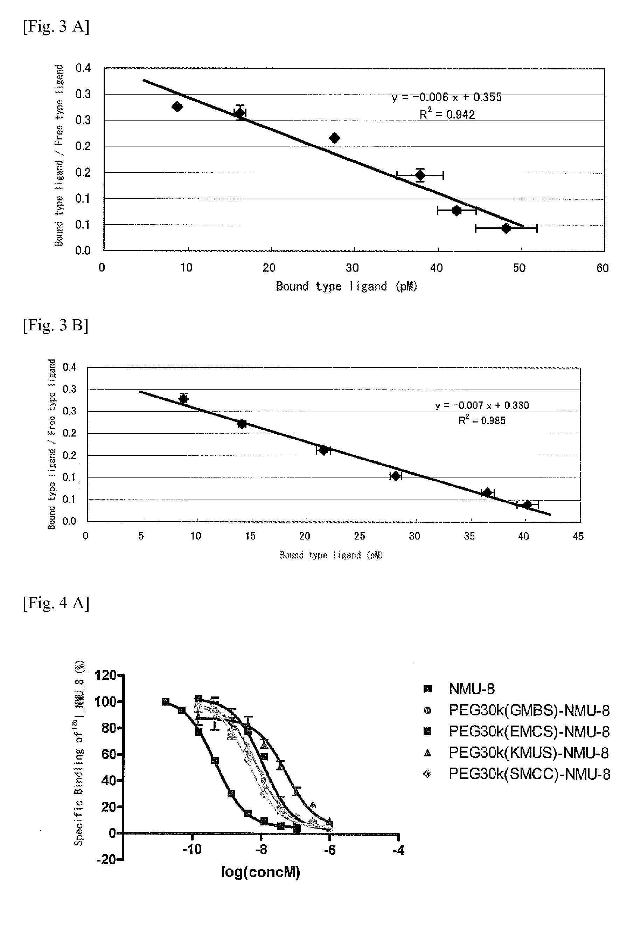

Next, the reactivity between the radiation labeled ligand ¹²⁵I-NMU 8 and each receptor represented cell membrane fraction was analyzed by scatchard analysis using a reactive buffer (50 mM HEPES (pH 6.8), 1 mM EDTA, 0.1% BSA, Protein Inhibitors).
The FM3 membrane fraction (diluted by 100 times) and the TGR1 membrane fraction (diluted by 50 times) 110 µl (diluted with the reaction buffer) were provided and 10 µl ¹²⁵I-NMU 8 was added. After reacting at 25°C for 75 min., 1.5 ml ice-cold rinsing buffer (20 mM (pH 7.4), 1 mM EDTA, 5 mM MgCl₂, 0.1% (CHAPS) was added. Immediately, this was passed though a sample manifold (Millipore) containing a glass filter which has been treated with polyethylene imine. The amount of the labeled ligand remaining in the filter was measured using a γ-counter. The results were as follows: FM3 membrane fraction contained Kd 164 pM, Bmax 4.8 pmol/mg protein and TGR1 fraction contained 135 pM, Bmax 2.0 pmol/mg protein. Both fractions had uniform binding sites (Fig. 3A(FM3/CHO cellular membrane fraction), Fig. 3B (TGR1/CHO cellular membrane fraction)). The protein concentrations in the FM3 and TGR1 membrane fractions were 12 and 22 µg/ml, respectively.

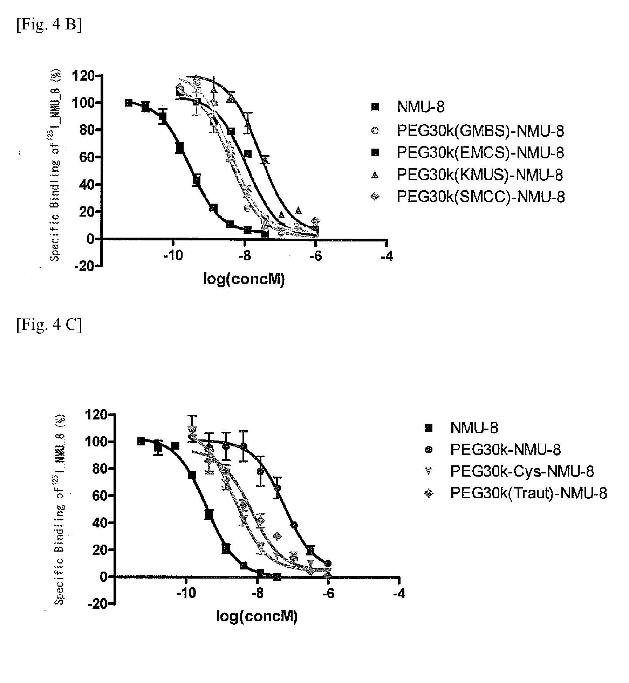

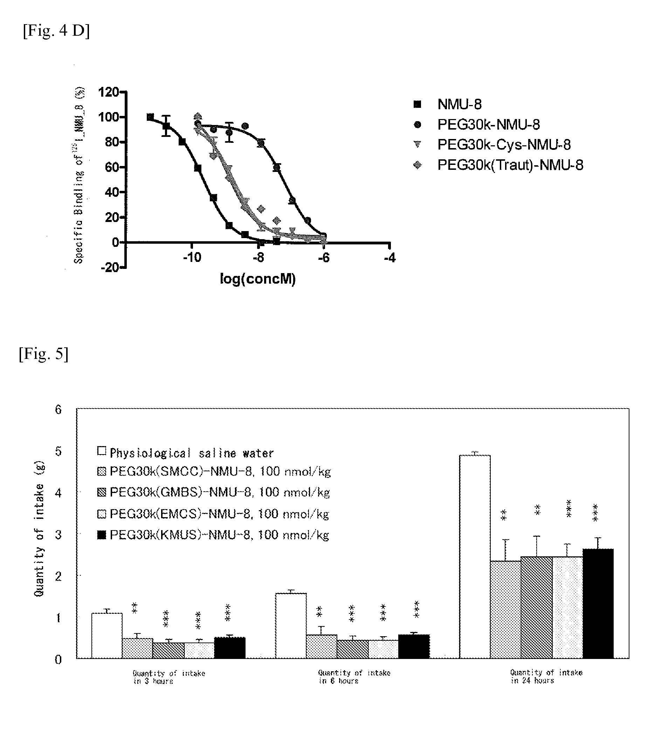

The affinity of each NMU-8 PEG conjugate to each receptor shown in Table 9 was evaluated by 125I-NMU 8 labeled ligand binding inhibition to the FM3 membrane fraction and TGR1 membrane fraction. Namely, dilution strings of NMU derivatives and PEG conjugates were provided and diluted membrane fraction solutions (200 µl) were added. The mixture was thoroughly blended with a Vortex and the labeled ligand 2 µl (final concentration 75 µM) was added to carry out the reaction at 25°C for 60 min. According to the aforementioned operations, the amount of binding of the labeled ligand remaining in the filter was measured and the IC50 values were calculated using graph pad PRISM (Fig. 4A (FM3 receptor binding), Fig. 4B (TGR1 receptor binding), Fig. 4C (FM3 receptor binding), Fig. 4D (TGR1 receptor binding) Table 9). In Fig. 4A and Fig. 4B, the horizontal axis indicates logarithmic values of the concentration of each derivative and the vertical axis indicates binding inhibition rates of various derivatives that are standardized by 0% to 100% residual radioactivity calculated from the binding of NMU.

**[Table 9]**

| | FM3 RBA IC₅₀ (nM) | TGR1 RBA IC₅₀ (nM) |
|---|---|---|
| NMU-8 | 0.19 | 0.089 |
| PEG30k (GMBS)-NMU-8 | 7.5 | 4.1 |
| PEG30k (EMCS)-NMU-8 | 11 | 11 |
| PEG30k (KMUS)-NMU-8 | 51 | 26 |
| PEG30k (SMCC)-NMU-8 | 4.5 | 4.3 |
| PEG30k-NMU-8 | 57 | 66 |
| PEG30k-Cys-NMU-8 | 2.3 | 1.9 |
| PEG30k (AD)-NMU-8 | 130 | 120 |
| PEG30k (AU)-NMU-8 | 60 | 54 |
| PEG30k (AMBz)-NMU-8 | 75 | 110 |
| PEG30k (ACP)-NMU-8 | 59 | |
| PEG30k (AP)-NMU-8 | 64 | |
| PEG30k (AN)-NMU-8 | 82 | |
| (PEG20k)₂(AD)-NMU-8 | 380 | 730 |
| (PEG20k)₂(AU)-NMU-8 | 330 | 560 |
| (PEG20k)₂(AMBz)-NMU-8 | 210 | 320 |
| (PEG20k)₂(ACP)-NMU-8 | 280 | 420 |
| (PEG20k)₂(AP) -NMU-8 | 230 | |
| (PEG20k₂(AB)-NMU-8 | 380 | |
| (PEG20k)₂-NMU-8 | 270 | 210 |
| PEG30k (NH-AD)-NMU-8 | 7 | 32 |
| PEG30k(NH-AU)-NMU-8 | 18 | 22 |
| PEG30k (NH-AMBz)-NMU-8 | 6.7 | 9.4 |
| PEG30k (NH-ACP)-NMU-8 | 7.3 | 37.9 |
| PEG30k (NH-AP)-NMU-8 | 6.3 | 6.9 |
| PEG30k (NH-AB)-NMU-8 | 2.4 | 2.5 |
| PEG30k-NH-NMU-8 | 11 | 11 |
| PEG30k (NHAc)-Cys-NMU-8 | 18 | 9.1 |
| PEG30k (traut)-NMU-8 | 6.4 | 1.2 |

### Test Example 4

### Antifeedant activity of NMU-8 PEG conjugate in mice

7 weeks old male C57BL/6J mice delivered from the Japan Charles River Company were raised for 5 to 10 days after delivery under a feeding environment regulated for temperature and humidity with lighting time (25°C, 12 hours of lighted period and 12 hours of dark period, light was lit at 8:00). Four animals were placed in one cage. After handling the mice for 5 to 8 days, the animals were housed singly in each cage where a floor mesh was spread and acclimated by intraperitoneal injection for 3 days prior to the administration of a peptide (conjugate). The animals acclimated were fasted for 16 hours from 18:00 prior to the administration of the peptide (conjugate). However, the animals had free access to drinking water). Subsequently, a peptide dissolved in physiological saline solution (conjugate) was intraperitoneally injected into the mice, namely 24.2 µM PEG30k (GMBS) - NMU-8 [1], 24.2 µM PEG30k (EMCS) - NMU-8 [2], 24.2 µM PEG30k (KMUS) - NMU-8 [3], 24.2 µM PEG30k (SMCC) - NMU-8 [4] were administered intraperitoneally at 10:00 on the day of administration to the mice such that the dosage of each solution (100µl) was 100 nmol/kg. After the injection of the peptide (conjugate) solution, MF feed which had been weighed (Oriental Yeast Industry) was given freely to the mice. Further, the residual amount of the feed was measured after 3, 6 and 24 hours. The food intakes at 3, 6 and 24 hours were calculated by subtracting the residual amount of the feed after 3, 6 and 24 hours from the amount of the feed that was originally given. The results are shown in Fig. 5. As clearly shown in Fig. 5, each NMU-8 PEG conjugate significantly suppressed the intakes of the feed at 3, 6 and 24 hours.

### Test Example 5

### Antifeedant activity of NMU-8 PEG conjugate in mice

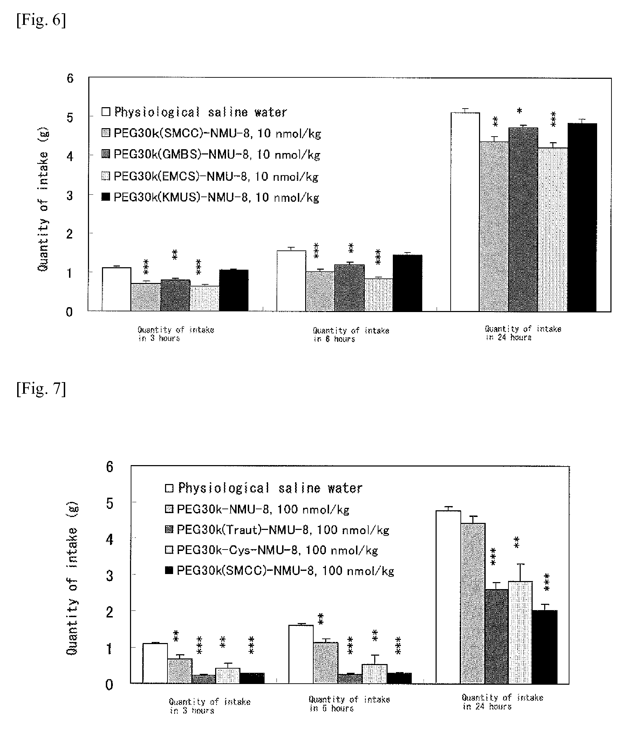

7 weeks old male C57BL/6J mice delivered from the Japan Charles River Company were raised for 5 to 10 days after delivery under a feeding environment regulated for temperature and humidity with lighting time (25°C, 12 hours of lighted period and 12 hours of dark period, light was lit at 8:00). Four animals were placed in one cage. After handling the mice for 5 to 8 days, the animals were housed singly in each cage where a floor mesh was spread and acclimated by intraperitoneal injection using a syringe (micro injector, syringe for insulin administration, Terumo) for 3 days prior to the administration of a peptide (conjugate). The animals acclimated were fasted for 16 hours from 18:00 prior to the administration of the peptide (conjugate). However, the animals had free access to drinking water). Subsequently, a peptide dissolved in physiological saline solution (conjugate) namely 2.39 µM PEG30k (GMBS) - NMU-8 [1], 2.39 µM PEG30k (EMCS) - NMU-8 [2], 2.39 µM PEG30k (KMUS) - NMU-8 [3], 2.39 µM PEG30k (SMCC) - NMU-8 [4] were administered intraperitoneally at 10:00 on the day of administration to the mice such that the dosage of each solution (100µl) was 10 nmol/kg. After the injection of the peptide (conjugate) solution, MF feed which had been weighed (Oriental Yeast Industry) was given freely to the mice. Further, the residual amount of the feed was measured after 3, 6 and 24 hours. The food intakes at 3, 6 and 24 hours were calculated by subtracting the residual amount of the feed after 3, 6 and 24 hours from the amount of the feed that was originally given. The results are shown in Fig. 6. As clearly shown in Fig. 6, each NMU-8 PEG conjugate significantly suppressed the intakes of the feed at 3, 6 and 24 hours.

### Test Example 6

### Antifeedant activity of NMU-8 PEG conjugate in mice

7 weeks old male C57BL/6J mice delivered from the Japan Charles River Company were raised for 5 to 10 days after delivery under a feeding environment regulated for temperature and humidity with lighting time (25°C, 12 hours of lighted period and 12 hours of dark period, light was lit at 8:00). Four animals were placed in one cage. After handling the mice for 5 to 8 days, the animals were housed singly in each cage where a floor mesh was spread and acclimated by intraperitoneal injection for 3 days prior to the administration of a peptide (conjugate). The animals acclimated were fasted for 16 hours from 18:00 prior to the administration of the peptide (conjugate). However, the animals had free access to drinking water). Subsequently, a peptide (conjugate) dissolved in physiological saline solution was intraperitoneally injected to the mice, namely 24.5 µM PEG30k NMU-8 [5], 24.5 µM PEG30k -Cys-NMU-8 [6], 24.5 µM PEG30k (Traut) - NMU-8 [7], 24.5 µM PEG30k (SMCC) - NMU-8 [4] were administered intraperitoneally at 10:00 on the day to the mice such that the dosage of each solution (100µl) was 100 nmol/kg. After the injection of the peptide (conjugate) solution, MF feed which had been weighed (Oriental Yeast Industry) was given freely to the mice. Further, the residual amount of the feed was measured after 3, 6 and 24 hours. The food intakes at 3, 6 and 24 hours were calculated by subtracting the residual amount of the feed after 3, 6 and 24 hours from the amount of the feed that was originally given. The results are shown in Fig. 7. As clearly shown in Fig. 7, each NMU-8 PEG conjugate significantly suppressed the intakes of the feed at 3, 6 and 24 hours.

### Test Example 7

### Antifeedant activity of NMU-8 PEG conjugate in mice

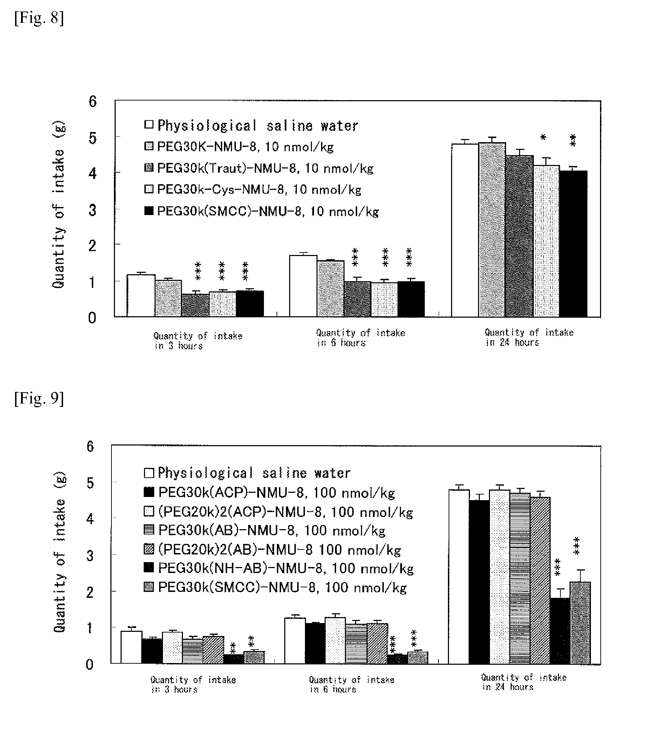

7 weeks old male C57BL/6J mice delivered from the Japan Charles River Company were raised for 5 to 10 days after delivery under a feeding environment regulated for temperature and humidity with lighting time (25°C, 12 hours of lighted period and 12 hours of dark period, light was lit at 8:00). Four animals were placed in one cage. After handling the mice for 5 to 8 days, the animals were housed singly in each cage where a floor mesh was spread and acclimated by intraperitoneal injection using a syringe (micro injector, syringe for insulin administration, Terumo) for 3 days prior to the administration of a peptide (conjugate). The animals acclimated were fasted for 16 hours from 18:00 prior to the administration of the peptide (conjugate). However, the animals were free access to drinking water). Subsequently, a peptide dissolved in physiological saline solution (conjugate) namely 2.51 µM PEG30k - NMU-8 [5], 2.51 µM PEG30k -Cys - NMU-8 [6], 2.51 µM PEG30k -(Traut) -NMU-8 [7], 2.51 µM PEG30k (SMCC) -NMU-8 [4] were administered intraperitoneally at 10:00 on the day of administration to the mice such that the dosage of each solution (100µl) was 10 nmol/kg. After the injection of the peptide (conjugate) solution, MF feed which had been weighed (Oriental Yeast Industry) was given freely to the mice. Further, the residual amount of the feed was measured after 3, 6 and 24 hours. The food intakes at 3, 6 and 24 hours were calculated by subtracting the residual amount of the feed after 3, 6 and 24 hours from the amount of the feed that was originally given. The results are shown in Fig. 8. As clearly shown in Fig. 8, each NMU-8 PEG conjugate significantly suppressed the intakes of the feed at 3, 6 and 24 hours.

### Test Example 8

### Antifeedant activity of NMU-8 PEG conjugate in mice

7 weeks old male C57BL/6J mice delivered from the Japan Charles River Company were raised for 5 to 10 days after delivery under a feeding environment regulated for temperature and humidity with lighting time (25°C, 12 hours of lighted period and 12 hours of dark period, light was lit at 8:00). Four animals were placed in one cage. After handling the mice for 5 to 8 days, the animals were housed singly in each cage where a floor mesh was spread and acclimated by intraperitoneal injection for 3 days prior to the administration of a peptide (conjugate). The animals acclimated were fasted for 16 hours from 18:00 prior to the administration of the peptide (conjugate). However, the animals has free access to drinking water). Subsequently, a peptide (conjugate) dissolved in physiological saline solution was intraperitoneally injected to the mice, namely 24.3 µM PEG30k (ACP) -NMU-8 [14], 24.3 µM (PEG20k)2 (ACP)-NMU-8 [29], 24.3 µM PEG30k (AB) -NMU-8 [11], 24.3 µM (PEG20k)2 (AB) - NMU-8 [26], 24.3 µM PEG30k (NH -AB) -NMU-8 [42], 24.3 µM PEG30k (SMCC) - NMU-8 [4] were administered intraperitoneally at 10:00 on the day of administration to the mice such that the dosage of each solution (100µl) was 100 nmol/kg. After the injection of the peptide (conjugate) solution, MF feed which had been weighed (Oriental Yeast Industry) was given freely to the mice. Further, the residual amount of the feed was measured after 3, 6 and 24 hours. The food intakes at 3, 6 and 24 hours were calculated by subtracting the residual amount of the feed after 3, 6 and 24 hours from the amount of the feed that was originally given. The results are shown in Fig. 9. As clearly shown in Fig. 9, each NMU-8 PEG conjugate significantly suppressed the intakes of the feed at 3, 6 and 24 hours.

### Test Example 9

### Antiobesity activity of NMU-8 PEG conjugate in mice

5 weeks old male C57BL/6J mice delivered from the Japan Charles River Company were raised for 18 to 28 weeks to after delivery under a feeding environment regulated for temperature and humidity with lighting time (25°C, 12 hours of lighted period and 12 hours of dark period, light was lit at 8:00) using a special feed (58%, high fat diet, D12331, Research Diet Inc.). Five animals were placed in one cage. After handling the mice at a frequency of once every 1 to 2 weeks, the animals were moved under the following conditions: light and dark cycle of 12 hours (light on 0550 h). The animals were housed singly in each cage where a floor mesh was spread and acclimated for more than one week when the average bodyweight of the 60 mice exceeded 50 g. The mice when spilled food was observed were excluded and those which satisfied the range of the mean values ± 2SD were selected. 36 animals were selected on a completely random assignment basis based on the bodyweight on the day before administration as a single variable. The bodyweight of the acclimated mice and the food intake were measured at the time between 13:30 to 14:30 on the day before administration of the peptide (conjugate). The bodyweight of the mice and the food intake on the day of administration of the peptide (conjugate) were also measured at the time between 13:30 and 14:30. Subsequently, the peptide (conjugate) dissolved in physiological saline solution, namely each 100 µl solution of the following peptide solutions: 13.5 µM, 4.5 µM, 1.35 µM, 0.45 µM or 0.135 µM of PEG30k (EMCS) -NMU-8 [2] was administered subcutaneously on the back of the mice at the time between 15:00 and 16:00. After the treatment with the peptide solution (conjugate), the mice were allowed to eat food and behave freely until the time between 13:30 and 14:30 when bodyweight and food intake will be measured on the following day. Measurement of bodyweight of the mice and food intake, and administration of the peptide were repeated 7 times. After the 7^{th} administration at the time between 15:00 and 16:00, bodyweight of the mice and food intake were measured on the following day (8^{th} day) and on the 10^{th} day. The daily food intake was calculated by subtracting the remaining amount of food on the following day from the amount of the feed given.

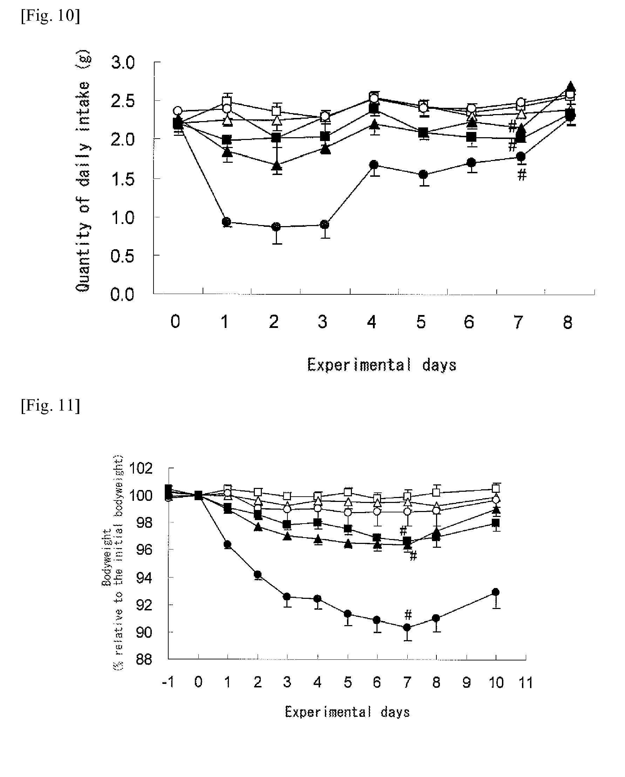

Fig. 10 shows the results of measurement of bodyweight and food intake.
[In the figure, ●: 27 nmol/kg, ○: 9 nmol/kg, △: 2.7 nmol/kg, ▲: 0.9 nmol/kg, ■: 0.27 nmol/kg, □: physiological saline. #: food intake on the 7^{th} day or bodyweight changes tested by William's test. The level of significance was less than 0.25 (P <0.25)]

### Test Example 10

### Antifeedant activity of NMU-8 PEG conjugate in mice

7 weeks old male C57BL/6J mice delivered from the Japan Charles River Company were raised for 5 to 10 days after delivery under a feeding environment regulated for temperature and humidity with lighting time (25°C, 12 hours of lighted period and 12 hours of dark period, light was lit at 8:00). Four animals were placed in one cage. After handling the mice for 5 to 8 days, the animals were housed singly in each cage where a floor mesh was spread and acclimated by intraperitoneal injection for 3 days prior to the administration of a peptide (conjugate). The animals acclimated were fasted for 16 hours from 18:00 prior to the administration of the peptide (conjugate). However, the animals had free access to drinking water). Subsequently, a peptide dissolved in physiological saline solution (conjugate) was intraperitoneally injected to the mice, namely 7.47 µM, 2.49 µM, 0.75 µM, 0.25 µM PEG30k (N -PIP -AC) -NMU-8 [51] were administered intraperitoneally at 10:00 on the day of administration to the mice such that the dosage of each solution (100µl) was 30, 10, 3, 1 nmol/kg. After the injection of the peptide (conjugate) solution, MF feed which had been weighed (Oriental Yeast Industry) was given freely to the mice. Further, the residual amount of the feed was measured after 3 and 6 hours. The food intakes at 3 and 6 hours were calculated by subtracting the residual amount of the feed after 3 and 6 hours from the amount of the feed that was originally given. The results are shown in Fig. 11. As clearly shown in Fig. 11, the PEG30k -N -PIP -Ac-NMU-8 dose-dependently suppressed the food intake at 3 and 6 hours. #: food intake was tested by William's test. It indicated that the level of significance was less than 0.0025 (P < 0.025).

### Embodiment 9

### Preparation of NMU-8 PEG conjugate using PEG -NHS (3)

According to the method as in Embodiment 5, a porcine NMU-8 (Bachem) [Sequence No. 2], Boc -Gly (Peptide Institute), Boc -9 -amino nonanic acid (Tyger Sci, Inc.) or Boc -2 -Abz -OH, Boc -3 -Abz -OH, Boc -4 = Abz -OH (All the above were provided by Watanabe Chemicals Industries) were reacted to obtain ω amino carboxylic acid -NMU-8 conjugate.

According to the same method as in Embodiment 5, the obtained ω amino carboxylic acid -NMU-8 conjugate and n-hydroxysuccimide introduced PEG (SUNBRIGHT MEGC -30TS, Nippon Yushi) were reacted and the obtained NMU-8 PEG conjugates [56 -60] were freeze-dried.
The freeze-dried products of the obtained NMU-8 PEG conjugates [56 -60] were dissolved in distilled water and the peptide concentrations were determined by amino acid analysis.

**[Table 10]**

| | Structure | Conjugate name |
|---|---|---|
| [56] | 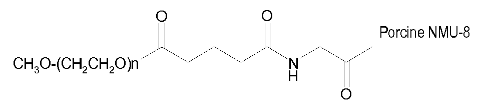 | PEG30k(Gly)-NMU-8 |
| [57] | 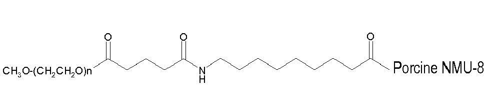 | PEG30k(AN)-NMU-8 |
| [58] | 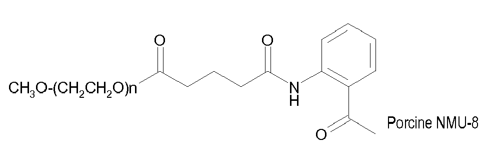 | PEG30k(Abz(2))-NMU-8 |
| [59] | 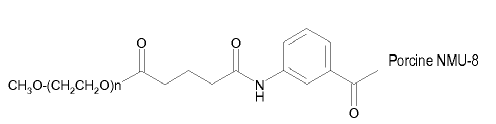 | PEG30k(Abz(3))-NMU-8 |
| [60] | 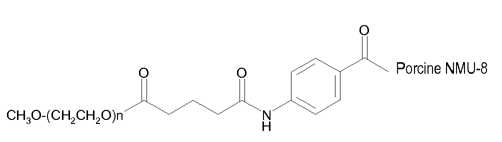 | PEG30k(Abz(4))-NMU-8 |

### Embodiment 10

### Preparation of NMU-8 PEG conjugate using PEG -NHS (4)

According to the method as in Embodiment 5, a rat NMU-8 (Anygen) wherein a L -Lys residue was introduced to the N-terminal and n-hydroxysuccimide introduced PEG (SUNBRIGHT MEGC -30 TS, Nippon Yushi) were reacted and the obtained NMU-8 PEG conjugate [61] was freeze-dried.
The freeze-dried product of the NMU-8 PEG conjugate [61] was dissolved in distilled water and the peptide concentration was determined by the amino acid analysis.

**[Table 11]**

| | | |
|---|---|---|
| [61] | 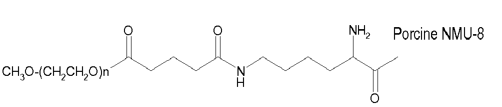 | PEG30k(εLys)-NMU-8 |

### Embodiment 11

### Preparation of a NMU-8 PEG conjugate using PEG-NHS (5)

A porcine NMU-8 (Bachem) [Sequence No. 2] 18 µpmol (20.0 mg) was dissolved in 500 µl of dimethylformamide. Subsequently, 27 µmol of sodium cyanotrihydroborate and 54 µmol of triethylamine were added to a solution prepared by dissolving 27 µmol equivalent Fmoc -cis -1, 4 -aminocyclohexane carboxylic acid (by Watanabe Kagaku Kogyo) in 500 µl of dimethylformamide. The reaction was carried out at room temperature for 1 hour. The reaction mixture was concentrated to approximately 100 µl by evaporator. Further, diethylamine in an equivalent to 54 µmol was added and the reaction was carried out at room temperature for 2 hours in order to remove the Fmoc group. After the reaction solution was neutralized using 0.1 M acetic acid, distilled water containing 0.1% FTA was added to dilute it to 20 ml. The sample solution was injected to a CAPCELL PAK C18 column (MG11, 20 x 25;0 mm, Shiseido) which was equilibrated by Solution A 100% and Solution B 0% at a flow rate of 9.0 ml/ml. After sharply elevating the concentration to Solution A 75% - Solution B 25%, further the concentration was linearly elevated to Solution A 50% and Solution B 50% during a period of 40 min. in order to elute a NMU-8 PEG conjugate [62]. A fraction of the peak of the NMU-8 PEG conjugate was separated and further freeze-dried.
The freeze-dried product of the NMU-8 PEG conjugate obtained [62] was dissolved in distilled water and the peptide concentration was analyzed by amino acid analysis.

**[Table 12]**

| | | |
|---|---|---|
| [62] | 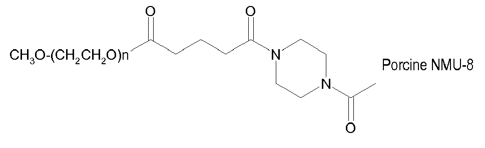 | PEG30k(cHex)-NMU-8 |

### Test Example 11

### Antiobesity activity of NMU-8 PEG conjugate in mice (2)

7 weeks old male C57BL/6J mice delivered from the Japan Charles River Company were raised for 23 weeks after delivery under a feeding environment regulated for temperature and humidity with lighting time (25°C, 12 hours of lighted period and 12 hours of dark period, light was lit at 8:00) using a special feed (58% high fat diet, D12331, Research Diet Inc.). Five animals were placed in one cage. The animals were moved to other conditions of a 12 hour dark and light cycle (light on at 0400 h) and acclimated under such conditions for more than 1 week. The animals were housed singly in each cage where a floor mesh was spread and acclimated with handling for 6 days prior to administration of the peptide (conjugate). The mice when spilled food was observed were excluded and those which satisfied the range of the mean values±2SD were selected. 24 animals were selected on a completely random assignment basis based on the bodyweight on the day before administration as a single variable. The bodyweight on the day of administration of the peptide solution (conjugate) was measured at the time between 13:00 and 15:00. The peptide dissolved in physiological saline solution (conjugate), namely 7.5 µM, 2.5 µM, 0.75 µM of PEG30k (N -PIP -AC) -NMU-8 [51] was administered subcutaneously on the back of the mice at a dose of the bodyweight of each mice x 4 µl. After the treatment with the peptide solution (conjugate), the mice were allowed to eat food and behave freely until the time between 13:30 and 15:00 when bodyweight was measured on the following day.
The bodyweight was measured at the time between 15:00 and 16:00 before the treatment on the arbitrary days. The dose was calculated based on the bodyweight on the nearest day from the day of administration. The administration to the mice was repeated 26 times.

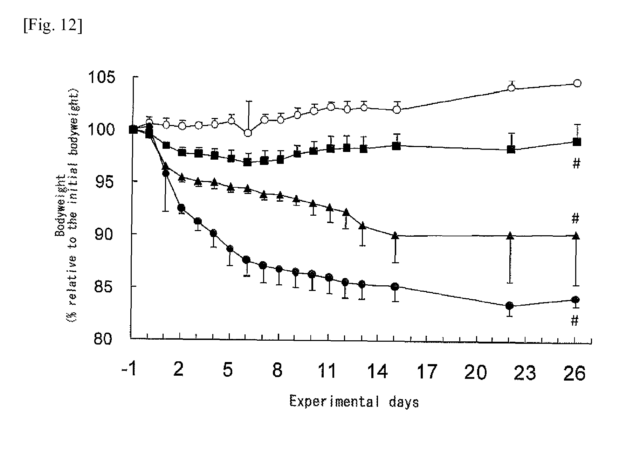

The results of measurement of bodyweight are shown in Fig. 12.
[In the figure, ●: 30 nmol/kg, ▲: 10 nmol/kg, ■: 3 nmol/kg, ○: physiological saline. #: Examined by William's test for changes in bodyweight on the 26^{th} day. This indicated that the level of significance was less than 0.025 (P <0.025).

### Formulation Example 1

| | | |
|---|---|---|
| (1) | Compound of the present invention | 50 mg |
| (2) | Lactose | 34 mg |
| (3) | Corn starch | 10.6 mg |
| (4) | Corn starch (in paste form) | 5 mg |
| (5) | Magnesium stearate | 0.4 mg |
| (6) | Calcium carboxymethyl cellulose | 20 mg |
| | Total | 120 mg |

According to the common method, the aforementioned items (1) through (6) were mixed and tablets were punctured using a tablet puncturing device to produce tablets.

### Embodiment 12

### Preparation of a NMU-8 PEG conjugate using PEG-NHS (6)

A ω amino carboxylic acid -NMU-8 conjugate obtained by the same method as in Embodiment 5 [Gly-NMU-8, β Ala-NMU-8, or AB-NMU-8] and Boc-ω amino carboxylic acid [Bo Gly (Peptide Institution), Boc-β-Ala-OH, Boc-Abu (4)-OH, Boc-Ape (5)-OH, Boc-Acp (6)-OH (by Watanabe Chemical Industries)] were reacted by the same method as in Embodiment 5 to obtain ω amino carboxylic acid (linked) -NMU-8 conjugate.

The ω amino carboxylic acid (linked)-NMU-8 conjugate obtained by the method as in Embodiment 5 and PEG containing n-hydroxysuccimide (SUNBRIGHT MEGC - 30TS, Nippon Yushi) were reacted and a NMU-8 PEG conjugate [63 -69] was treated by freeze drying.

The freeze-dried product of the NMU-8 PEG conjugate [63 -69] was dissolved in distilled water and the peptide concentration was measured by amino acid analysis.

**[Table 13]**

| | | |
|---|---|---|
| [63] | 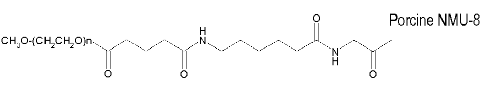 | PEG30k(ACP-G1y)-NMU-8 |
| [64] | 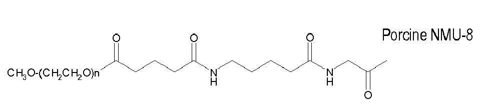 | PEG30k(AP-Gly)-NMU-8 |
| [65] | 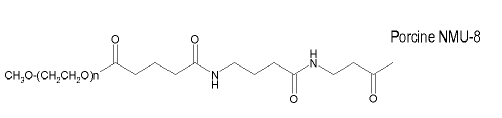 | PEG30k(AB-βAla)-NMU-8 |
| [66] | 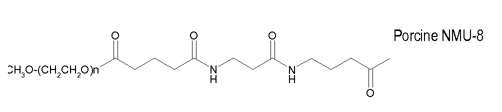 | PEG30k(βAla-AB)-NMU-8 |
| [67] | 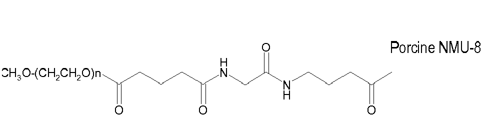 | PEG30k(AB-Gly)-NMU-8 |
| [68] | 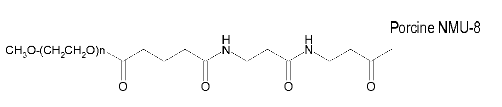 | PEG30k(βAla-βAla)-NMU-8 |
| [69] | 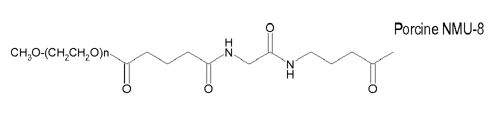 | PEG30k(Gly-AB)-NMU-8 |
| [70] | 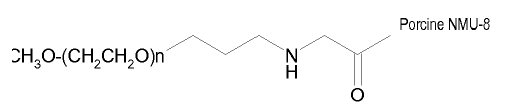 | PEG30k(NH-Gly)-NMU-8 |
| [71] | 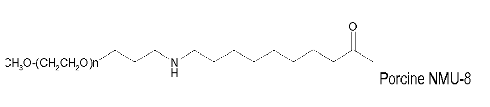 | PEG30k(NH-AN)-NMU-8 |
| [72] | 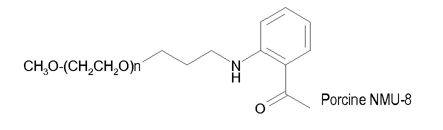 | PEG30k(NH-Abz(2))-NMU-8 |
| [73] | 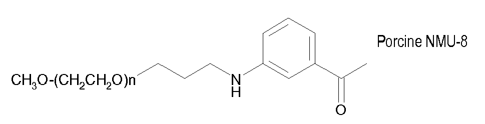 | PEG30k(NH-Abz(3))-NMU-8 |
| [74] | 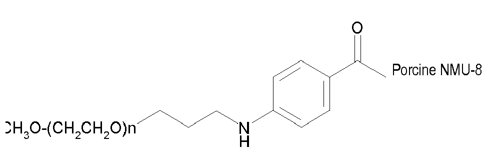 | PEG30k(NH-Abz(4))-NMU-8 |
| [75] | 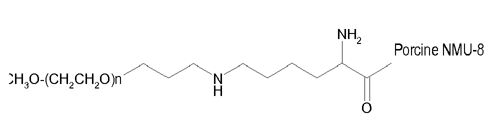 | PEG30k(εNH-Lys)-NMU-8 |
| [76] | 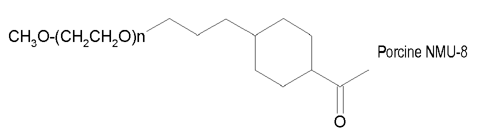 | PEG30k(NH-cHex)-NMU-8 |
| [77] | 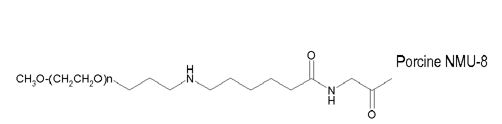 | PEG30k(NH-ACP-Gly)-NMU- 8 |
| [78] | 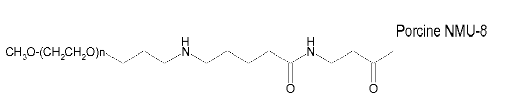 | PEG30k(NH-AP-βAla)-NMU-8 |
| [79] | 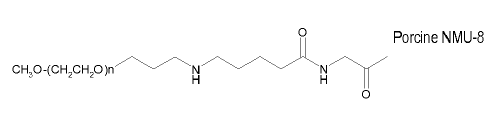 | PEG30k(NH-AP-Gly)-NMU-8 |

**[Table 14 -2]**

| | | |
|---|---|---|
| [80] | 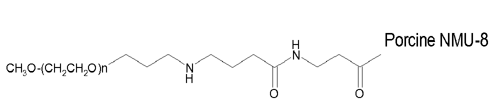 | PEG30k(NH-AB-βAla)-NMU-8 |
| [81] | 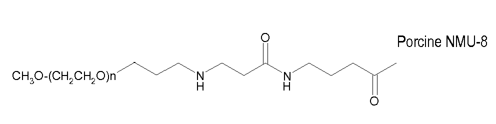 | PEG30k(NH-βAla-AB)-NMU-8 |
| [82] | 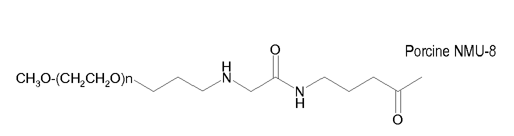 | PEG30k(NH-AB-Gly)-NMU-8 |
| [83] | 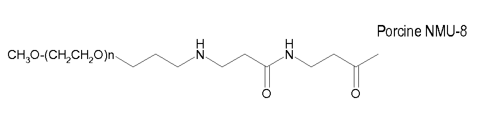 | PEG30k(NH-βAla-βAla)-NMU-8 |
| [84] | 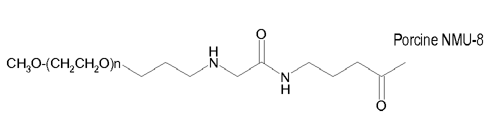 | PEG30k(NH-Gly-AB)-NMU-8 |

The freeze-dried product and the PEG containing an aldehyde group (SUNBRIGHT ME -300AL, Japan Yushi) were reacted by the same method as in Embodiment 7 to obtain a NMU-8 PEG conjugate [85 -90]. Further, the Fmoc group was removed by the same method as in Embodiment 11 to obtain a NMU-8 PEG conjugate [91 -96].

The freeze-dried product of the NMU-8 PEG conjugate [85 -96] obtained was dissolved in distilled water and the peptide concentration was measured by amino acid analysis.

**[Table 15]**

| | | |
|---|---|---|
| [85]-[88] | 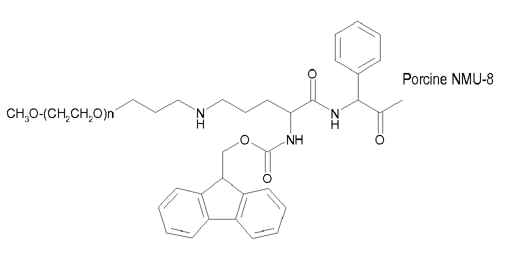 | PEG30k(δNH-Orn(Fmoc)-Phg)-NMU8 |
| | | PEG30k(δNH-_{D-}Orn(Fmoc)-Phg)-NMU8 |
| | | PEG30k(δNH-Orn(Fmoc)-DPhg)-NMU8 |
| | | PEG30k(δNH-_{D-}Orn(Fmoc)-_{D-} Phg)-NMU8 |
| [89]-[90] | 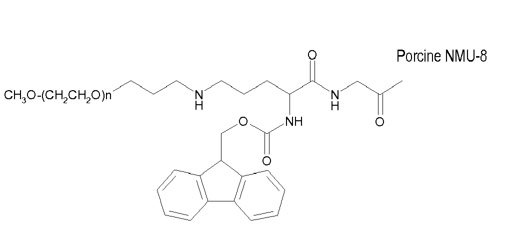 | PEG30k(δNH-Orn(Fmoc)-Gly)-NMU8 |
| | | PEG30k(δNH-^{III}Orn(Fmoc)-Gly)-NMU8 |
| [91]-[94] | 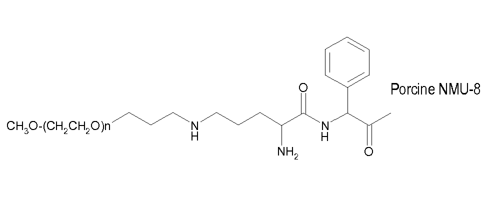 | PEG30k(δNH-Orn-Phg)-NMU8 |
| | | PEG30k(δNH-_{D-}Orn-Phg)-NMU8 |
| | | PEG30k(δNH-Orn-_{D-}Phg)-NMU8 |
| | | PEG30k(δNH-_{D-}Orn-_{D-}Phg)-NMU8 |
| [95]-[96] | 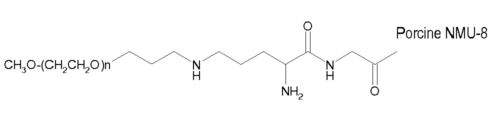 | PEG30k(δNH-Orn-Gly)-NMU8 |
| | | PEG30k(δNH-_{D-}Orn-Gly)-NMU8 |

### Embodiment 15

### Preparation of a NMU-8 PEG conjugate using PEG-Aldehyde (4)

A porcine NMU-8 containing Phg (L or D) or Phe (L or D) at the N-terminus (Sigmagenosys) and Boc-Ape (5) -OH (by Watanabe Chemical Industry Co., Ltd.) were reacted by the same method as in Embodiment 5 to obtain a NMU-8 conjugate containing ω aminocarboxylic acid-Phg (L or D) or Phe (L or D).

A NMU-8 conjugate containing the ω aminocarboxylic acid-Phg (L or D) or Phe (L or D), porcine NMU-8 containing Phg (L or D) or Phe (L or D) at the N-terminus (Sigmagenosys) and a PET containing an aldehyde group (SUNBRIGHT ME -300AL, Nippon Yushi) were reacted by the same method as in Embodiment 7 to obtain a NMU-8 conjugate [97 -104].
The freeze-dried product of the NMU-8 PEG conjugate [97 -104] obtained was dissolved in distilled water and the peptide concentration was measured by amino acid analysis.

**[Table 16]**

| | | |
|---|---|---|
| [97]-[98] | 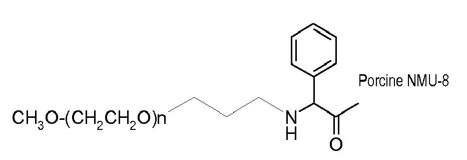 | PEG30k(NH-Phg)-NMU-8 PEG30k(NH-_{D-}Phg)-NMU-8 |
| [99]-[100] | 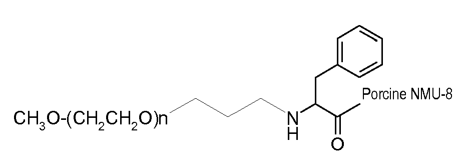 | PEG30k(NH-Phe)-NMU-8 PEG30k(NH-_{D-}Phe)-NMU-8 |
| [101]-[102] | 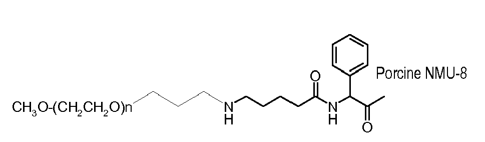 | PEG30k(NH-AP-Phg)-NMU-8 |
| | | PEG30k(NH-AP-_{D-}Phg)-NMU-8 |
| [103]-[104] | 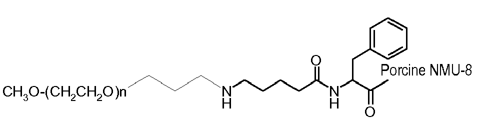 | PEG30k(NH-AP-Phe)-NMU-8 |
| | | PEG30k(NH-AP-_{D-}Phe)-NMU-8 |

### Embodiment 16

### Preparation of a NMU-8 PEG conjugate using PEG-Aldehyde (5)

Porcine NMU-8 containing Lys at the N-terminus (Anygen) and a PEG containing an aldehyde group (SUNBRIGHT ME -300AL, Nippon Yushi) were reacted with 0.1M acetic acid (Conditions at pH 5.0) instead of dimethylformamide in order to carry out reductive amination selectively at amino group by the same method as in Embodiment 7 to obtain a NMU-8 PEG conjugate [105].

The freeze-dried product of the NMU-8 PEG conjugate [105] obtained was dissolved in distilled water and the peptide concentration was measured by amino acid analysis.

**[Table 17]**

| | | |
|---|---|---|
| [105] | 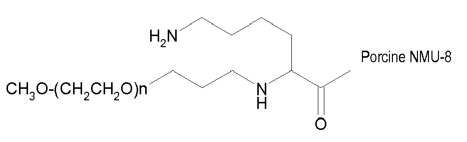 | PEG30k(αNH-Lys)-NMU-8 |

### Embodiment 17

### Preparation of a NMU-8 PEG conjugate using PEG-Aldehyde (6)

A NMU-8 PEG conjugate [75, 105], a ω aminocarboxylic acid [Boc-Gly (Peptide Institution) or Boc-Ape (5) -OH (Watanabe Chemical Industries Corporation)] were reacted by the same method as in Embodiment 5 to obtain a NMU-8 PEG conjugate [106 -109].

The freeze-dried product of the NMU-8 PEG conjugate [106 -109] obtained was dissolved in distilled water and the peptide concentration was measured by amino acid analysis.

**[Table 18]**

| | | |
|---|---|---|
| [106] | 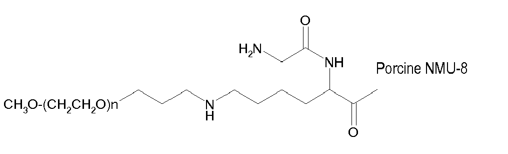 | PEG30k(ωNH-Lys(aNH-Gly))-NMU-8 |
| [107] | 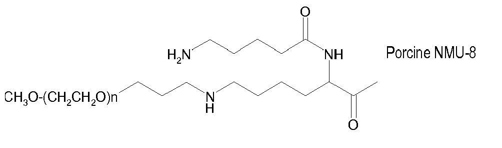 | PEG30k(ωNH-Lys(aNH-AP))-NMU-8 |
| [108] | 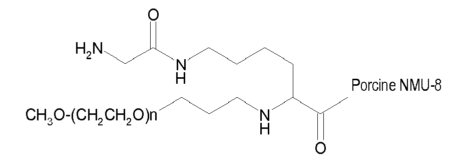 | PEG30k(αNH-Lys(εNH-Gly))-NMU-8 |
| [109] | 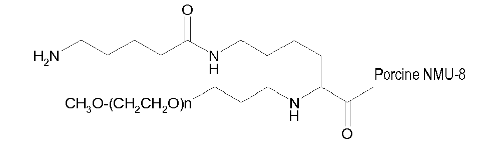 | PEG30k(αNH-Lys(εNH-AP))-NMU-8 |

### Embodiment 18

### Preparation of a NMU-8 PEG conjugate using PEG-Aldehyde (7)

A NMU-8 PEG conjugate [75 or 105] and a PEG containing an aldehyde group (SUNBRIGHT ME -300 AL, Nippon Yushi) were reacted by the same method as in Embodiment 7 to obtain a NMU-8 PEG conjugate [110].

The freeze-dried product of the NMU-8 PEG conjugate [110] obtained was dissolved in distilled water and the peptide concentration was measured by amino acid analysis.

**[Table 19]**

| | | |
|---|---|---|
| [110] | 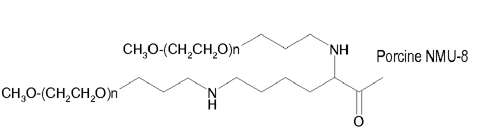 | PEG30k(α,εNH-Lys)-NMU-8 |

### Embodiment 19

### Preparation of a NMU-8 PEG conjugate using PEG-Aldehyde (8)

A NMU-8 PEG conjugate [51] and a PEG containing an aldehyde group (SUNBRIGHT ME -200AL, ME -400AL2, GL 2 -200AL, GL 4 -400 AL, GL 2 -400AL, and GL 3 -400Al100U, Nippon Yushi) were reacted by the same method as in Embodiment 7 to obtain a NMU-8 PEG conjugate [111 -116].

The freeze-dried product of the NMU-8 PEG conjugate [111 -116] obtained was dissolved in distilled water and the peptide concentration was measured by amino acid analysis.

**[Table 20]**

| | | |
|---|---|---|
| [111] | 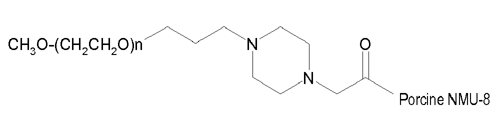 | PEG20k(N-PIP-AC)-NMU-8 |
| [112] | 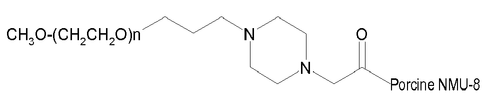 | PEG40k(N-PIP-AC)-NMU-8 |
| [113] | 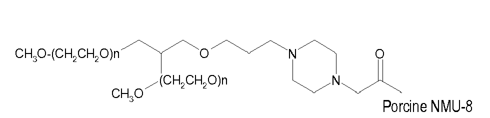 | (PEG10k)₂(N-PIP-AC)-NMU-8 |
| [114] | 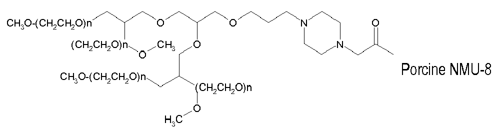 | (PEG10k)₄(N-PIP-AC)-NMU-8 |
| [115] | 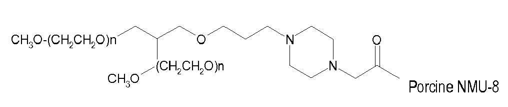 | (PEG20k)₂(N-PIP-AC)-NMU-8 |
| [116] | 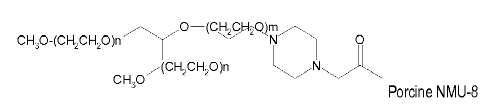 | (PEG20k)₂PEG10k-(N-PIP-AC)-NMU-8 |

### [Possible Use in Industry]

According to the present invention, a new antifeedant is provided.

## Claims

1. A neuromedin U derivative
which is a polypeptide consisting of an amino acid sequence which is bound with a methoxypolyethylene glycol(s) via a linker,
said amino acid sequence contains at least 8 amino acids of the C-terminus of an amino acid sequence of neuromedin U, and is the same or substantially the same as the amino acid sequence of neuromedin U, and
which is represented by a formula: 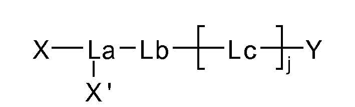 wherein
Y represents a polypeptide consisting of a amino acid sequence which contains at least 8 amino acids of the C-terminus of neuromedin U and is the same or substantially the same as the amino acid sequence of neuromedin U;
X represents a methoxyethylene glycol;
X' is absent or represents a methoxypolyethylene glycol;
the part represented by a formula (II): 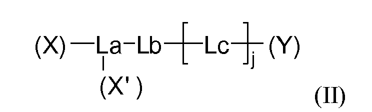 represents a linker,
La represents a divalent or trivalent group selected from 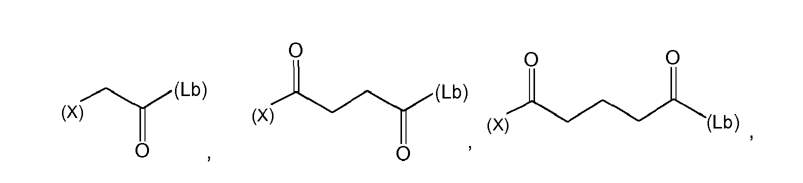 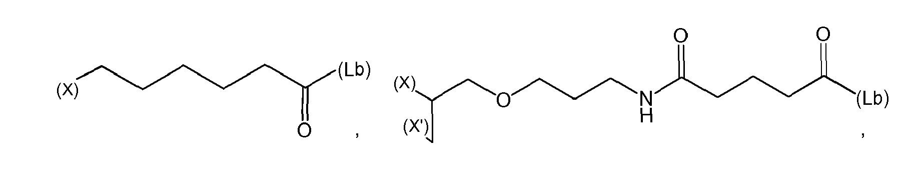 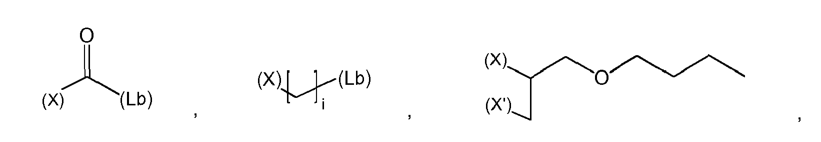 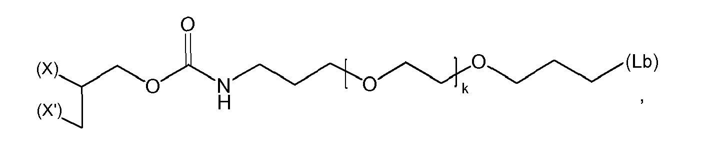 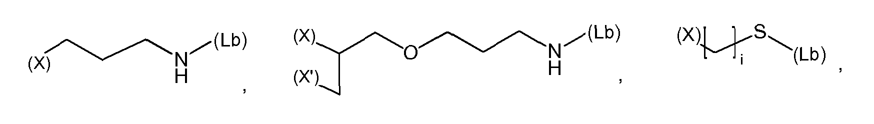 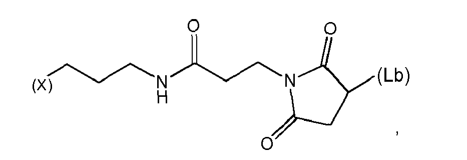 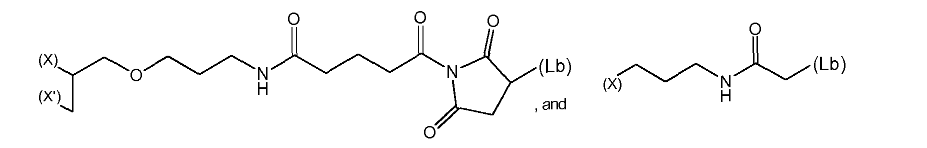 wherein
i represents an integer ranging from 1 to 5 and
k represents an integer ranging from 1 to 100;
Lb represents
(i) a bond,
(ii) a divalent group represented by a formula: -B^{1a}-Q^{b1}-B^{1b}-
wherein
B^{1a} and B^{1b} represent -CO-,
Q^{b1} represents a divalent group selected from 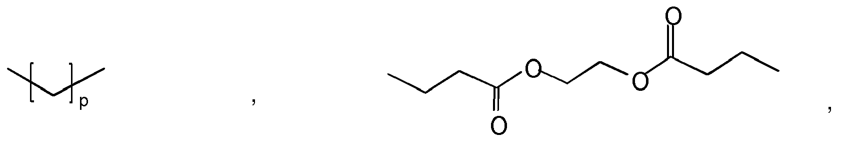 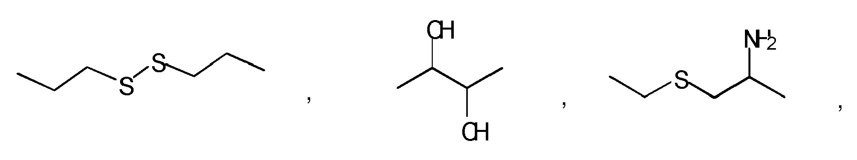 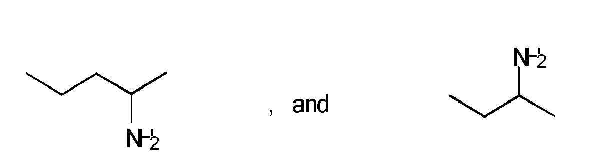 wherein p represents an integer ranging from 2 to 8,
(iii) a divalent group represented by a formula: -B^{2a}-Q^{b2}-B^{2b}-
wherein
B^{2a} represents -CO-,
B^{2b} represents 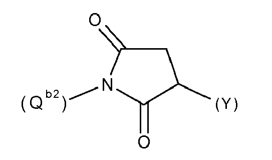 Q^{b2} represents a divalent group selected from 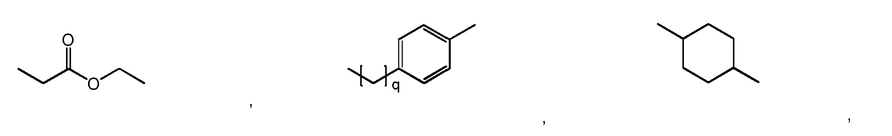 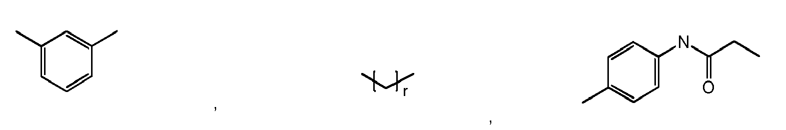 , and 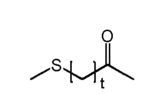 wherein
q represents an integer ranging from 3 to 10,
r represents an integer ranging from 1 to 10, and
t represents an integer ranging from 1 to 10, or
(iv) a divalent group represented by a formula: -B^{3a}-Q^{b3}-B^{3b}-
wherein
B^{3a} represents 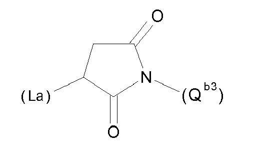 or a bond,
B^{3b} represents -CO-,
Q^{b3} represents a divalent group represented by a formula: -(CH₂)ₙ₁-Z-(CH₂)ₙ₂-
wherein n1 represents an integer ranging from 0 to 5,
n2 represents an integer ranging from 0 to 5,
Z represents a bond, -O-CO-, -CO-NH-, -CO-O-, -NH-CO-, 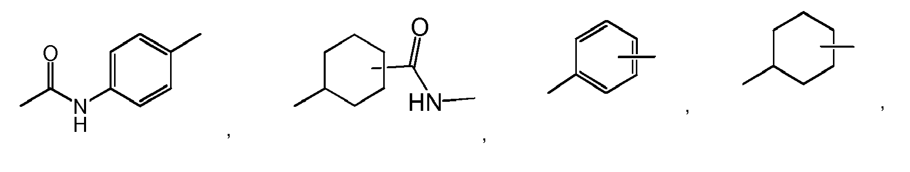 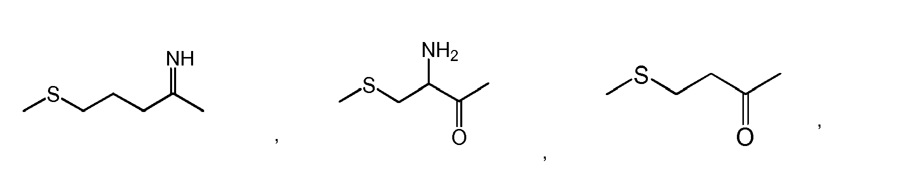 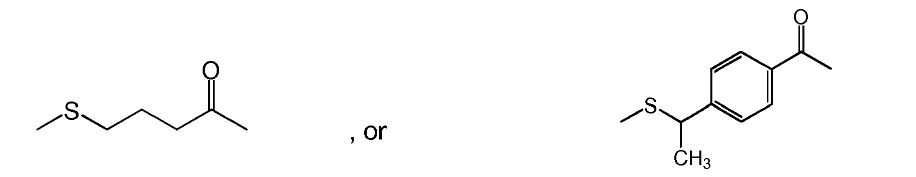 Lc represents
(i) a divalent group represented by a formula: -C^{a}-Q^{c}-C^{b}-
wherein
C^{a} represents -NH-,
Q^{c} represents a divalent group of a formula: -(CH₂)ₘ₁-Z^{c}-(CH₂)ₘ₂-
wherein
m1 represents an integer ranging from 0 to 15,
Z^{c} represents
(a) a bond or
(b) a divalent group selected from -CO-, -O-CO-, -CO-O-, -CO-NH-, -NH-CO-, -CO-NH-CO-, -NH-CO-NH-, -CH(NH₂)-, -CH(-NHR^{Zc1})-, -CH(R^{Zc2})-, -CH(OH)-, -CH(COOH)-, -C(=NH)-, -CH(-NHX)-, 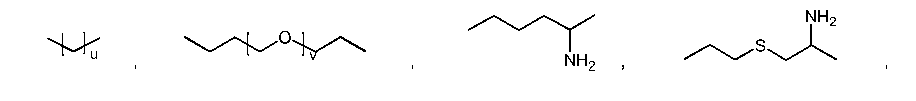 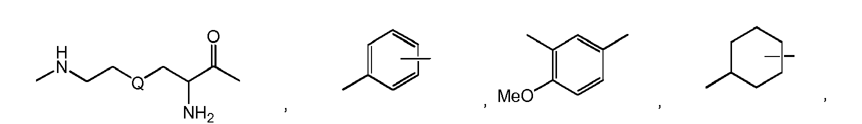 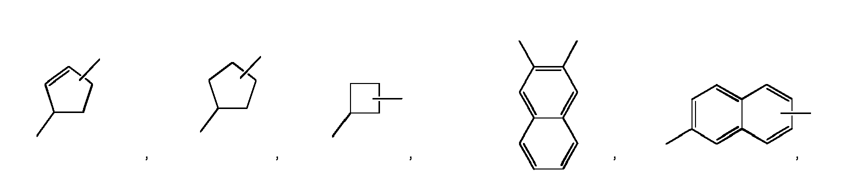 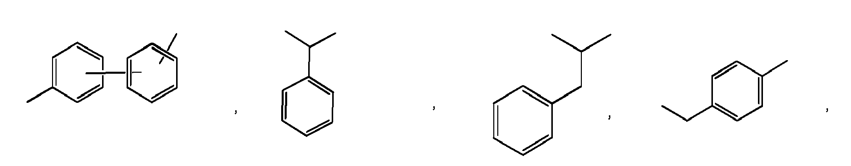 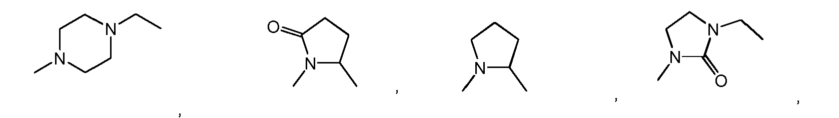 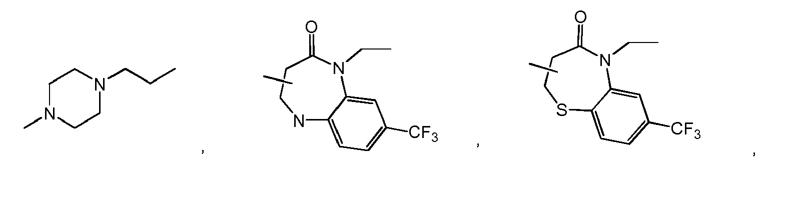 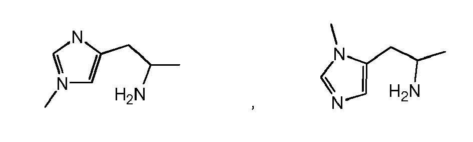 , and 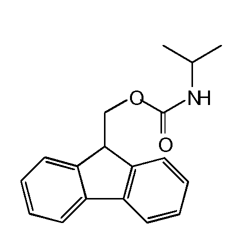 wherein
u represents an integer ranging from 1 to 18,
v represents an integer ranging from 1 to 12,
R^{Zc1} represents an amino - straight chain C₁-₅ alkyl-carbonyl group or X -straight chain C₁-₅ alkyl group,
R^{Zc2} represents an amino-straight chain C₁-₅ alkyl-carbonyl amino - straight chain C₁-₅ alkyl group, and
X represents the same as mentioned above, and
m2 represents an integer ranging from 0 to 15, and
C^{b} represents a bond, -CO-, or -SO₂-, or
(ii) a divalent group represented by a formula: -Q^{c'}-C^{b'}-
wherein
Q^{c'} represents a divalent group represented by a formula: -(CH₂)_{m1'}-Z^{c'}-(CH₂)_{m2'}-
wherein
m1' represents an integer ranging from 0 to 15,
Z^{c'} represents 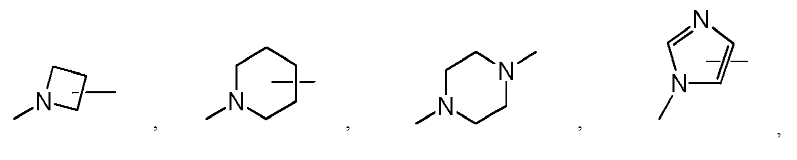 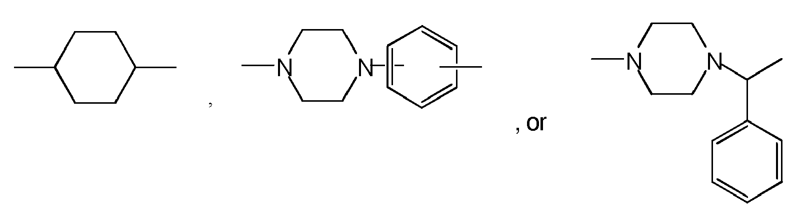 and
m2' represents an integer ranging from 0 to 15,
C^{b'} represents -CO- or -SO₂-;
j represents an integer ranging from 0 to 3,
provided that, if La is 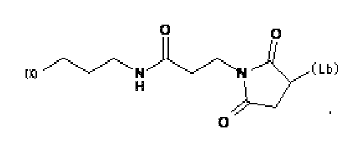 and Lb is a bond,
then Lc is not a bond; and
further provided that
if La is 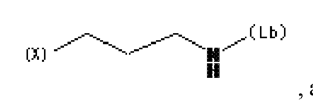 and
Lb is a divalent group represented by a formula: -CO-Q^{b2}-B^{2b}-
wherein
Q^{b2} is 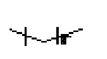 wherein r is 2,
B^{2b} is 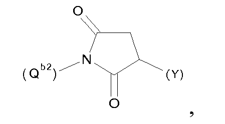 then Lc is not a bond.

2. The neuromedin U derivative according to claim 1 wherein the neuromedin U consists of an amino acid sequence represented by one of the sequence numbers: 1 to 6.

3. The neuromedin U derivative according to claim 1 wherein the polypeptide consists of 8 amino acids of the C-terminus of an amino acid sequence of neuromedin U.

4. The neuromedin U derivative according to claim 3 wherein the polypeptide consists of an amino acid sequence represented by one of the sequence numbers: 2, 4 and 6.

5. The neuromedin U derivative according to claim 1 which is represented by a formula: 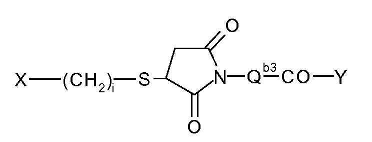 wherein
X represents a methoxypolyethylene glycol;
i represents an integer ranging from 0 to 5;
Q^{b3} represents a divalent group represented by a formula: -(CH₂)ₙ₁-Z-(CH₂)ₙ₂-
wherein
n1 represents an integer ranging from 0 to 5,
n2 represents an integer ranging from 0 to 5,
Z represents a bond, -O-CO-, -CO-NH-, -CO-O-, -NH-CO-, 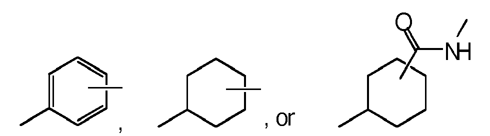 Y represents a polypeptide consisting of a amino acid sequence which contains at least 8 amino acids of the C-terminus of neuromedin U and is the same or substantially the same as the amino acid sequence of neuromedin U.

6. The neuromedin U derivative according to claim 1 which is represented by a formula: 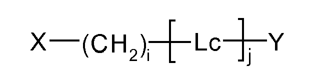 wherein
X represents a methoxypolyethylene glycol;
i represents an integer ranging from 1 to 5;
Lc represents
(i) a divalent group represented by a formula: -C^{a}-Q^{c}-C^{b}-
wherein
C^{a} represents -NH-,
Q^{c} represents a divalent group represented by a formula: -(CH₂)ₘ₁-Z^{c}-(CH₂)ₘ₂-
wherein
m1 represents an integer ranging from 0 to 15,
Z^{c} represents
(a) a bond or
(b) a divalent group selected from -CO-, -O-CO-, -CO-NH-, -NH-CO-, -CO-NH-CO-, -NH-CO-NH-, -CH(NH₂)-, -CH(NHR^{Zc1})-, -CH(R^{Zc2})-, -CH(OH)-, -CH(COOH)-, -C(=NH)-, -CH(-NHX)-, 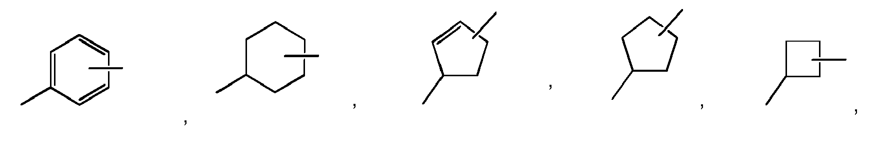 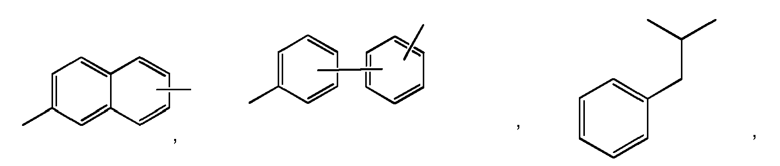 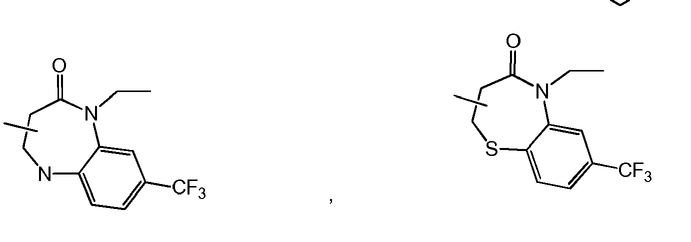 , or 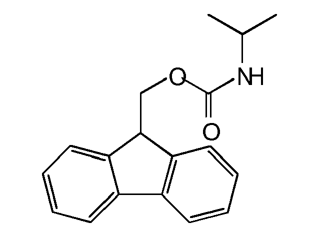 wherein
R^{Zc1} represents an amino-straight chain C₁₋₅ alkyl - carbonyl group or X -straight chain C₁₋₅ alkyl group,
R^{Zc2} represents an amino-straight chain C₁-₅ alkyl - carbonyl amino-straight chain C₁-₅ alkyl group, and
X represents the same as mentioned above, and m2 represents an integer ranging from 0 to 15, and
C^{b} represents -CO-, or -SO₂-, or
(ii) a divalent group represented by a formula: -Q^{c'}-C^{b'}-
wherein
Q^{c'} represents a divalent group represented by a formula: -(CH₂)_{m1'}-Z^{c'}-(CH₂)_{m2'}-
wherein
m1' represents an integer ranging from 0 to 15,
Z^{c'} represents 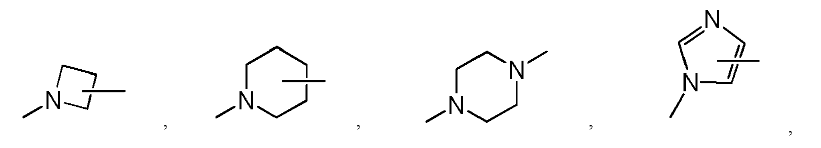 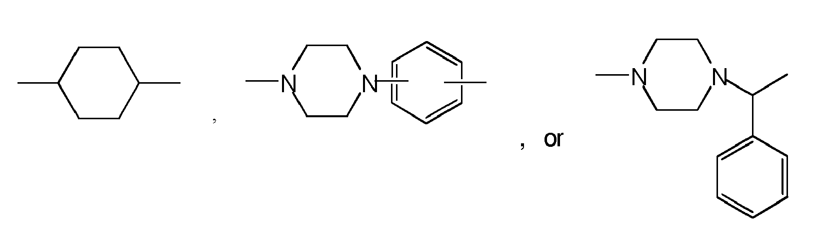 and
m2' represents an integer ranging from 0 to 15)
C^{b'} represents -CO- or -SO₂-; j' represents an integer ranging from 0 to 3; and
Y represents a polypeptide consisting of a amino acid sequence which contains at least 8 amino acids of the C-terminus of neuromedin U and is the same or substantially the same as the amino acid sequence of neuromedin U,
Lc can be identical or different when repeated.

7. The neuromedin U derivative according to claim 1 which is represented by a formula: 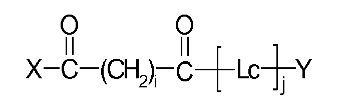 wherein
X represents a methoxypolyethylene glycol;
i represents an integer ranging from 1 to 5;
Lc represents
(i) a divalent group represented by a formula: -C^{a}-Q^{c}-C^{b'}-
wherein
C^{a} represents -NH-,
Q^{c} represents a divalent group represented by a formula: -(CH₂)ₘ₁-Z^{c}-(CH₂)ₘ₂-
wherein
m1 represents an integer ranging from 0 to 15,
Z^{c} represents a bond, -CO-, -O-CO-, -CO-NH-, -NH-CO-, -CO-NH-CO-, - NH-CO-NH-, -CH(NH₂)-, -CH(OH)-, -CH(COOH)-, -C(=NH)-, 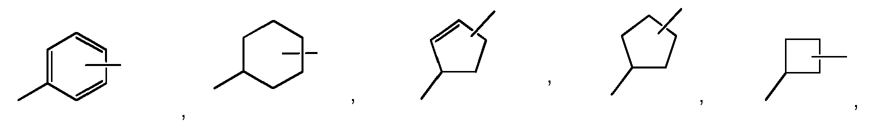 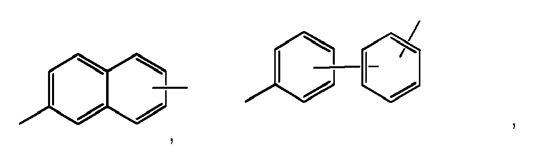 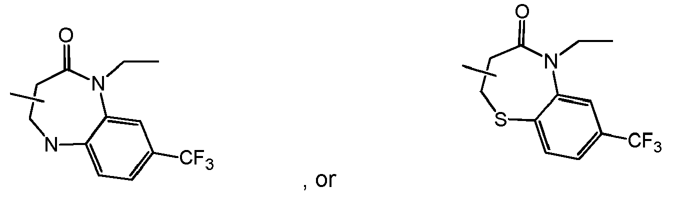 m2 represents an integer ranging from 0 to 15,
C^{b'} represents -CO-, or -SO₂-, or
(ii) a divalent group represented by a formula: -Q^{c'}-C^{b'}-
wherein
Q^{c'} represents a divalent group represented by a formula: -(CH₂)_{m1'}-Z^{c'}-(CH₂)_{m2'}-
wherein
m1' represents an integer ranging from 0 to 15,
Z^{c'} represents 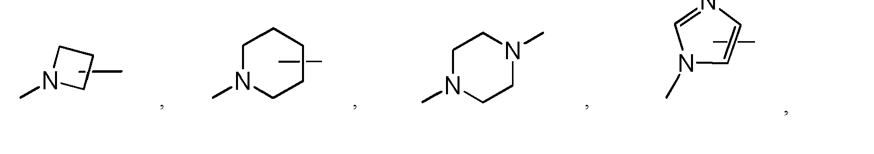 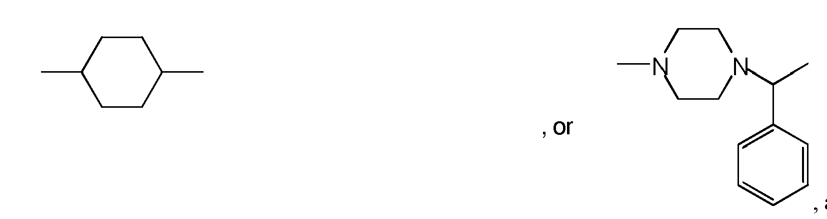 and
m2' represents an integer ranging from 0 to 15, and
C^{b'} represents -CO- or -SO₂-,
j' represents an integer ranging from 0 to 3, and
Y represents a polypeptide consisting of a amino acid sequence which contains at least 8 amino acids of the C-terminus of neuromedin U and is the same or substantially the same as the amino acid sequence of neuromedin U, and
Lc can be identical or different when repeated.

8. An antifeedant containing the neuromedin U derivative according to claim 1.

9. An agent for preventing or treating obesity which contains the neuromedin U derivative according to claim 1.

10. A method for preventing and treating obesity which is **characterized in that** an effective amount of the neuromedin U derivative according to claim 1 is administered in mammals.

11. Use of the neuromedin U derivative according to claim 1 for manufacturing an agent for preventing or treating.

12. A neuromedin U derivative
which is a polypeptide consisting of an amino acid sequence which is bound with a methoxypolyethylene glycol(s) via a linker,
said amino acid sequence contains at least 8 amino acids of the C-terminus of an amino acid sequence of neuromedin U, and is the same or substantially the same as the amino acid sequence of neuromedin U, and
which is represented by a formula: 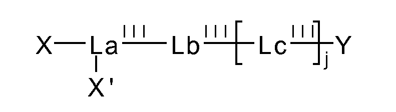 wherein
Y represents a polypeptide consisting of a amino acid sequence which contains at least 8 amino acids of the C-terminus of neuromedin U and is the same or substantially the same as the amino acid sequence of neuromedin U;
X represents a methoxyethylene glycol;
X' is absent or represents a methoxypolyethylene glycol;
the part represented by a formula (III): 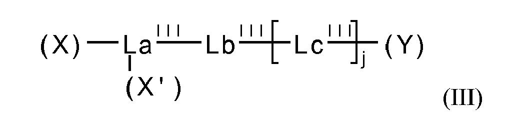 represents a linker,
La^{III} represents a divalent or trivalent group represented by a formula: 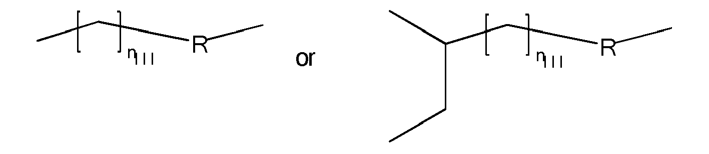 wherein
R represents a bond, -O-, -CO-O-, -O-CO-, -NH-, -CO-, -S-, -S-S-, -SO-, -SO₂-, -NH-SO₂, -SO₂-NH-, -C(=O)-NH-N=CH-, -C(=NH)-NH-, -CO-CH₂-S-, or 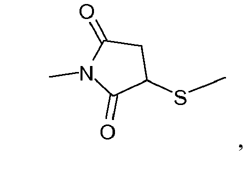 and
n _{III} represents an integer ranging from 0 to 5;
Lb^{III} represents -(CH₂)ᵢ- (wherein i represents an integer ranging from 1 to 5);
Lc^{III} represents
(i) a divalent group represented by a formula: -NH-Q^{cIII}-C^{bIII}-
wherein
Q^{cIII} represents a divalent group represented by a formula: -(CH₂)ₘ₁-Z^{cIII}-(CH₂)ₘ₂-
wherein
m1 represents an integer ranging from 0 to 15,
Z^{cIII} represents
(a) a bond or
(b) a divalent group selected from -CO-, -O-CO-, -CO-O-, -CO-NH-, -NH-CO-, -CO-NH-CO-, -NH-CO-NH-, -CH(NH₂)-, -CH(-NHR^{Zc1})-, -CH(R^{Zc2})-, - CH(OH)-, -CH(COOH)-, -C(=NH)-, -S-, -S-S-, -SO-, -SO₂-, -NH-SO₂, -SO₂-NH-, 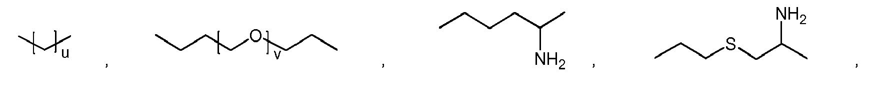 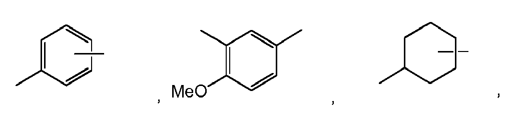 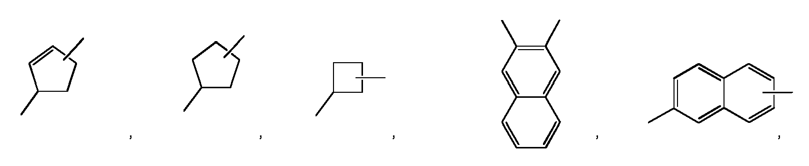 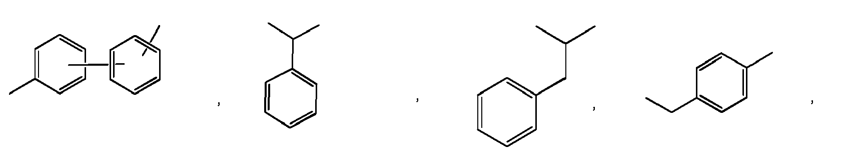 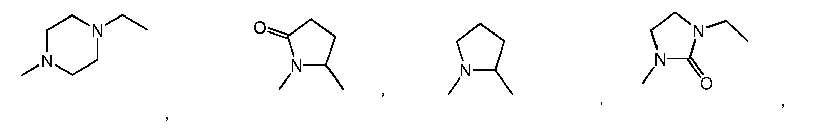 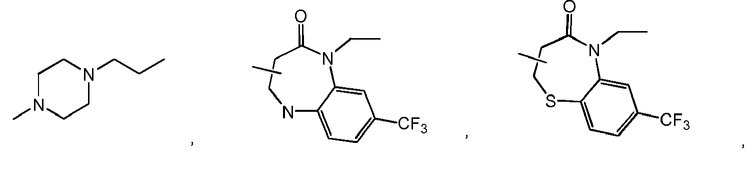 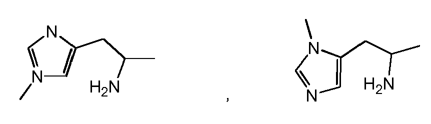 , and 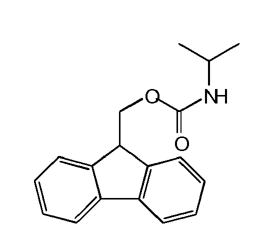 wherein
u represents a integer ranging from 1 to 18,
v represents an integer ranging from 1 to 12,
R^{Zc1} represents an amino-straight chain C₁-₅ alkyl-carbonyl group or X -straight chain C₁-₅ alkyl group,
R^{Zc2} represents an amino-straight chain C₁-₅ alkyl-carbonyl amino-straight chain C₁-₅ alkyl group, and
X represents the same as mentioned above), and
m2 represents an integer ranging from 0 to 15),
C^{bIII} represents a bond, -CO-, or -SO₂-, or
(ii) a divalent group represented by a formula: -Q^{cIII'}-C^{bIII'}-
wherein
Q^{cIII'} represents a formula: -(CH₂)_{m1'}-Z^{cIII'}-(CH₂)_{m2'}-
wherein
m1' represents an integer ranging from 0 to 15,
Z^{cIII'} represents a divalent group selected from 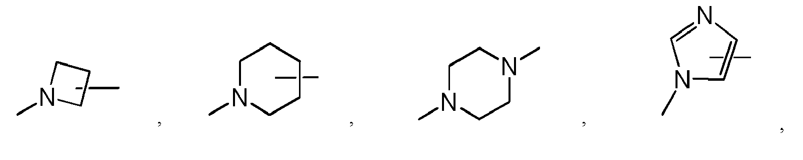 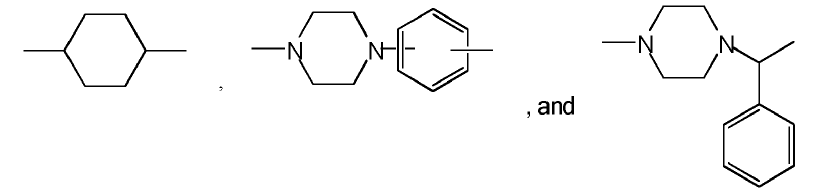 and
m2' represents an integer ranging from 0 to 15, and
C^{bIII'} represents -CO- or -SO₂-; and
j^{III} represents an integer ranging from 1 to 3.

13. The neuromedin U derivative as in the aforementioned [12] wherein the distance from the nitrogen atom closest to the Lb in the Lc to the nitrogen atom at the N-terminus of neuromedin U ranges from 3.5 to 30 Å.

14. The neuromedin U derivative as in the aforementioned [12] wherein
if L^{cIII} is
(i) a divalent group represented by a formula: -NH-Q^{cIII}-C^{bIII}-
wherein
Q^{cIII} represents a divalent group represented by a formula: -(CH₂)ₘ₁-
wherein m1 is an integer ranging from 0 to 15, and
C^{bIII} represents a bond, -CO-, or -SO₂-,
then the distance from the nitrogen atom ofNH in the formula: -NH-Q^{cIII}-C^{bIII}- to the nitrogen atom of the N-terminus of neuromedin U ranges from 3.5 to 7.0 Å.

15. The neuromedin U derivative as in the aforementioned [12] wherein
if L^{cIII} is
(i) a divalent group represented by a formula: -NH-Q^{cIII}-C^{bIII}-
wherein
Q^{cIII} represents a divalent group represented by a formula: -(CH₂)ₘ₁-Z^{cIII}-(CH₂)ₘ₂-
wherein
m1 is an integer ranging from 0 to 10,
Z^{cIII} represents a divalent group selected from -CO-, -O-CO-, -CO-O-, - CO-NH-, -NH-CO-, -CO-NH-CO-, -NH-CO-NH-, -CH(NH₂)-, -CH(-NHR^{Zc1})-, -CH(R^{Zc2})-, -CH(OH)-, -CH(COOH)-, -C(=NH)-, -S-, -S-S-, -SO-, -SO₂-, -NH-SO₂, -SO₂-NH-, 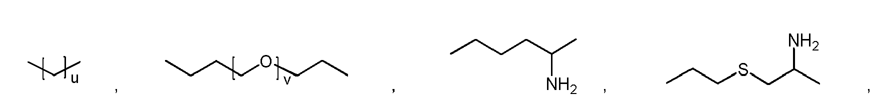 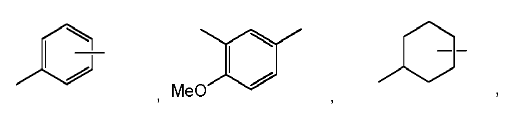 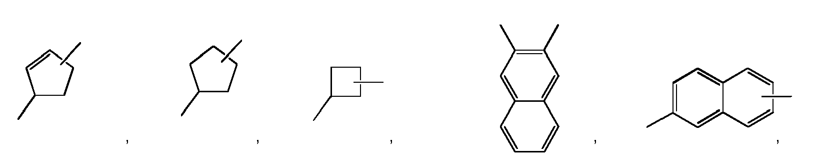 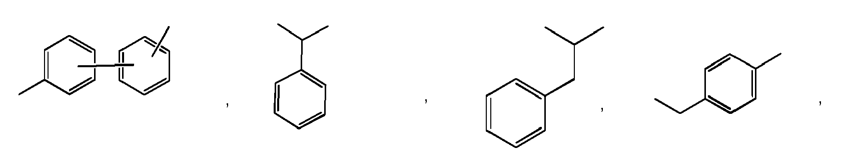 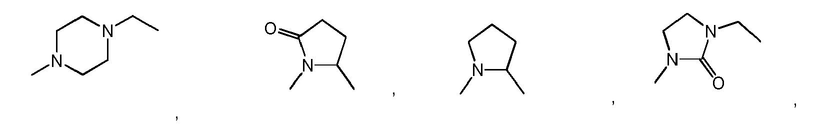 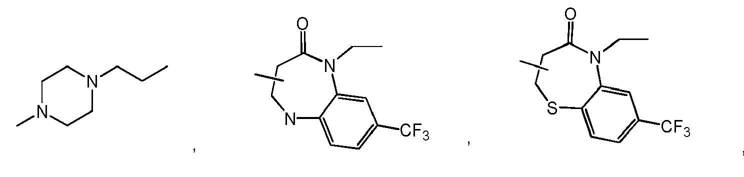 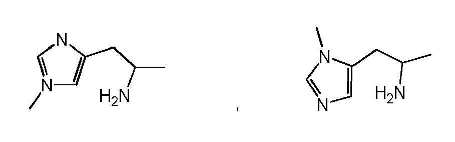 , and 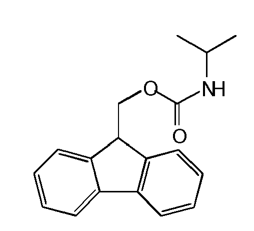 wherein
u represents an integer ranging from 1 to 10,
v represents an integer ranging from 1 to 10,
R^{Zc1} represents an amino-straight chain C₁-₅ alkyl-carbonyl group or X -straight chain C₁-₅ alkyl group,
R^{Zc2} represents an amino-straight chain C₁-₅ alkyl-carbonyl amino-straight chain C₁-₅ alkyl group, and
m2 represents an integer ranging from 0 to 5, and
C^{bIII} represents a bond, -CO-, or -SO₂-,
then the distance from the nitrogen atom ofNH in the formula: -NH-Q^{c}-C^{b}- to the atom nearest to the -(CH₂)ₘ₁- part in the Z^{c} ranges from 3.5 to 10 Å, and
the distance from the atom nearest to the -(CH₂)ₘ₁- part in the Z^{c} to the nitrogen atom of the N terminus of neuromedin U ranges from 3.5 to 7.0 Å.

16. The neuromedin U derivative as in the aforementioned [12] wherein
if L^{cIII} is
(ii) a divalent group represented by a formula: -Q^{cIII'}-C^{bIII'}-
wherein
Q^{cIII'} is a formula: -(CH₂)ₘ₁-Z^{c'}-(CH₂)_{m2'}-
wherein
m1' represents an integer ranging from 0 to 15,
Z^{c'} represents 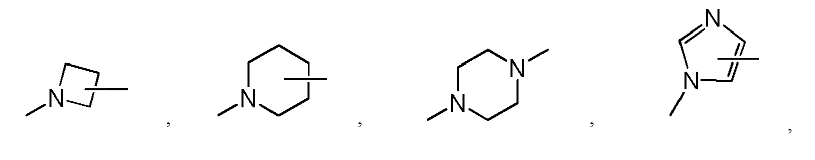 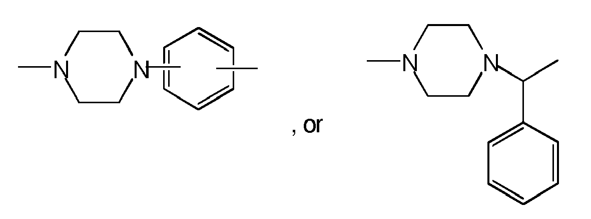 , and
m2' represent an integer ranging from 0 to 15), C^{bIII'} represents a bond, -CO- or -SO₂-,
then the distance from the nitrogen atom nearest to Lb in 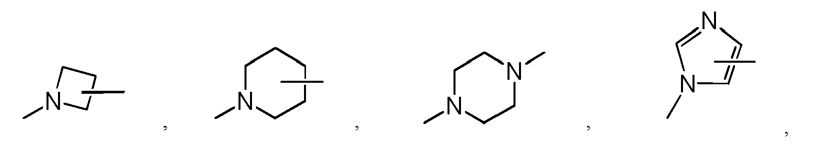 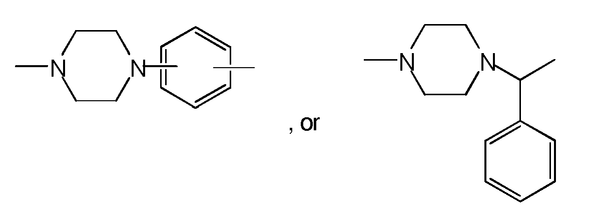 to the nitrogen atom of the N terminus of neuromedin U ranges from 5 to 10 Å.

17. A neuromedin U derivative
which is a polypeptide consisting of an amino acid sequence which is bound with a methoxypolyethylene glycol(s) via a linker,
said amino acid sequence contains at least 8 amino acids of the C-terminus of an amino acid sequence of neuromedin U, and is the same or substantially the same as the amino acid sequence of neuromedin U, and
which is represented by a formula: 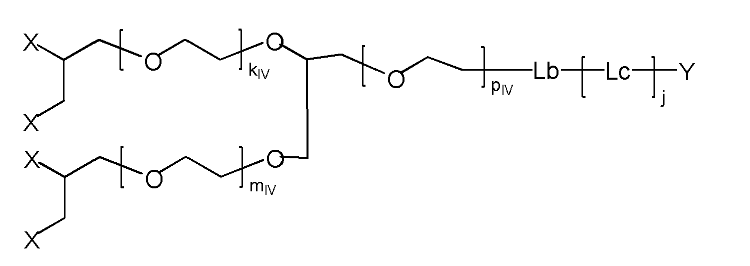 , or 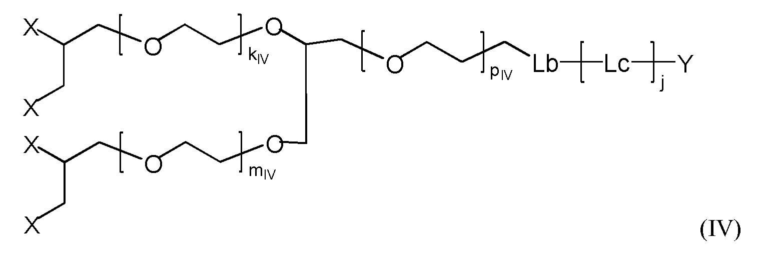 wherein
Y represents a polypeptide consisting of a amino acid sequence which contains at least 8 amino acids of the C-terminus of neuromedin U and is the same or substantially the same as the amino acid sequence of neuromedin U;
X represents a methoxypolyethylene glycol (here, the methoxypolyethylene glycol represented by plural Xs can be identical or different;
Lb represents
(i) a bond,
(ii) a divalent group represented by a formula: -B^{1a}-Q^{b1}-B^{1b}-
wherein
B^{1a} and B^{1b} represent -CO-,
Q^{b1} represents a divalent group selected from 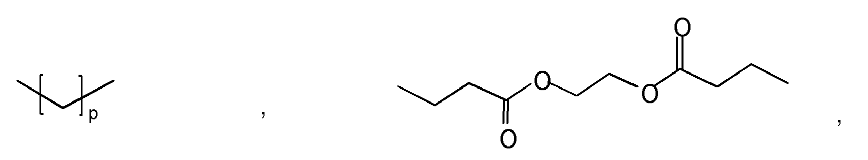 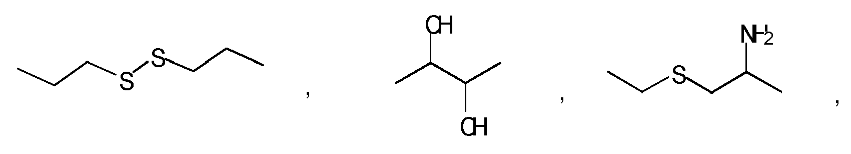 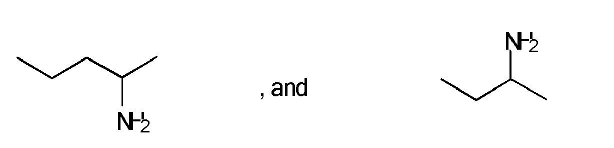 wherein p represents an integer ranging from 2 to 8,
(iii) a divalent group represented by a formula: -B^{2a}-Q^{b2}-B^{2b}-
wherein
B^{2a} represents -CO-,
B^{2b} represents 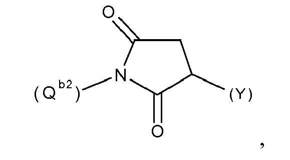 Q^{b2} represents a divalent group selected from 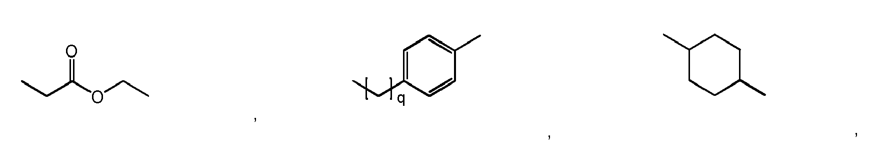 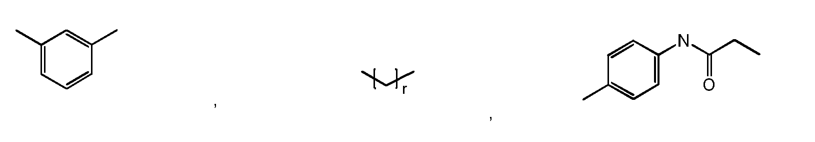 , and 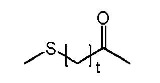 wherein
q represents an integer ranging from 3 to 10,
r represents an integer ranging from 1 to 10, and
t represents an integer ranging from 1 to 10, or
(iv) a divalent group represented by a formula: -B^{3a}-Q^{b3}-B^{3b}-
wherein
B^{3a} represents 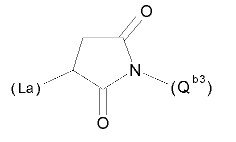 or a bond,
B^{3b} represents -CO-,
Q^{b3} represents a divalent group represented by -(CH₂)ₙ₁-Z-(CH₂)ₙ₂-
wherein
n1 represents an integer ranging from 0 to 5,
n2 represents an integer ranging from 0 to 5,
Z represents a bond, -O-CO-, -CO-NH-, -CO-O-, -NH-CO-, 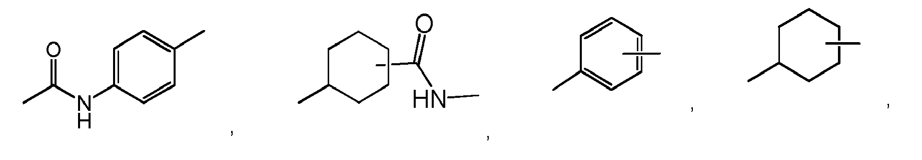 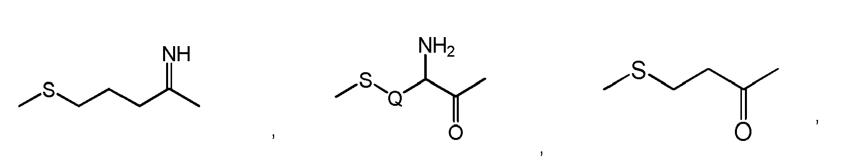 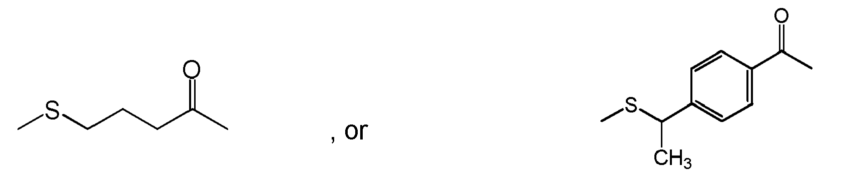 Lc represents
(i) a divalent group represented by a formula: -C^{a}-Q^{c}-C^{b}-
wherein
C^{a} represents -NH-,
Q^{c} represents a divalent group represented by a formula: -(CH₂)ₘ₁-Z^{c}-(CH₂)ₘ₂-
wherein
m1 represents an integer ranging from 0 to 15,
Z^{c} represents
(a) a bond or
(b) a divalent group selected from -CO-, -O-CO-, -CO-O-, -CO-NH-, -NH-CO-, -CO-NH-CO-, -NH-CO-NH-, -CH(NH₂)-, -CH(-NHR^{Zc1})-, -CH(R^{Zc2})-, -CH(OH)-, -CH(COOH)-, -C(=NH)-, -CH(NHX)-, 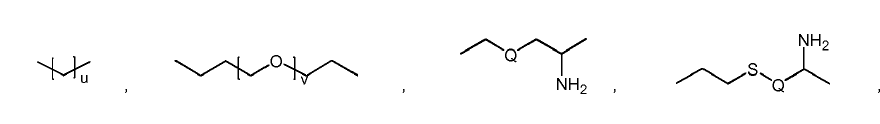 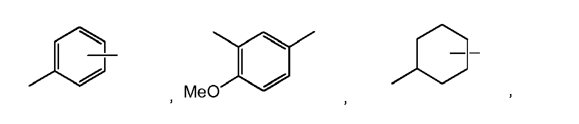 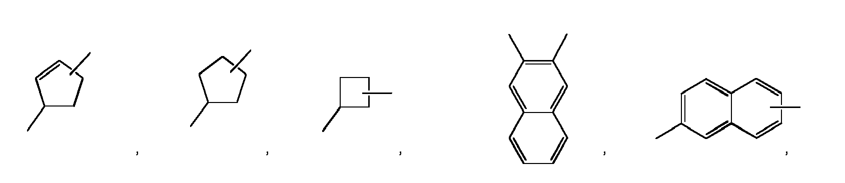 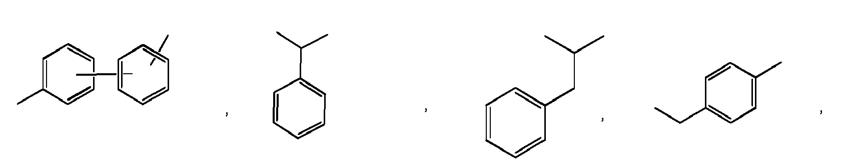 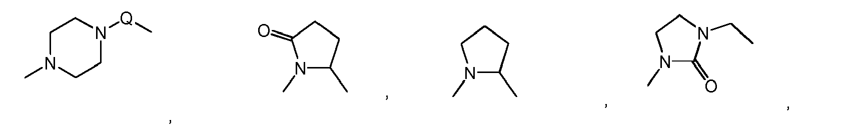 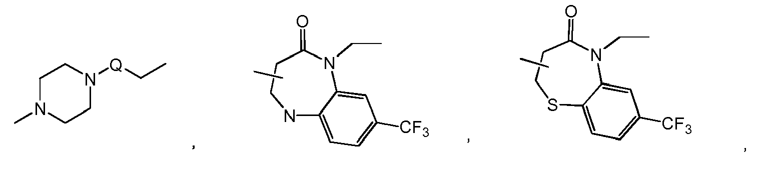 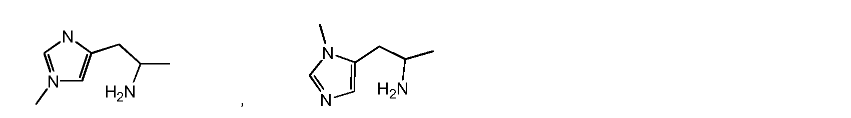 , and 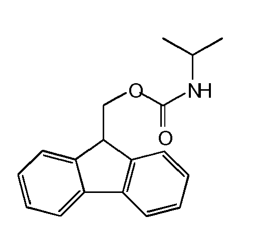 wherein
u represents an integer ranging from 1 to 18,
v represents an integer ranging from 1 to 12,
R^{Zc1} represents an amino-straight chain C₁-₅ alkyl-carbonyl group or X -straight chain C₁-₅ alkyl group,
R^{Zc2} represents an amino-straight chain C₁-₅ alkyl-carbonyl amino-straight chain C₁-₅ alkyl group,
X represents the same as above,
m2 represents an integer ranging from 0 to 15,
C^{b} represents a bond, -CO- or -SO₂-, or
(ii) a divalent group represented by -Q^{c'}-C^{b'}-
wherein
Q^{c'} represents a formula: -(CH₂)_{m1'}-Z^{c'}-(CH₂)_{m2'}-
wherein
m1' represents an integer ranging from 0 to 15,
Z^{c'} represents 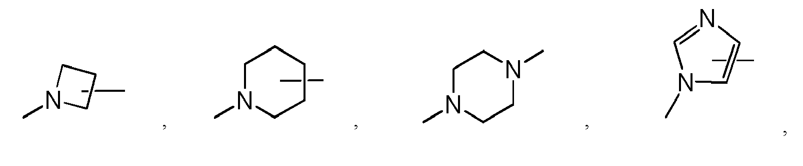 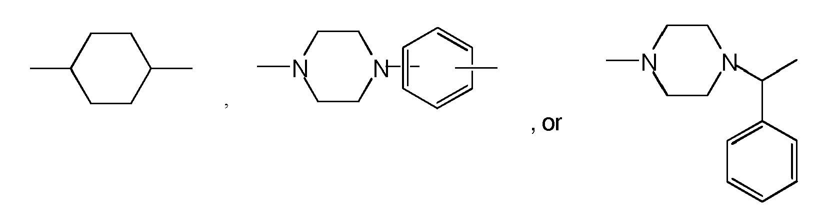 and
m2' represents an integer ranging from 0 to 15,
C^{b'} represents -CO- or -SO₂-;
k_{IV} represents an integer ranging from 1 to 100;
m_{IV} represents an integer ranging from 1 to 100;
p_{IV} represents an integer ranging from 1 to 100; and
j represents an integer ranging from 0 to 3.

18. A neuromedin U derivative
which is a polypeptide consisting of an amino acid sequence which is bound with a methoxypolyethylene glycol(s) via a linker,
said amino acid sequence contains at least 8 amino acids of the C-terminus of an amino acid sequence of neuromedin U, and is the same or substantially the same as the amino acid sequence of neuromedin U, and
which is represented by a formula: 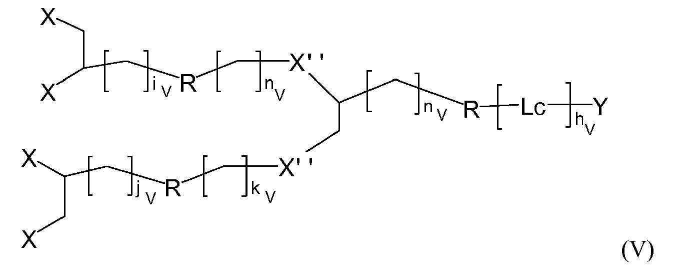 wherein
Y represents a polypeptide consisting of a amino acid sequence which contains at least 8 amino acids of the C-terminus of neuromedin U and is the same or substantially the same as the amino acid sequence of neuromedin U;
X represents a methoxypolyethylene glycol (here, the methoxypolyethylene glycols represented by plural Xs can be identical or different),
X" represents a polyethylene glycol (here, the polyethylene glycols represented by plural X" can be identical or different),
Lc represents
(i) a divalent group represented by a formula: -C^{a}-Q^{c}-C^{b}-
wherein
C^{a} represents -NH-,
Q^{c} represents a divalent group: -(CH₂)ₘ₁-Z^{c}-(CH₂)ₘ₂-
wherein
m1 represents an integer ranging from 0 to 15,
Z^{c} represents
(a) a bond or
(b) a divalent group selected from -CO-, -O-CO-, -CO-O-, -CO-NH-, -NH-CO-, -CO-NH-CO-, -NH-CO-NH-, -CH(NH₂)-, -CH(-NHR^{Zc1})-, -CH(R^{Zc2})-, -CH(OH)-, -CH(COOH)-, -C(=NH)-, -CH(NHX)-, -S-, -S-S-, -SO-, -SO₂-, -NH-SO₂-, - SO₂ -NH-, 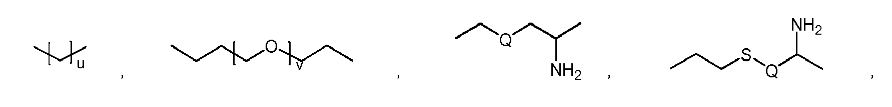 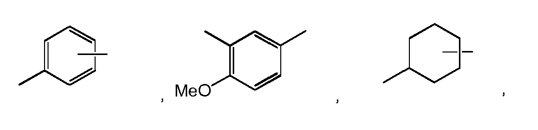 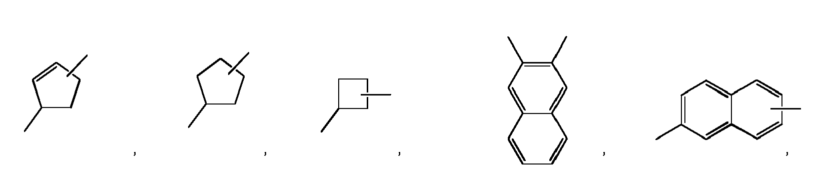 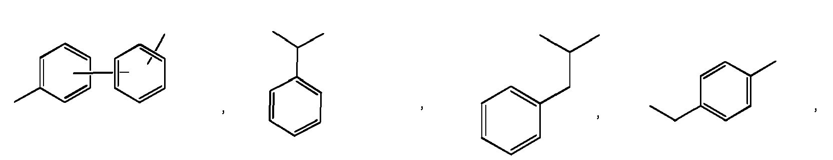 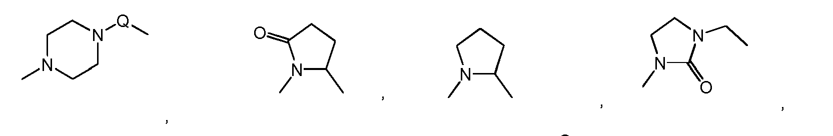 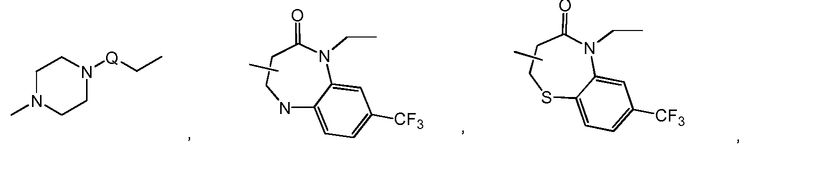 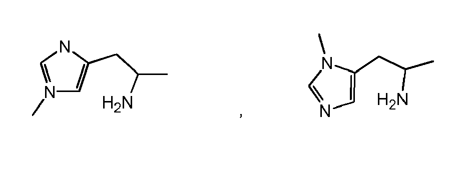 , and 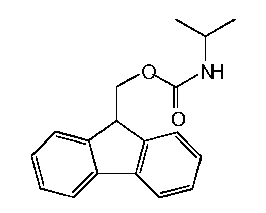 wherein
u represents an integer ranging from 1 to 18,
v represents an integer ranging from 1 to 12,
R^{Zc1} represents an amino-straight chain C₁-₅ alkyl-carbonyl group or X -straight chain C₁-₅ alkyl group,
R^{Zc2} represents an amino-straight chain C₁-₅ alkyl-carbonyl amino-straight chain C₁-₅ alkyl group,
X represents the same as above, and
m2 represents an integer ranging from 0 to 15, and
C^{b} represents a bond, -CO- or -SO₂-, or
(ii) a divalent group represented by -Q^{c'}-C^{b'}-
wherein
Q^{c'} represents a formula: -(CH₂)_{m1'}-Z^{c'}-(CH₂)_{m2'}-
wherein
m1' represents an integer ranging from 0 to 15, Z^{c'} represents 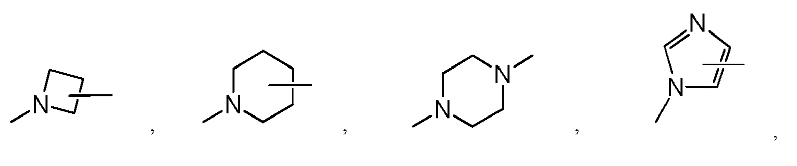 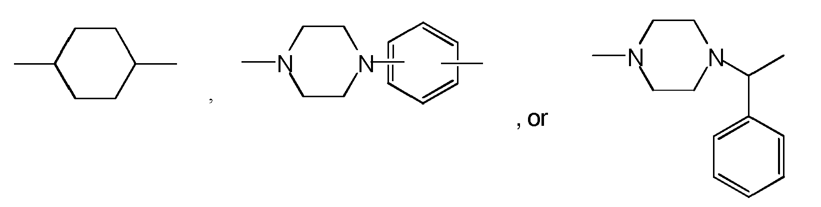 m2' represents an integer ranging from 0 to 15, and
C^{b'} represents -CO- or -SO₂-;
R is , at each occurrence, identical or different, and represents a divalent group selected from a bond, -O-, -CO-O-, -O-CO-, -NH-, -CO-, -S-, -S-S-, -SO-, -SO₂-, -NH-SO₂-, -SO₂-NH-, -C(=O)-NH-N=CH-, -C(=NH)-NH-, -CO-CH₂-S-, and 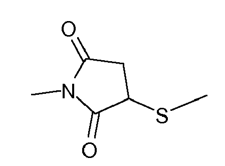 h_{V} represents an integer ranging from 0 to 3; and
i_{V}, j_{V}, k_{V}, m_{V} and n_{V} can be respectively identical or different, which represent an integer ranging from 0 to 5.
